(19) Europäisches Patentamt
European Patent Office
Office européen des brevets

(11) Publication number: 0 133 309 B1

(12) EUROPEAN PATENT SPECIFICATION

(45) Date of publication of patent specification: 23.11.89

(51) Int. Cl.$^4$: C 07 D 471/14, C 07 D 491/22, C 07 D 495/22, A 01 N 43/50

(21) Application number: 84108921.2

(22) Date of filing: 27.07.84

(60) Divisional application 88101870 filed on 09.02.88.

# (54) Herbicides and method for the preparation thereof.

(30) Priority: 02.08.83 US 519603

(43) Date of publication of application: 20.02.85 Bulletin 85/08

(45) Publication of the grant of the patent: 23.11.89 Bulletin 89/47

(84) Designated Contracting States: AT BE CH DE FR GB IT LI NL SE

(56) References cited:
EP-A-0 041 623
US-A-4 017 510
US-A-4 041 045
US-A-4 188 487

(73) Proprietor: AMERICAN CYANAMID COMPANY
1937 West Main Street P.O. Box 60
Stamford Connecticut 06904-0060 (US)

(72) Inventor: Los, Marinus
107 Drummond Drive
Pennington New Jersey 08534 (US)

(74) Representative: Wächtershäuser, Günter, Dr.
Tal 29
D-8000 München 2 (DE)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel herbicidally effective, dihydroimidazopyrrolopyridines and derivatives thereof which are useful for the control of undesirable monocotyledonous and dicotyledonous plant species. The invention also relates to a process for the preparation of said herbicidally effective compounds.

More particularly, this invention relates to novel, herbicidally effective, dihydroimidazopyrrolopyridines, quinolines, thieno- and furo[2,3-*b*]pyridines, dihydrothieno and furo(2,3-*b*]pyridines, thieno- and furo[3,2-*b*]pyridines und dihydrothieno- and furo[3,2-*b*]pyridines depicted by formula (I) below:

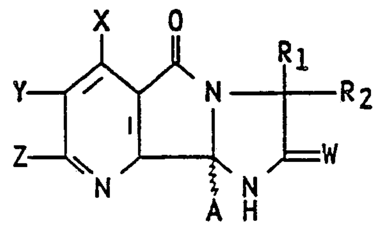

(I)

wherein

$R_1$ and $R_2$ each represent $C_1$—$C_3$ alkyl or cyclopropyl, with the proviso that the sum of the number of carbon atoms in $R_1$ and $R_2$ is 2 to 5; and when $R_1$ and $R_2$ are taken together with the carbon to which they are attached, they may form a $C_3$—$C_6$ cycloalkyl ring optionally substituted with methyl;

W is oxygen or sulfur;

A is hydrogen, hydroxyl, $C_3$—$C_6$ alkenyloxy, $C_3$—$C_6$ alkynyloxy, $C_1$—$C_6$ alkylthio, $NR_{13}R_{14}$ or $C_1$—$C_6$ alkoxy optionally substituted with phenyl, halophenyl, $C_1$—$C_3$ alkylphenyl, $C_1$—$C_3$ alkoxyphenyl or di-$C_1$—$C_3$ alkylaminophenyl;

$R_{13}$ is hydrogen, $C_1$—$C_4$ alkyl optionally substituted with phenyl, halophenyl, $C_1$—$C_3$ alkylphenyl or $C_1$—$C_3$ alkoxyphenyl;

$R_{14}$ is hydrogen or $C_1$—$C_4$ alkyl;

X is hydrogen, halogen or methyl;

Y and Z are each hydrogen, halogen, $C_1$—$C_6$ alkyl, $C_1$—$C_4$ hydroxyalkyl, $C_1$—$C_6$ alkoxy, $C_1$—$C_4$ alkylthio, phenoxy, $C_1$—$C_4$ haloalkyl, $OCF_2CHF_2$, $OCF_3$, $OCHF_2$, nitro, cyano, $NR_4R_5$, $C_3$—$C_8$ straight or branched alkenyloxy optionally substituted with one to three halogens, $C_3$—$C_8$ straight or branched alkynyloxy optionally substituted with one to three halogens, or phenyl optionally substituted with one $C_1$—$C_4$ alkyl, $C_1$—$C_4$ alkoxy or halogen;

$R_4$ is hydrogen or $C_1$—$C_4$ alkyl;

$R_5$ is $C_1$—$C_4$ alkyl;

And, when taken together, Y and Z may form a ring in which YZ is represented by

(1) the structure: $—(CH_2)_n—$, where n is an integer of 2, 3 or 4, provided that X is hydrogen; or

(2) by the structure:

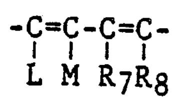

where, L, M, $R_7$ and $R_8$ each represent hydrogen, halogen, $C_1$—$C_4$ alkyl, $C_1$—$C_4$ alkoxy, $C_1$—$C_4$ alkylthio, $C_1$—$C_4$ alkylsulfonyl, $C_1$—$C_4$ haloalkyl, $NO_2$, CN, phenyl, phenoxy, amino, $OCF_3$, $OCHF_2$, $OCF_2CHF_2$, $C_1$—$C_4$ alkylamino, dialkyl($C_1$—$C_4$)amino, chlorophenyl, methylphenyl, $C_3$—$C_8$ straight or branched alkenyloxy optionally substituted with one to three halogens, $C_3$—$C_8$ straight or branched alkynyloxy optionally substituted with one to three halogens, or phenoxy substituted with one Cl, $CF_3$, $NO_2$ or $CH_3$ group, with the proviso that only one of L, M, $R_7$ or $R_8$ may represent a substituent other than hydrogen, halogen, $C_1$—$C_4$ alkyl or $C_1$—$C_4$ alkoxy; and when A is OH at least one of L, M, $R_7$ and $R_8$ is other than hydrogen; or

(3) by the structures;

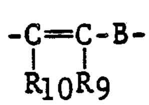 , 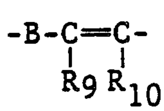 , 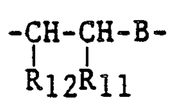 , or 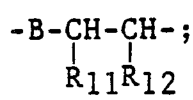 ;

where B is oxygen or sulfur; $R_8$ and $R_{10}$ each represent hydrogen, halogen, phenyl, or $C_1$—$C_4$ alkyl; $R_{11}$ and $R_{12}$ each represent hydrogen, $C_1$—$C_4$ alkyl or phenyl;
and when $R_1$ and $R_2$ are not the same, the *cis*- or *trans*-isomers or mixtures thereof or the optical isomers (*cis* or *trans*- or mixtures thereof).

As used in the present specification and claims, the term "halogen" means F, Cl, Br or I, unless otherwise specified.

It should, of course, be understood that when $R_1$ and $R_2$ are not the same, the formula (I) compounds can exist as both the *cis*- and *trans*-isomers. These can be illustrated as follows:

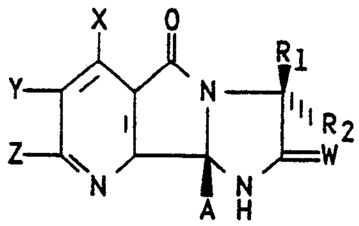

(Ia) *cis*, and

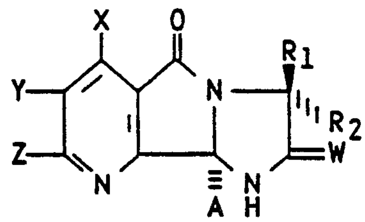

(Ib) *trans*.

Especially preferred compounds of the present invention are more precisely illustrated by formulas II, III, V, VI, VII and VIII shown below.

Preferred dihydroimidazopyrrolopyridine compounds of this invention are depicted by formula (II), hereinafter illustrated:

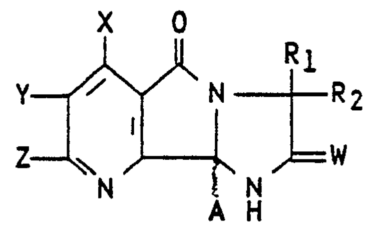

(II)

wherein A, $R_1$, $R_2$, W and X are as defined in reference to formula I above; Y and Z each, independently, represent hydrogen, halogen, $C_1$—$C_6$ alkyl, $C_1$—$C_6$ alkoxy, CN, $NO_2$, $OCF_3$, $OCHF_2$, $OCF_2CHF_2$, phenoxy, $C_1$—$C_4$ haloalkyl, $C_1$—$C_4$ alkylthio, $C_1$—$C_4$ hydroxyalkyl, $NR_4R_5$, $C_3$—$C_8$ straight or branched alkenyloxy optionally substituted with one to three halogens, $C_3$—$C_8$ straight or branched alkynyloxy optionally substituted with one to three halogens, or phenyl optionally substituted with one $C_1$—$C_4$ alkyl, $C_1$—$C_4$ alkoxy or halogen; $R_4$ is hydrogen or $C_1$—$C_4$ alkyl; $R_5$ is $C_1$—$C_4$ alkyl; and when taken together Y and Z may form a ring in which YZ are represented by the structure: $—(CH_2)_n—$, where n is an integer of 2, 3 or 4; and when $R_1$ and $R_2$ are not the same, the *cis*- or *trans*-isomers or mixtures thereof or the optical isomers (*cis*- or *trans*- or mixtures thereof).

Preferred dihydroimidazopyrroloquinolines are illustrated by formula (III):

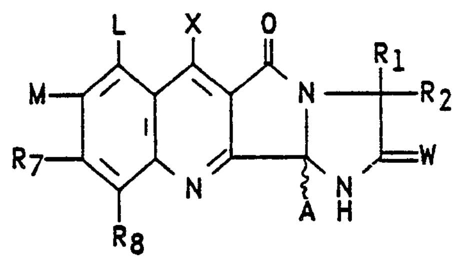

(III)

wherein A, $R_1$, $R_2$, W and X, are as defined above in reference to formula I, and L, M, $R_7$ and $R_8$ represent hydrogen, halogen, $C_1$—$C_4$ alkyl, $C_1$—$C_4$ alkoxy, $C_1$—$C_4$ alkylthio, $C_1$—$C_4$ alkylsulfonyl, $C_1$—$C_4$ haloalkyl, $NO_2$, CN, phenyl, phenoxy, amino, $CF_3$, $OCHF_2$, $OCF_2CHF_2$, $C_1$—$C_4$ alkylamino, dialkyl($C_1$—$C_4$)amino, chlorophenyl, methylphenyl, $C_3$—$C_8$ straight or branched alkenyloxy optionally substituted with one to three halogens, $C_3$—$C_8$ straight or branched alkynyloxy optionally substituted with one to three halogens, or phenoxy substituted with one Cl, $CF_3$, $NO_2$ or $CH_3$ group, with the proviso that only one of L, M, $R_7$ or $R_8$, may represent a substituent other than hydrogen, halogen, $C_1$—$C_4$ alkyl or $C_1$—$C_4$ alkoxy; and when $R_1$ and $R_2$ are not the same, the *cis*- or *trans*-isomers or mixtures thereof or the optical isomers (*cis*- or *trans*- or mixtures thereof).

Preferred dihydroimidazopyrrolothieno- and furo[3,2-*b*]pyridines and dihydroimidazopyrrolo-dihydrothieno- and furo[3,2-*b*]pyridines are, respectively, illustrated by formulas (V) and (VI), shown below:

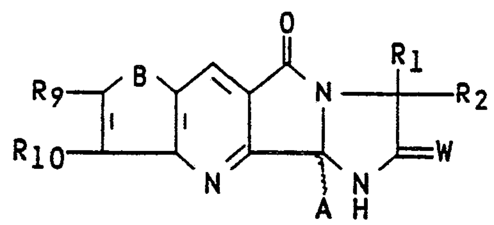

(V) and,

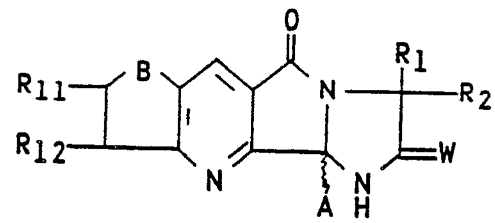

(VI)

wherein A, $R_1$, $R_2$, W and B, are as defined above in reference to formula I; $R_9$ and $R_{10}$ each represent hydrogen, halogen, $C_1$—$C_4$ alkyl or phenyl; and $R_{11}$ and $R_{12}$ each represent hydrogen, $C_1$—$C_4$ alkyl or phenyl; and when $R_1$ and $R_2$ are not the same, the *cis*- or *trans*-isomers or mixtures thereof or the optical isomers (*cis*- or *trans*- or mixtures thereof).

The preferred dihydroimidazopyrrolothieno- and furo[2,3-*b*]pyridines and the dihydroimidazo-pyrrolodihydrothieno- and furo[2,3-*b*]pyridines are, respectively, depicted by formulas (VII) and (VIII) illustrated as follows:

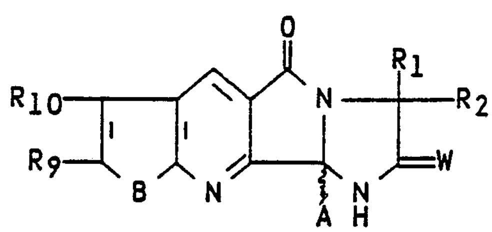

(VII) and,

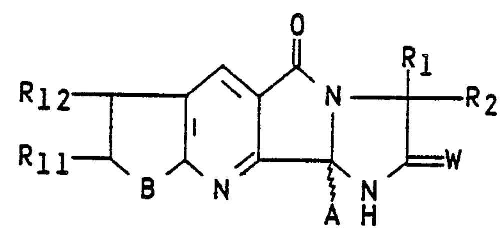

(VIII)

wherein A, $R_1$, $R_2$, W and B, are as defined above in reference to formula I, $R_9$ and $R_{10}$ each represent hydrogen, halogen, $C_1$—$C_4$ alkyl or phenyl; $R_{11}$ and $R_{12}$ each represent hydrogen, $C_1$—$C_4$ alkyl or phenyl; and when $R_1$ and $R_2$ are not the same, the *cis*- or *trans*-isomers or mixtures thereof or the optical isomers (*cis*- or *trans*- or mixtures thereof).

It is obvious that in the case of formula (VI) and (VIII) when either $R_{11}$ or $R_{12}$ is other than hydrogen, additional optical and *cis*- and *trans*-isomers are possible, and all of these are considered to be within the scope of the invention.

Although dihydroimidazoisoindolediones are described as herbicidal agents in United States Patent 4,041,045 issued August 9, 1977, said publication does not render the compounds of this invention obvious since it does not disclose or imply the disclosure of herbicidal compounds containing a pyridine or substituted pyridine ring. Moreover, it is surprising to find that the formula I compounds of this invention are frequently found to be highly selective herbicidal agents effective for the preemergence and postemergence control of a wide variety of undesirable broadleaf weeds and grasses in the presence of graminaceous crops such as corn, rice and wheat; leguminous crops such as soybeans and in the presence of a variety of other crops including cotton, when compared with compounds known from EP—A—0 041 623 or US—A—4 017 510.

In accordance with the process of the present invention, the novel formula (I) dihydroimidazoimidazo-pyrrolopyridines, quinolines and the like can be prepared by reaction of the appropriately substituted or unsubstituted formula (XII) 2-(2-imidazolidinyl)nicotinic acid; 2-(2-imidazolidinyl)quinoline-3-carboxylic acid; 2-(2-imidazolidinyl)thieno- or furo[3,2-*b*]pyridine-6-carboxylic acid; 2-(2-imidazolidinyl)dihydrothieno- or furo[3,2-*b*]pyridine-6-carboxylic acid; 2-(2-imidazolidinyl)thieno- or furo[2,3-*b*]pyridine-5-carboxylic acid or 2-(2-imidazolidinyl)dihydrothieno or furo[2,3-*b*]pyridine-5-carboxylic acid, with at least one molar equivalent and preferably a slight excess of acetic anhydride in the presence of pyridine and acetonitrile.

In practice, it is generally desirable to warm the reaction mixture to about 40 to 60°C until an essentially clear solution is obtained. Cooling of the thus-prepared solution to about ambient temperature or below then yields the desired formula (I) dihydroimidazopyrrolopyridine, quinoline, thieno or furo-pyridine or

dihydrothieno or furopyridine, corresponding to the isomeric mixture of the substituted or unsubstituted acid employed as starting material for the reaction.

The reaction may be graphically illustrated as follows:

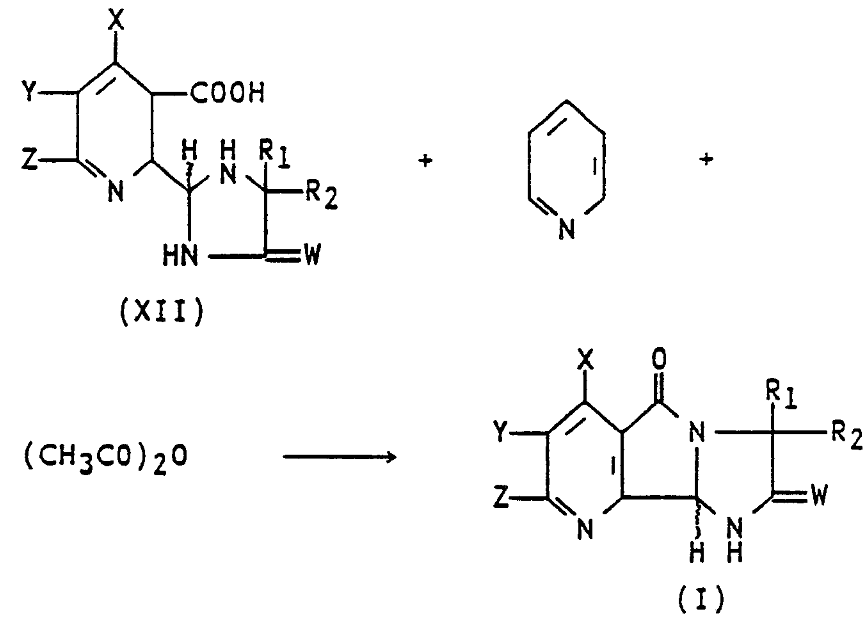

(XII)

$(CH_3CO)_2O$

(I)

wherein X, Y, Z, $R_1$, $R_2$ and W are as described for formula (I) above.

Similarly, in an alternate procedure it has also been found that the substituted or unsubstituted 2-(2-imidazolidinyl)nicotinic acid; 2-(2-imidazolidinyl)quinoline-3-carboxylic acid; 2-(2-imidazolidinyl)-thieno- or furo[3,2-*b*]pyridine-6-carboxylic acid; 2-(2-imidazolidinyl)dihydrothieno- or furo[3,2-*b*]pyridine-6-carboxylic acid; 2-(2-imidazolidinyl)thieno- or furo[2,3-*b*]pyridine-5-carboxylic acid or 2-(2-imidazolidinyl)-dihydrothieno- or furo[2,3-*b*]pyridine-5-carboxylic acid, which are themselves pre- and postemergence herbicides, can be converted to the corresponding formula (I) dihydroimidazopyrrolopyridine, quinoline, thieno- or furo[3,2-*b*]pyridine, dihydrothieno- or furo[3,2-*b*]pyridine, thieno- or furo[2,3-*b*]pyridine or the dihydrothieno- or furo[2,3-*b*]pyridine, by reaction thereof with at least an equimolar amount of N,N'-dicyclohexylcarbodiimide in the presence of a chlorinated hydrocarbon solvent such as methylene chloride, dichloroethane, chloroform or the like. The reaction may be conducted at ambient temperature and yields the desired formula (I) compound on evaporation or separation of the solvent from the reaction mixture. The reaction may be graphically illustrated as follows:

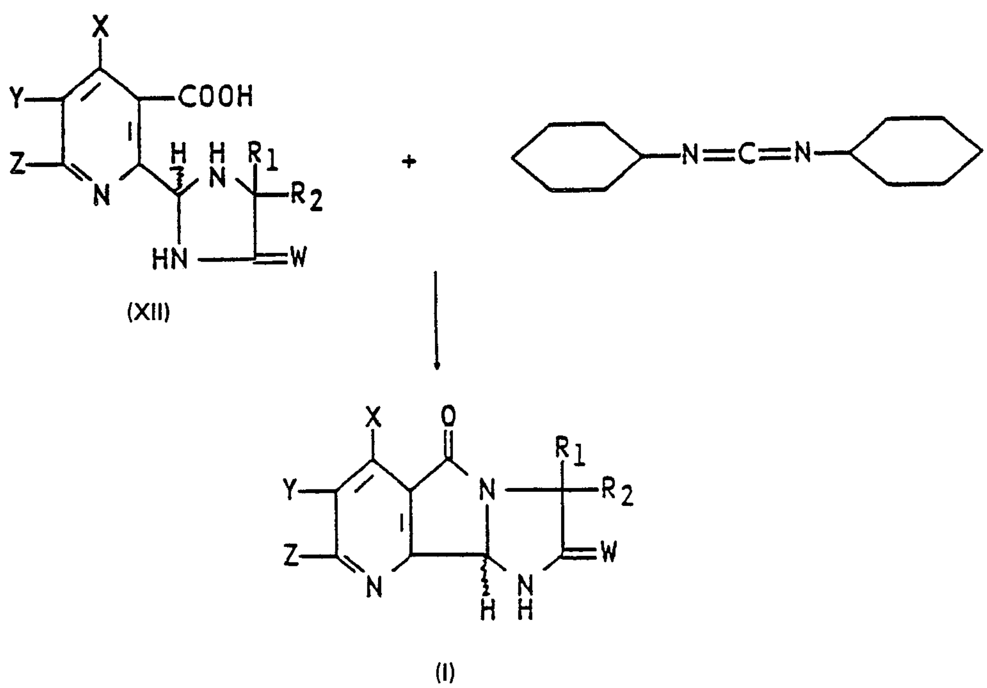

(XII)

(I)

wherein $R_1$, $R_2$, X, Y, Z and W are as described with respect to formula (I) above. It should be understood that if a *cis-*, *trans-* or mixture of *cis-* and *trans-*imidazolidinone is employed as starting material in the above reactions, then the *cis-*, *trans-* or mixture of *cis-* and *trans-* formula (I) compounds is obtained. These formula (I) dihydroimidazopyrrolopyridines, quinolines, thieno- and furopyridines and dihydrothieno- and furopyridines, are unexpectedly substantially more selective than their 2-(2-imidazolidinyl)acid precursors.

Many of the formula (I) dihydroimidazopyrrolopyridine, quinoline, thieno- and furopyridine and dihydrothieno- and furopyridine compounds of the present invention may also be prepared by reduction of the corresponding formula (XIV) 5*H*-imidazopyrrolopyridine, quinoline, thieno- or furopyridine, or dihydrothieno- or furopyridine, 2,5-dione. This reduction can be achieved by reaction of the formula (XIV) 2,5-dione with at least an equimolar amount of a reducing agent such as sodium or lithium borohydride in alcohol or aqueous alcohol or tetrahydrofuran or aqueous tetrahydrofuran, at a temperature between about −10 and +5°C. Acidification of the reaction mixture to a pH of about 3, using a strong mineral acid such as concentrated sulfuric acid, then yields the desired formula (I) product. The reaction may be graphically illustrated as follows:

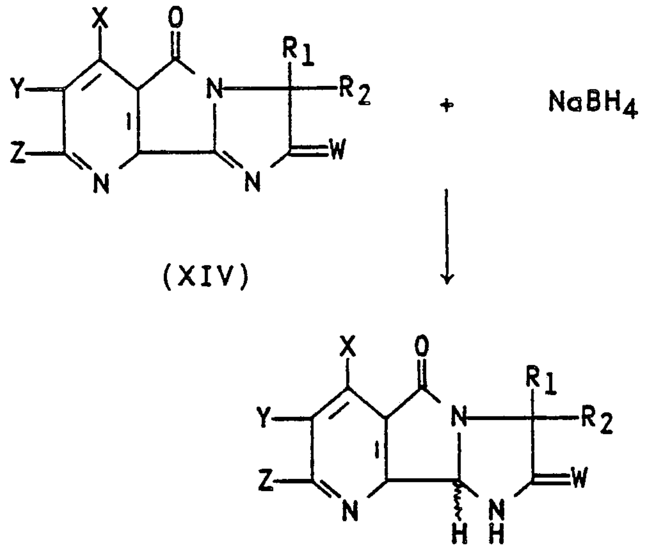

(XIV)

(I)

wherein X, Y, Z, W, $R_1$ and $R_2$ are as described with respect to formula (I) above.

This procedure usually forms a mixture of the *cis* and *trans*-isomers of the formula (I) compounds.

The preparation of the formula XIV 5*H*-imidazopyrrolopyridines and imidazopyrroloquinolines in which W is oxygen is described in the European Patent Application 0,041,623, published December 16, 1981.

In all the cases described above, the products from the reaction are those in which A is hydrogen. In order to prepare these compounds in which A is a group other than hydrogen, advantage is taken of the ability of the >C=N— function in the imidazopyraolopyridines, quinolines, thieno- and furopyridines to add a variety of nucleophiles such as alcohols, amines and thiols. The reaction may be graphically illustrated as follows:

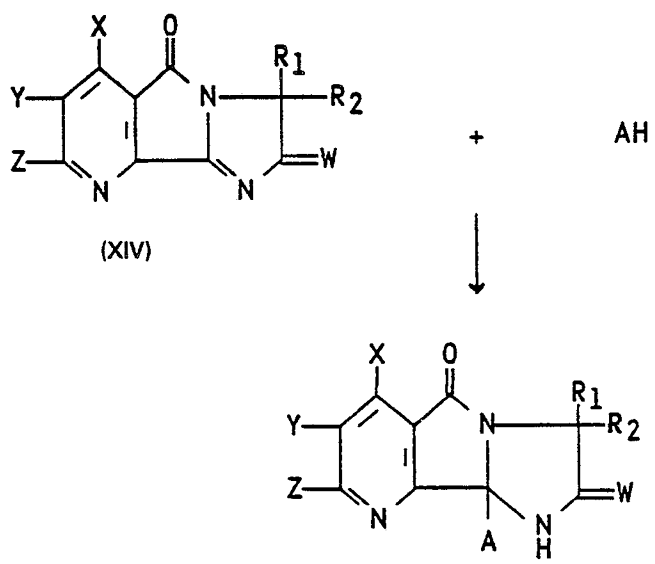

(XIV)

(I)

wherein AH is, for example, $CH_3OH$, $CH_3SH$, $CH_3NH_2$. This reaction may be catalyzed by acids such as *p*-toluenesulfonic acid or bases such as tertiary amines.

In practice it has been found that many substituted and unsubstituted aromatic and heteroaromatic imidazolidinone and imidazolidinethione compounds utilized as starting materials for the above described reactions for the preparation of compounds identified by formula (I) can be prepared by reduction of the corresponding formula (XV) imidazolinone or imidazolinethione with, for example, at least about an equimolar amount of sodium cyanoborohydride in the presence of a solvent such as $C_1$—$C_4$ aliphatic alcohol, aqueous alcoholic mixture or ether, followed by acidification to a pH between about 2.5 and 5 and preferably between 3 and 4, with a strong mineral acid such as hydrochloric acid, or an organic acid such as acetic or the like. This reduction is generally conducted at a temperature between 0 and 40°C and is particularly effective for treatment of 2-(2-imidazolinyl)nicotinic acids and esters, but preferably the methyl esters. It is likewise effective for reduction of the imidazolinyl function 2-(2-imidazolinyl)thieno and furo[3,2-*b*]pyridine-6-carboxylic acid esters and 2-(2-imidazolinyl)thieno and furo[2,3-*b*]pyridine-5-carboxylic acid esters.

The above described reduction may be graphically illustrated as follows:

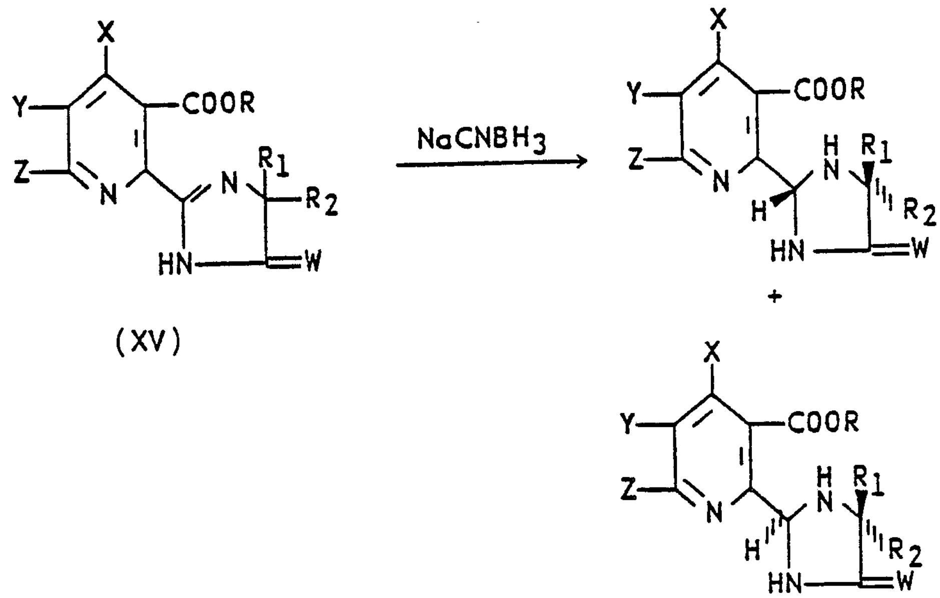


(XV)

*Cis*

*Trans*

wherein R may be hydrogen, but preferably a methyl group and $R_1$, $R_2$, W, X, Y and Z, are as defined in reference to said formula I. When R is a methyl group then this can advantageously be removed by reaction of the methyl ester in a $C_1$—$C_4$ aliphatic alcohol, preferably absolute methanol, and admixing therewith at least one equivalent of strong base.

As shown above, the imidazolidinones and imidazolidinethiones are obtained as a mixture of *cis*- and *trans*-isomers when $R_1$ and $R_2$ are not the same. These isomers are obtained in variable amounts. The mixtures are useful as such, but can frequently be separated chromatographically to give the pure *cis*- and *trans*-isomers, both of which are effective herbicidal agents.

Since the above-described reduction is not a universal method for the preparation of all substituted and unsubstituted aromatic and heteroaromatic imidazolidinones and imidazolidinethiones, a variety of synthetic routes have been explored in order to provide effective procedures for the manufacture of the imidazolidinones and imidazolidinethiones employed as starting materials for the preparation of the formula (I) compounds of this invention.

Accordingly, it has now been determined that both the oxo and thioxo derivatives of 2-(2-imidazolidinyl)nicotinates and 2-(2-imidazolidinyl)quinoline-3-carboxylates can be synthesized by heating to refluxing temperature, a mixture of a formula IX aminoamide or aminothioamide with about an equimolar amount of an appropriate formula X substituted or unsubstituted lower alkyl, 2-formylpyridine-3-carboxylate or 2-formylquinoline-3-carboxylate, in the presence of an inert organic solvent such as benzene, toluene, or the like, and a strong organic acid, such as *p*-toluenesulfonic acid, under a blanket of nitrogen. The thus-formed ester may then be converted to the corresponding formula (XII), acid, used as starting materials in the synthesis of the formula (I) compounds of the present invention, by dissolving or dispersing the imidazolidinone ester, or imidazolidinethione ester in a $C_1$—$C_4$ aliphatic alcohol, preferably absolute methanol and admixing therewith at least one equivalent of strong base.

In practice, the base is generally dissolved in water and the mixture heated to between about 20 and 50°C. The mixture is then cooled and adjusted to pH 6.5 to 7.5, and preferably about pH 7, with a strong mineral acid such as hydrochloric acid to yield the imidazolidinone or imidazolidinethione acid wherein $R_1$, $R_2$, $R_3$, W, X, Y and Z are as defined above. The reaction is illustrated in Flow Diagram (I).

FLOW DIAGRAM I

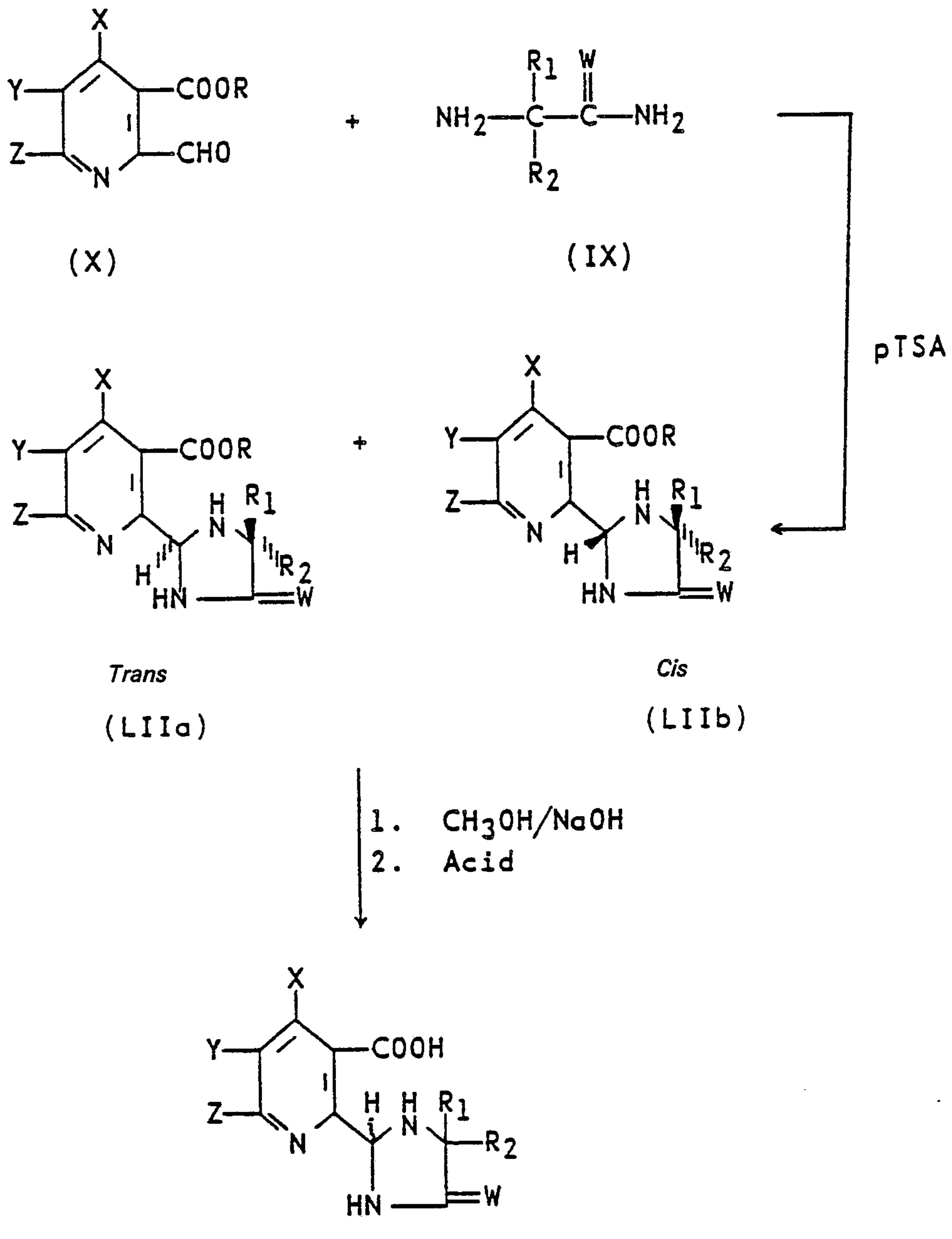


(X) (IX)

*Trans* (LIIa)

*Cis* (LIIb)

(XII)

wherein R is $C_1$—$C_4$ alkyl; and $R_1$, $R_2$, X, Y, Z and W are as described for formula (I) above.

The reaction of an aldehyde of formula (X) with an α-aminoamide or thioamide of formula (IX) under acid catalysis gives the corresponding Schiff's base as the initial product. Whether the Schiff's base of general formula

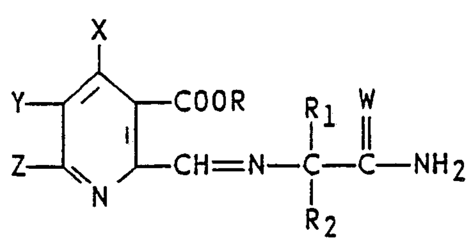

is isolated as such or cyclizes under the reaction conditions to the desired imidazolidinone depends on some unknown subtle factors. Nevertheless, if the Schiff's base is isolated it can, in a separate reaction, be cyclized with triflkuoroacetic acid to the imidazolidinone.

Substituted $C_1$—$C_4$ alkyl 2-formylnicotinates which are useful in the preparation of 2-(2-imidazolidinyl)-nicotinatic acids and esters by the aldehyde route described above and illustrated in Flow Diagram I, for synthesis of formula LIIa and LIIb substituted 5-oxo-2-imidazolidinyl nicotinates and the formula (XII) acids, can be prepared from substituted $C_1$—$C_{12}$ alkyl 2-methylnicotinates. For convenience and clarity, the following synthesis is described using substituted methyl 2-methylnicotinates as illustrative of this class of reactions.

In accordance with the process, equivalent amounts of a substituted methyl 2-methylnicotinate, represented by formula LIII and *m*-chloroperbenzoic acid are admixed in the presence of a chlorinated hydrocarbon such as methylene chloride, chloroform or the like. The reaction mixture is heated to refluxing temperature, then cooled to ambient temperature and excess peracid destroyed by addition of excess 1-hexane. Thereafter the solution is washed with sodium bicarbonate solution, dried and concentrated to give the corresponding substituted methyl methylnicotinate 1-oxide of formula LIV. The formula LIV 1-oxide is then heated to about 70 to 95°C with an excess of acetic anhydride to yield the formula LV substituted methyl 2-acetoxymethylnicotinate. A cosolvent such as pyridine or pyridine/dimethoxyethane may also be used in the reaction, but is not essential. Oxidation of the formula LV acetoxymethylnicotinate with hydrogen peroxide in acetic acid yields the methyl 2-acetoxymethylnicotinate 1-oxide represented by formula LVI. This 1-oxide is then readily converted to the formula LVII methyl 2-diacetoxymethylnicotinate by reaction with an excess of acetic anhydride at a temperature between about 70 and 95°C, with or without a cosolvent such as pyridine or pyridine/dimethoxyethane. Treatment of the formula LVII methyl diacetoxymethylnicotinate with an alkali metal alkoxide such as sodium methoxide, sodium ethoxide, potassium butoxide, or the like, in the presence of a $C_1$—$C_4$ aliphatic alcohol then yields the substituted alkyl formylnicotinate such as methyl 2-formylnicotinate of formula LVIII.

Alternatively, it has also been found that the reaction of a substituted $C_1$—$C_{12}$ alkyl 2-methylnicotinate, depicted by formula LIII, with benzaldehyde at an elevated temperature, yields the formula LIX methyl 2-styrylnicotinate which, when ozonized gives the formula LVIII substituted alkyl formylnicotinate.

Additionally, it has been found that treatment of the formula LV substituted methyl 2-acetoxymethylnicotinate with an alkali metal alkoxide such as sodium methoxide, in the presence of a lower aliphatic alcohol at an elevated temperature, yields the corresponding substituted methyl 2-hydroxymethylnicotinate of formula LX. The substituted methyl 2-hydroxymethylnicotinate is then converted to the formula LVIII substituted methyl formylnicotinate by oxidation with selenium dioxide or lead tetraacetate.

The above reactions are graphically illustrated in Flow Diagram II.

FLOW DIAGRAM II

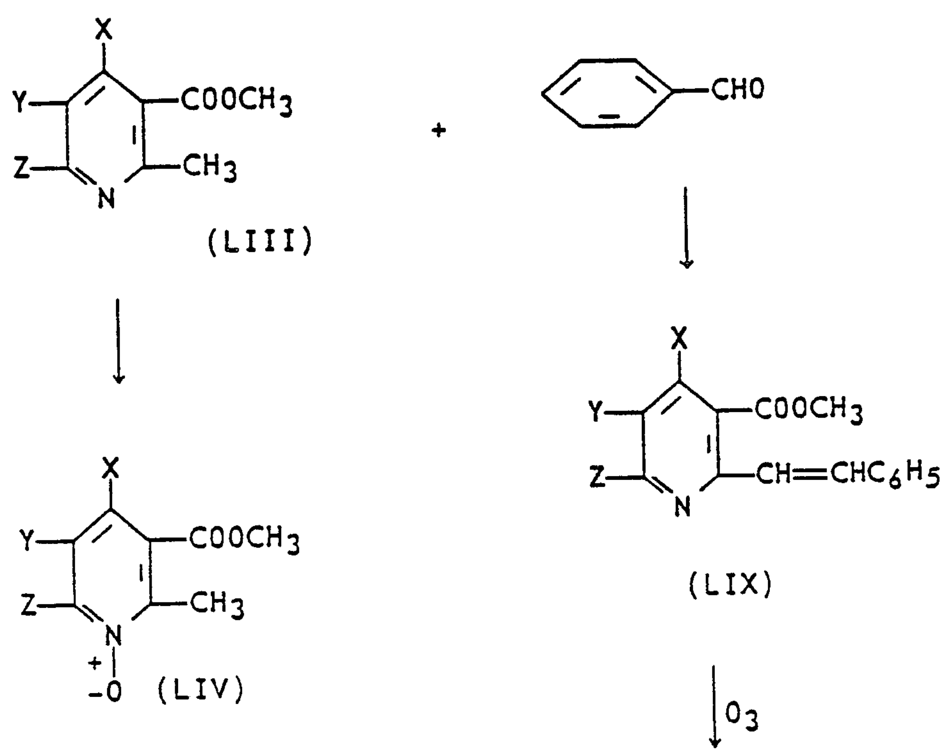

(LIII)
+
(LIX)
(LIV)

FLOW DIAGRAM II (Continued)

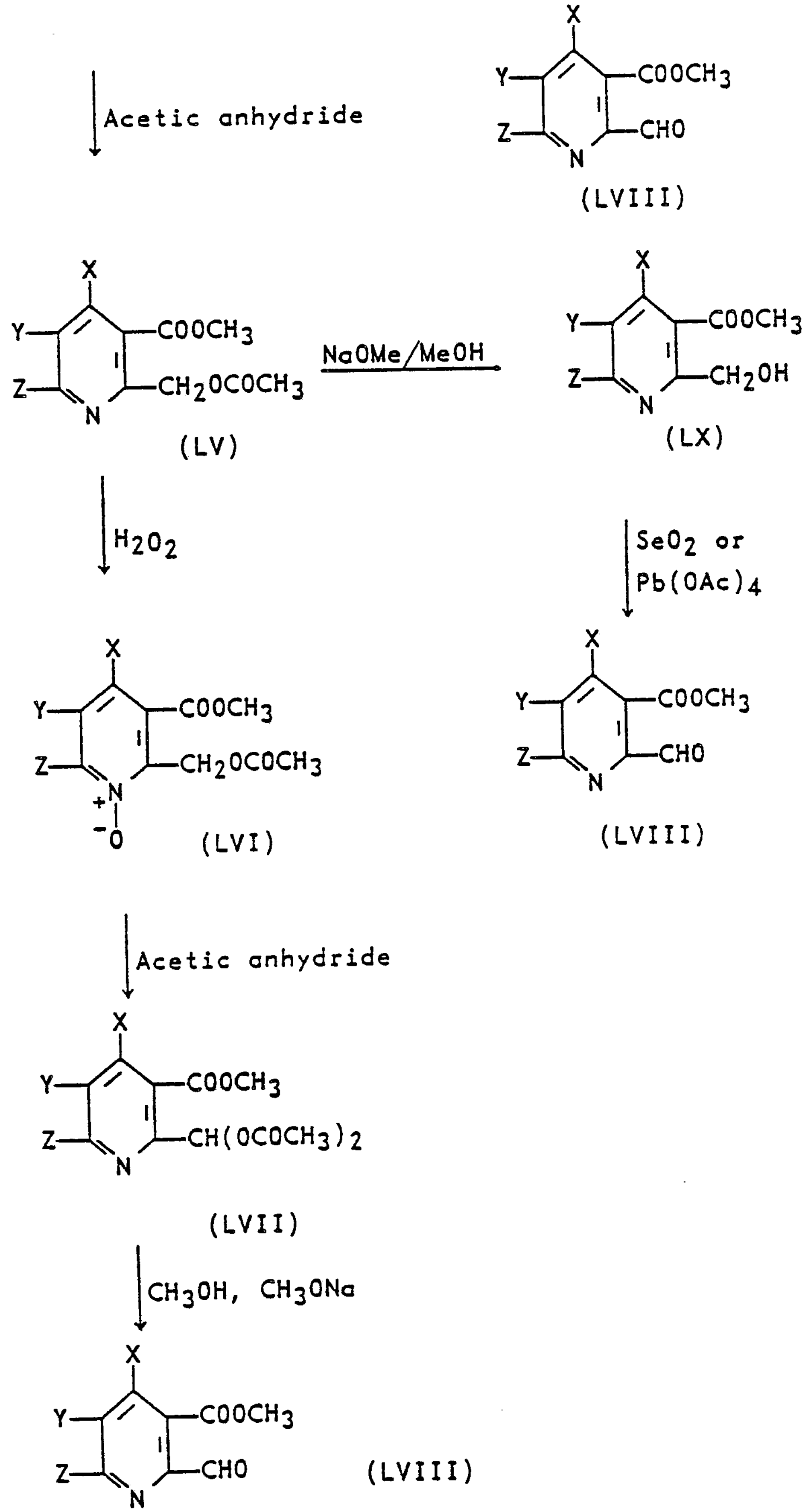


(LVIII)

(LV)

(LX)

(LVI)

(LVIII)

(LVII)

(LVIII)

The reduction of quinolinic acid diesters with diisobutylaluminum hydride is also an effective route to alkyl 3-formylnicotinates. The synthesis of these quinolinic acid diesters is described in European Patent Application 81103638.3, Publication Number 0 041 623.

The aldehyde route to the preparation of the formula LIIa and LIIb substituted (5-oxo(and thioxo)-2-imidazolidinyl)nicotinates is likewise effective for the preparation of the substituted and unsubstituted (5-oxo-2-imidazolidinyl)quinoline-3-carboxylates from the substituted 2-formylquinoline-3-carboxylates.

The process for the preparation of these substituted 2-formylquinoline-3-carboxylate intermediates involves the reaction of an appropriately substituted aniline, depicted by formula LXI:

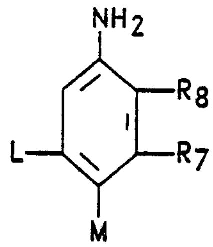

(LXI)

wherein L, M, $R_7$ and $R_8$ are as defined in reference to formula III quinolines; with approximately an equimolar amount of keto-ester depicted by formula LXII and having the structure:

$$R'—CO—CH_2COOR'' \quad \text{(LXII)}$$

wherein R′ is $CH_3$ or COOR″ and R″ is $C_1$—$C_4$ alkyl. This reaction is optionally conducted in the presence of an organic sulfonic acid such as *p*-toluenesulfonic acid hydrate, camphorsulfonic acid, or aniline hydrochloride, in the presence of an organic solvent such as cyclohexane, toluene, benzene, xylene, monochlorobenzene, orthodichlorobenzene and mixtures thereof, or the like at a temperature from about 20 to 110°C. It is preferred to continuously remove the water which is formed during the reaction by distillation either at atmospheric or under reduced pressures of as low as 50 mm of Hg while maintaining the reaction temperature in a range of 75 to 80°C. The reaction yields the β-anilino-α,β-unsaturated ester of formula LXIII i.e.,

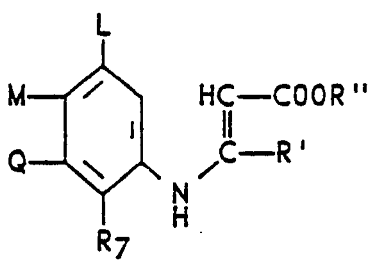

(LXIII)

wherein L, M, Q, $R_7$, R′, and R″ are as described above.

The thus-formed β-anilino-α,β-unsaturated ester of formula LXIII is then reacted with an approximately equimolar amount of an immunoium salt having the structure:

$$Cl—CH=\overset{\oplus}{N}—(R''')_2\cdot Cl^{\ominus}, \quad \text{(LXIV)}$$

wherein R‴ is $C_1$—$C_6$ alkyl or

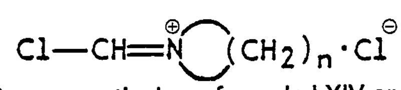

(LXIVa)

wherein n is 4 or 5, and referred to respectively as formula LXIV or LXIIa. The reaction is conducted in the presence of a hydrocarbon solvent such as toluene or a chlorinated hydrocarbon solvent such as methylene chloride, dichloroethane, orthodichlorobenzene, chlorobenzene, or mixtures thereof, at a temperature between about 40 and 110°C, for a period of time sufficient to essentially complete the reaction and yield the formula LXV alkyl ester of 2-methyl-3-quinolinecarboxylic acid, if R′ is $CH_3$ in the formula LXVII β-anilino-α,β-unsaturated ester or the quinoline-2,3-dicarboxylate if R′ is COOR″ in the formula LXVIII β-anilino-α,β-unsaturated ester.

Alternatively, the formula LXI substituted aniline, wherein L, M, $R_7$ and $R_8$ are as described above, can be reacted with about an equimolar amount of a formula LXVI acetylene dicarboxylate having the structure:

$$R''OOC—C{\equiv}C—COOR'',$$

where R'' is $C_1$—$C_4$ alkyl. This reaction is generally carried out in the presence of a solvent such as dichloroethane or a $C_1$—$C_4$ alcohol such as methanol, at a temperature between 0 and 100°C to yield a β-anilino-α,β-unsaturated ester as formula LXIII. The β-anilino-α,β-unsaturated ester of formula LXIII is then reacted with an ammonium salt depicted by formula LXIV having the structure:

$$Cl{-}CH{=}\overset{\oplus}{N}{-}(R''')_2\cdot Cl^{\ominus}$$

wherein R''' is $C_1$—$C_6$ alkyl or LXIVa having the structure:

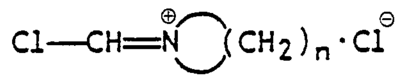

where n is 4 or 5. While the anion in formulas LXIV or LXIVa is shown as $Cl^{\ominus}$, it should be recognized that when $POCl_3$ is used to prepare the Vilsmeier reagent, the anion is $PO_2Cl_2^{\ominus}$. This reaction is generally conducted in the presence of a solvent such as methylene chloride, dichloroethane, monochlorobenzene, orthodichlorobenzene, or toluene at a temperature between 40 and 110°C for a period of time sufficient to complete the reaction and yield the quinoline-2,3-dicarboxylate shown as formula LXVa having the structure:

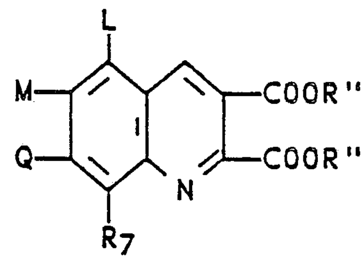

(LXVa)

wherein L, M, Q, $R_7$ and R'' are as described above.

The immonium salt formula LXIV or LXIVa utilized in the above cyclization reactions may, hereafter, be referred to as the Vilsmeier reagent. This reagent may be generated from a (N,N-dialkyl or N-alkyl, N-phenyl) formamide reaction with $POCl_3$, $COCl_2$, ClCO—COCl or $SOCl_2$ in a hydrocarbon or chlorinated hydrocarbon solvent.

Conversion of the 2-methyl-3-quinolinecarboxylate shown as formula LXV in which $R'=CH_3$ to the corresponding aldehyde of formula LXVII can be achieved in a manner similar to that described above for the conversion of the substituted 2-methylnicotinate of formula LIII to the corresponding 2-formylnicotinate of LVIII.

Conversion of the quinoline-2,3-dicarboxylate, shown as formulas LXV and LXVa, to the corresponding aldehyde shown as formula LXVII having the structure:

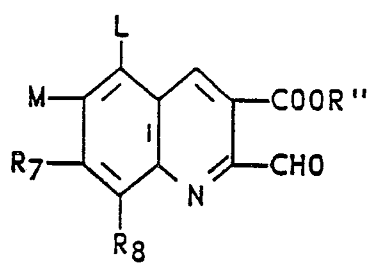

(LXVII)

where L, M, $R_7$, $R_8$ and R'' are as defined above, can be achieved by reaction of the formula LXV quinoline-2,3-dicarboxylate with diisobutylaluminum hydride. The reaction is preferably conducted in the presence of a non-protic solvent such as tetrahydrofuran under a blanket of inert gas.

These reactions are graphically illustrated in Flow Diagram III below.

FLOW DIAGRAM III

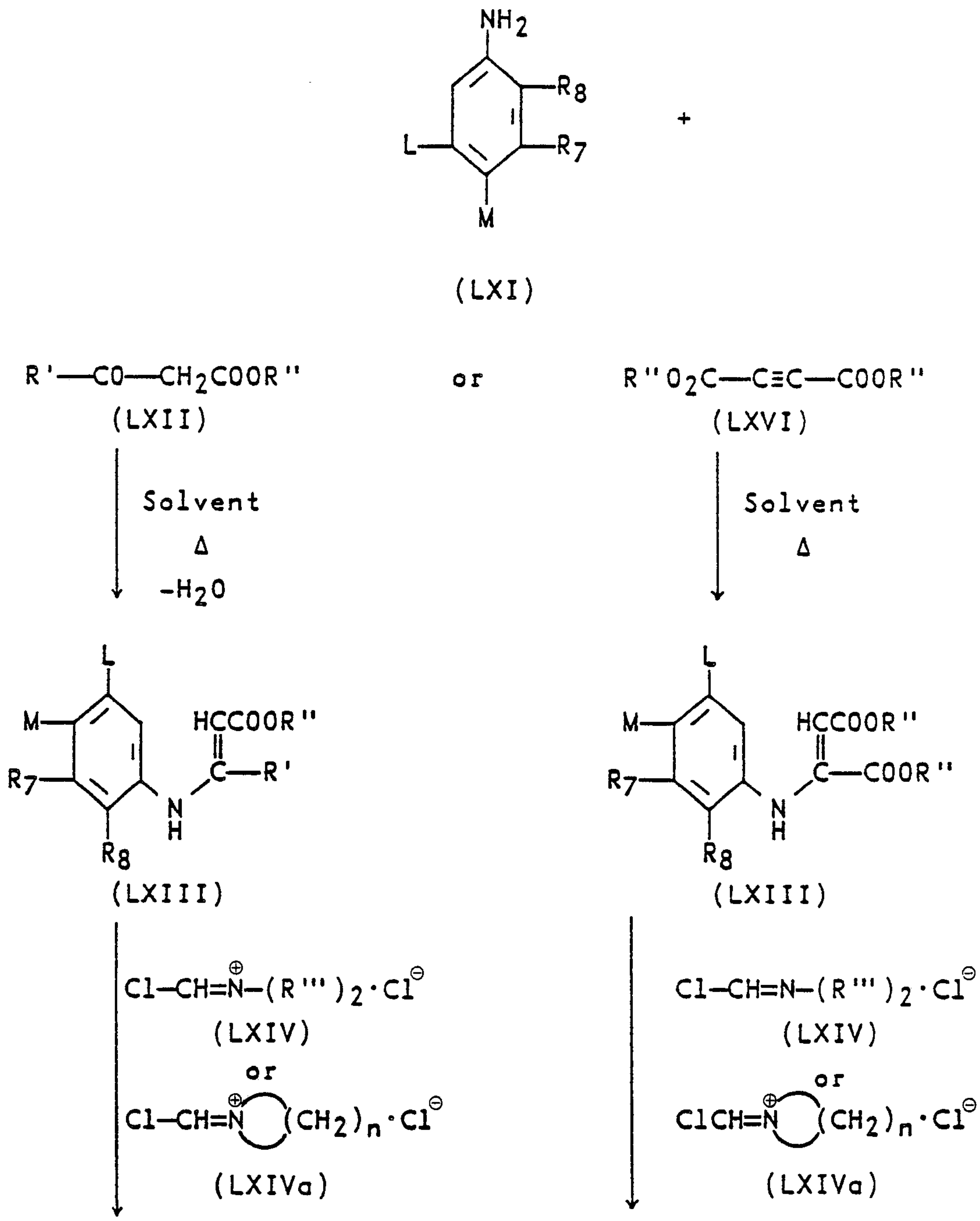


(LXI)

$R'—CO—CH_2COOR''$ (LXII) or $R''O_2C—C≡C—COOR''$ (LXVI)

(LXIII) (LXIII)

(LXIV) or (LXIVa)

(LXIV) or (LXIVa)

FLOW DIAGRAM III (Continued)

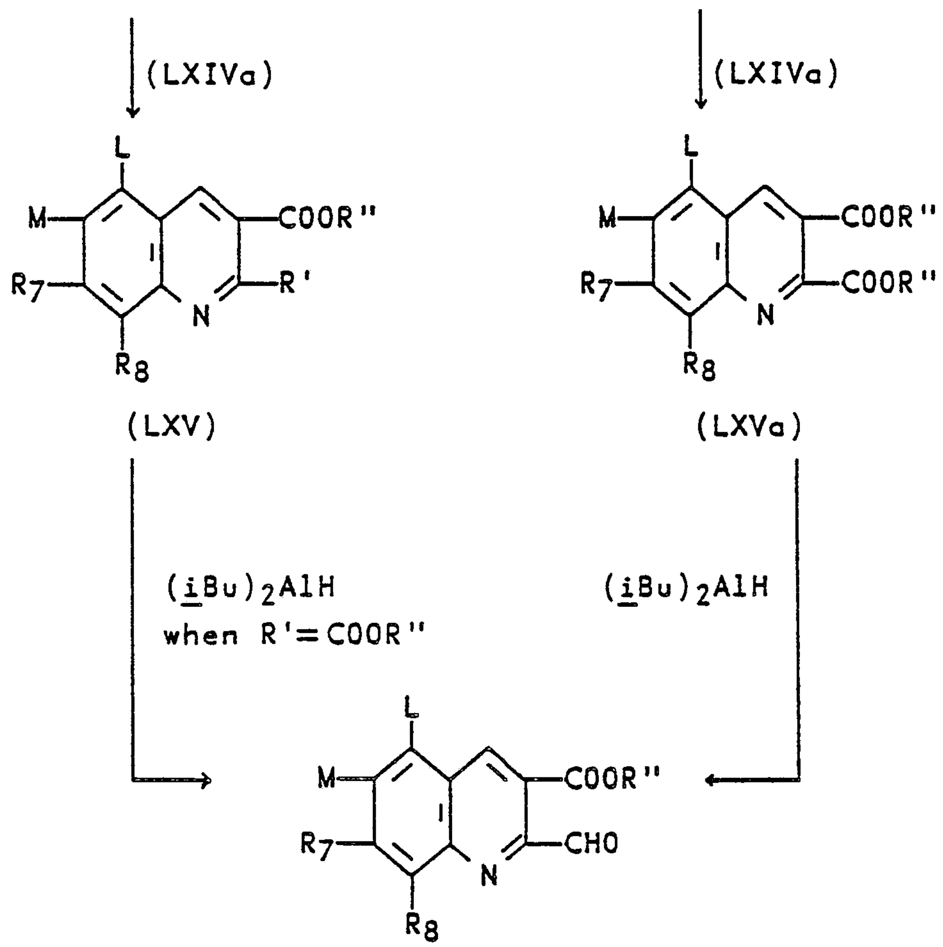


(LXV)

(LXVa)

(LXVII)

The 2-(2-imidazolidinyl)thieno- and furo[3,2-*b*]pyridine-6-carboxylates; 2-(2-imidazolidinyl)-2,3-dihydrothieno- and furo[3,2-*b*]pyridine-6-carboxylates; 2-(2-imidazolidinyl)thieno- and furo[2,3-*b*]pyridine-5-carboxylates and 2-(2-imidazolidinyl)-2,3-dihydrothieno- and furo[2,3-*b*]pyridine-5-carboxylates, useful as intermediates in the preparation of the formula (I) compounds of this invention can be obtained, by reduction of the corresponding (2-imidazolin-2-yl)-thieno- and furo[2,3-*b*] and [3,2-*b*]pyridines with sodium cyanoborohydride. These 2-(2-imidazolin-2-yl)-thieno- and furo[2,3-*b*] and [3,2-*b*]pyridine intermediates, necessary for the preparation of the formula V, VI, VII and VIII, thieno- and furopyridines, of the present invention are described in the copending application for United States Letters Patent or Marinus Los, David William Ladner and Barrington Cross, Serial No 500,219, filed June 2, 1983, and incorporated herein by reference thereto.

The 2-(2-imidazolin-2-yl)thieno and furo[2,3-*b*] and [3,2-*b*]pyridine intermediates, used in the synthesis of the 2-(2-imidazolidinyl)thieno- and furo[2,3-*b*] and [3,2-*b*]pyridines starting materials for the compounds of the present invention are depicted by formulas Va, VIa, VIIa and VIIIa; illustrated below.

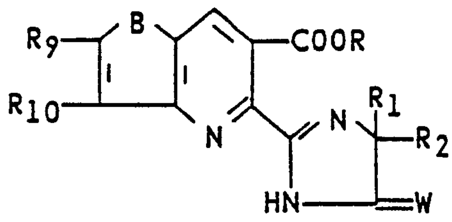

(Va)

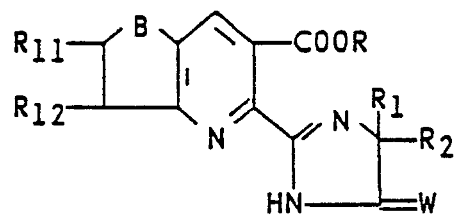

(VIa)

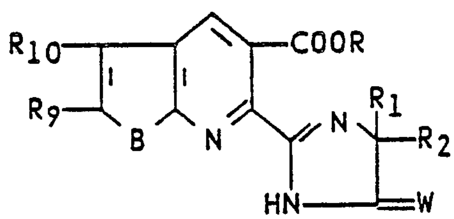

(VIIa)

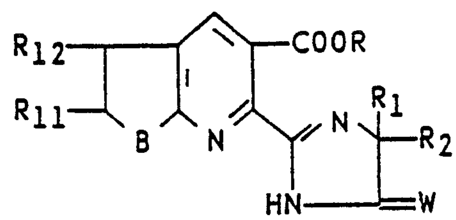

(VIIIa)

wherein R is hydrogen or $C_1$—$C_4$ and $R_1$, $R_2$, $R_9$, $R_{10}$, $R_{11}$, $R_{12}$, B and W are as described above in reference to compounds of formula V, VI, VII and VIII.

While for convenience, the imidazolinone and imidazolinethione intermediates referred to throughout are illustrated by single structures, it should be recognized that the imidazolinyl function in these compounds may exist in either tautomeric form, i.e.:

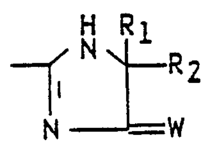

or

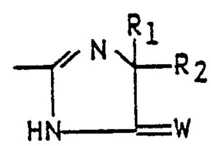

The formula Va, VIa, VIIa and VIIIa, intermediates for the compounds of the present invention may be prepared from the appropriately substituted thieno- and furo[2,3-*b*] and (3,2-*b*]pyridinedicarboxylic acids and esters of formulas LXXI and LXXIa illustrated below.

Since $R_9$ and $R_{10}$ represent substituents selected from hydrogen, halogen, $C_1$—$C_4$ alkyl and phenyl, and $R_{11}$ and $R_{12}$ represent hydrogen, $C_1$—$C_4$ alkyl and phenyl; for the purposes of the following discussion, which relates to the preparation of the formula Va, VIa, VIIa and VIIIa, 2-(2-imidazolin-2-yl)thieno and furo[2,3-*b*] and (3,2-*b*]pyridines, compound structures involved in the synthesis under discussion will be illustrated with $R_9$ and $R_{10}$.

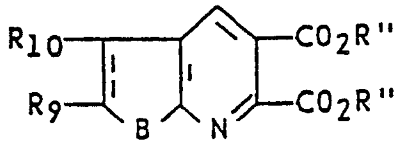

and

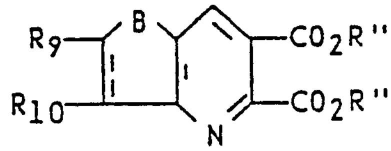

(LXXI) (LXXIa)

wherein $R_9$, $R_{10}$ and B are as previously described and R'' is methyl or ethyl.

Methods suitable for preparing formula Va, VIa, VIIa and VIIIa unsaturated compounds wherein — is a double bond from the formula (LXXI) and (LXXIa) pyridinedicarboxylic acid esters are illustrated in Flow Diagram IV below.

Thus formula (LXXI) and (LXXIa) diesters may be hydrolyzed to the corresponding thieno- and furo-2,3-pyridinedicarboxylic acids of formula (LXXII) and (LXXIIa) by reaction thereof with a strong base such as potassium hydroxide or sodium hydroxide. Acid anhydrides of formula (LXXIII) and (LXXIIIa) may then be prepared by treatment of the formula (LXXII) and (LXXIIa) pyridinedicarboxylic acids with, for example, acetic anhydride. Reaction of formula (LXXIII) and (LXXXIIIa) anhydrides with an appropriately substituted aminocarboxamide or aminothiocarboxamide depicted by formula (IX) yields carbamoyl nicotinic acids of formula (LXXIV) and (LXXIVa). Treatment of the thus-formed formula (LXXIV) and (LXXIVa) carbamoyl nicotinic acids with about 2 to 10 molar equivalents of aqueous or aqueous alcoholic sodium or potassium hydroxide, preferably under a blanket of inert gas such as nitrogen, cooling and acidifying to pH 2 to 4 with a strong mineral acid such as hydrochloric acid or sulfuric acid gives herbicidally effective 6-(4,4-disubstituted-5-oxo-(or thiono)-2-imidazolin- 2-yl)thieno- and furo[2,3-*b*]pyridine-6-carboxylic acids, and 5-(4,4-disubstituted-5-oxo-(or thiono)-2-imidazolin-2-yl)thieno- and furo[3,2-*b*]pyridine-6-carboxylic acids encompassed by formulas (Va) and (VIIa).

Formula (Va) and (VIIa) 5-(2-imidazolin-2-yl)thieno- and furopyridine esters, wherein R represents a substituent other than hydrogen or a salt-forming cation, and $R_1$, $R_2$, $R_9$, $R_{10}$ and B are as described above can be prepared by reacting a novel thieno- or furoimidazopyridinedione, represented by formulas (LXXV) and (LXXVa), hereinbelow, in Flow Diagram (V), with an appropriate alcohol and corresponding alkali metal alkoxide at a temperature ranging between about 20 and about 50°C.

Formula (LXXV) and (LXXVa) thieno- and furoimidazopyrrolopyridinediones may conveniently be prepared from formula (VIIa) and (Va) acids, where R is H by treatment with one equivalent of dicyclohexylcarbodiimide in an inert solvent such as methylene chloride as illustrated in Flow Diagram (V) below.

FLOW DIAGRAM (IV)

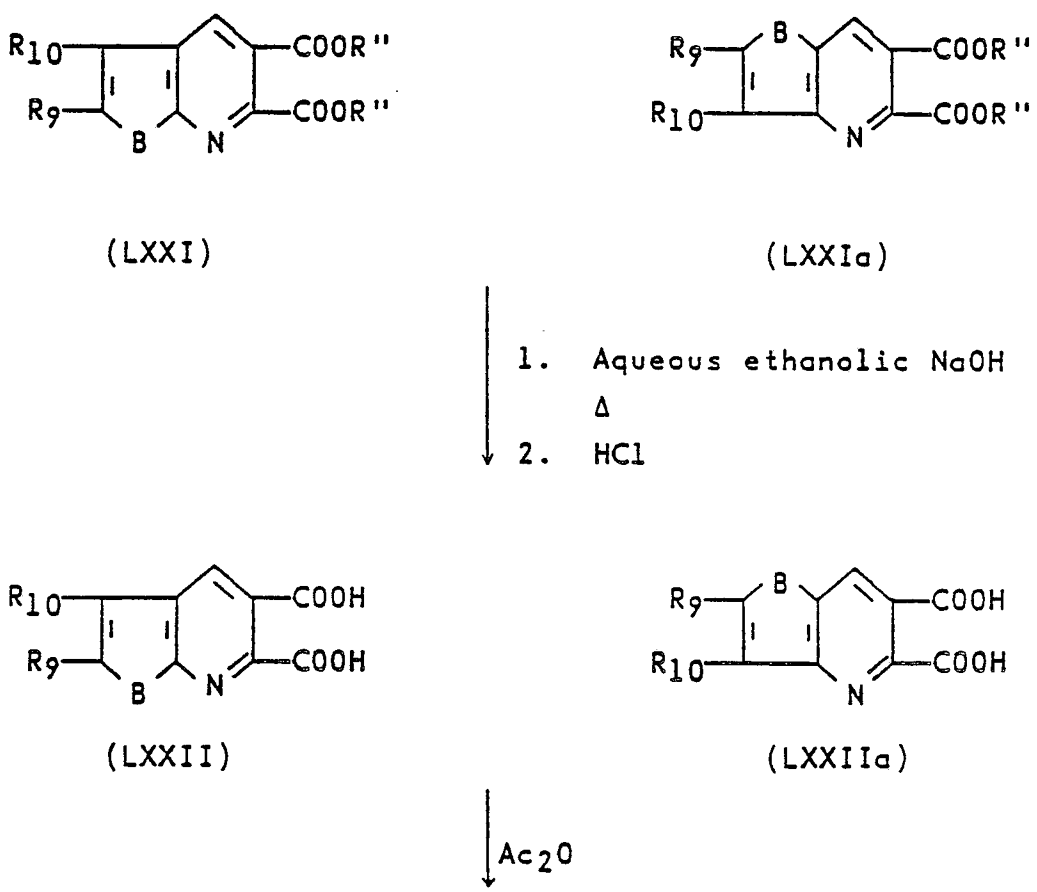


(LXXI)

(LXXIa)

(LXXII)

(LXXIIa)

FLOW DIAGRAM (IV) (Continued)

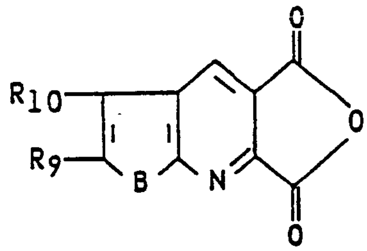

(LXXIII)

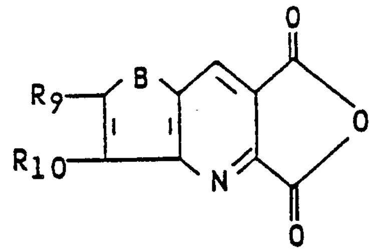

(LXXIIIa)

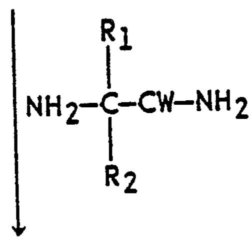

(IX)

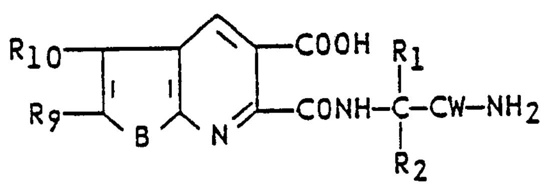

(LXXIV)

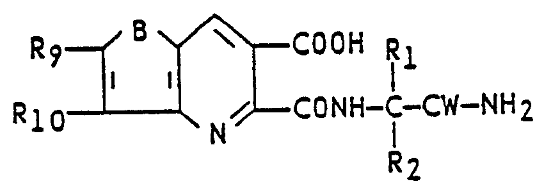

(LXXIVa)

NaOH

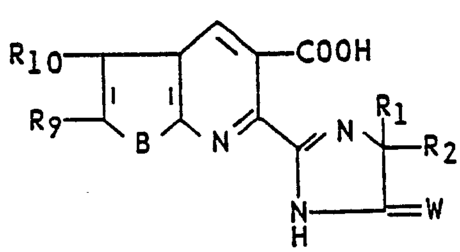

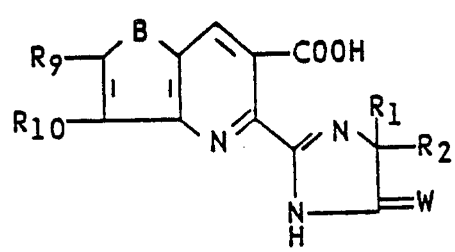

FLOW DIAGRAM (V)

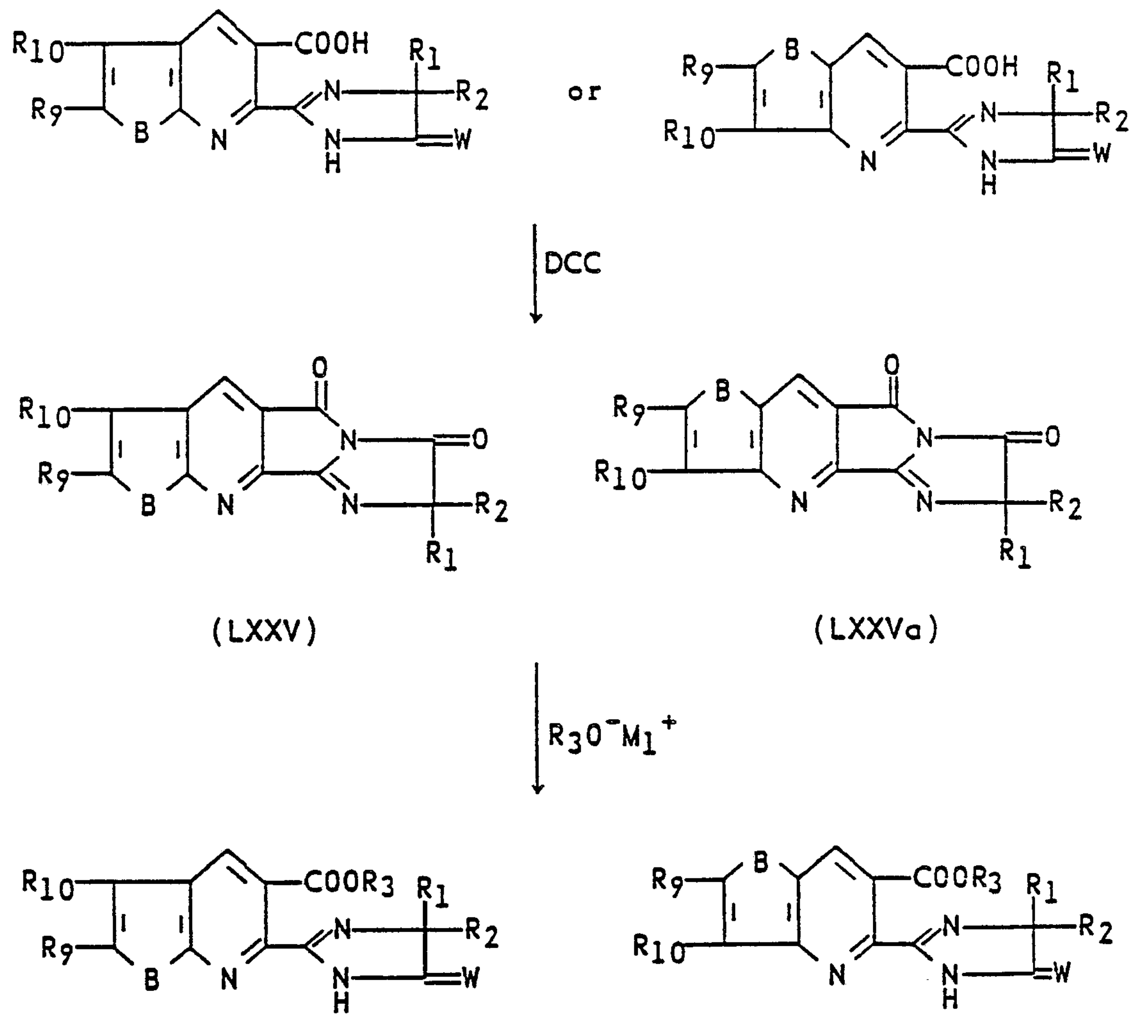


or

(LXXV) (LXXVa)

where $M_1$ is an alkali metal, and X, Y, Z, $R_1$, $R_2$ are as above defined and $R_3$ is $C_1$—$C_4$ alkyl.

Many formula (LXXI) thieno[2,3-*b*]pyridinedicarboxylic acids and (LXXIa) thieno[3,2-*b*]pyridinedicarboxylic acids may conveniently be prepared by reacting the appropriately substituted 2 or 3-aminothiophene of formula (LXXXIV) or (LXXXIVa) with a $C_1$—$C_4$ alkyl ester of acetylenedicarboxylic acid of formula (IX) as described by Bleckert et al. *Chem. Ber.* 1978, *106*, 368. The thus-formed β-aminothieno-α,β-unsaturated ester of formula (LXXXV) or (LXXXVa) is then reacted with an immonium salt depicted by the formula

$$Cl—CH=\overset{\oplus}{N}—(R''')_2\ \overset{\ominus}{Cl}$$

wherein R''' is $C_1$—$C_6$ alkyl or

$$Cl\text{-}CH=\overset{\oplus}{N}(CH_2)_{n'}\ \overset{\ominus}{Cl}$$

where n' is 4 or 5, in the presence of a low boiling chlorinated hydrocarbon solvent such as methylene chloride or dichloroethane at a temperature between about 40 and 90°C, for a period of time sufficient to essentially complete the reaction and yield the formula (LXXI) [2,3-*b*]thieno- or (LXXIa) [3,2-*b*]thieno-2,3-pyridinedicarboxylic acid as the dialkyl ester as illustrated in Flow Diagram (VI) below.

The furo[3,2-*b*]pyridinedicarboxylic acids may be prepared by reacting 3-amino-2-formylfuran of formula (LXXVI) prepared by the method of S. Gronowitz et al., *Acta Chemica Scand B29* 224 (1975) with ethyl oxalacetate to give the furopyridine compounds directly, as illustrated in Flow Diagram (VII) below

while the furo[2,3-*b*]pyridine compounds where $R_9$ and $R_{10}$ are H are obtained by bromination of the reaction product (LXXVII) of acetoacetamide with the diethyl ester of ethoxymethyleneoxalacetic acid followed by treatment with sodium borohydride and *para*-toluene sulfonic acid in refluxing xylene as illustrated in Flow Diagram (VIII) below.

FLOW DIAGRAM (VI)

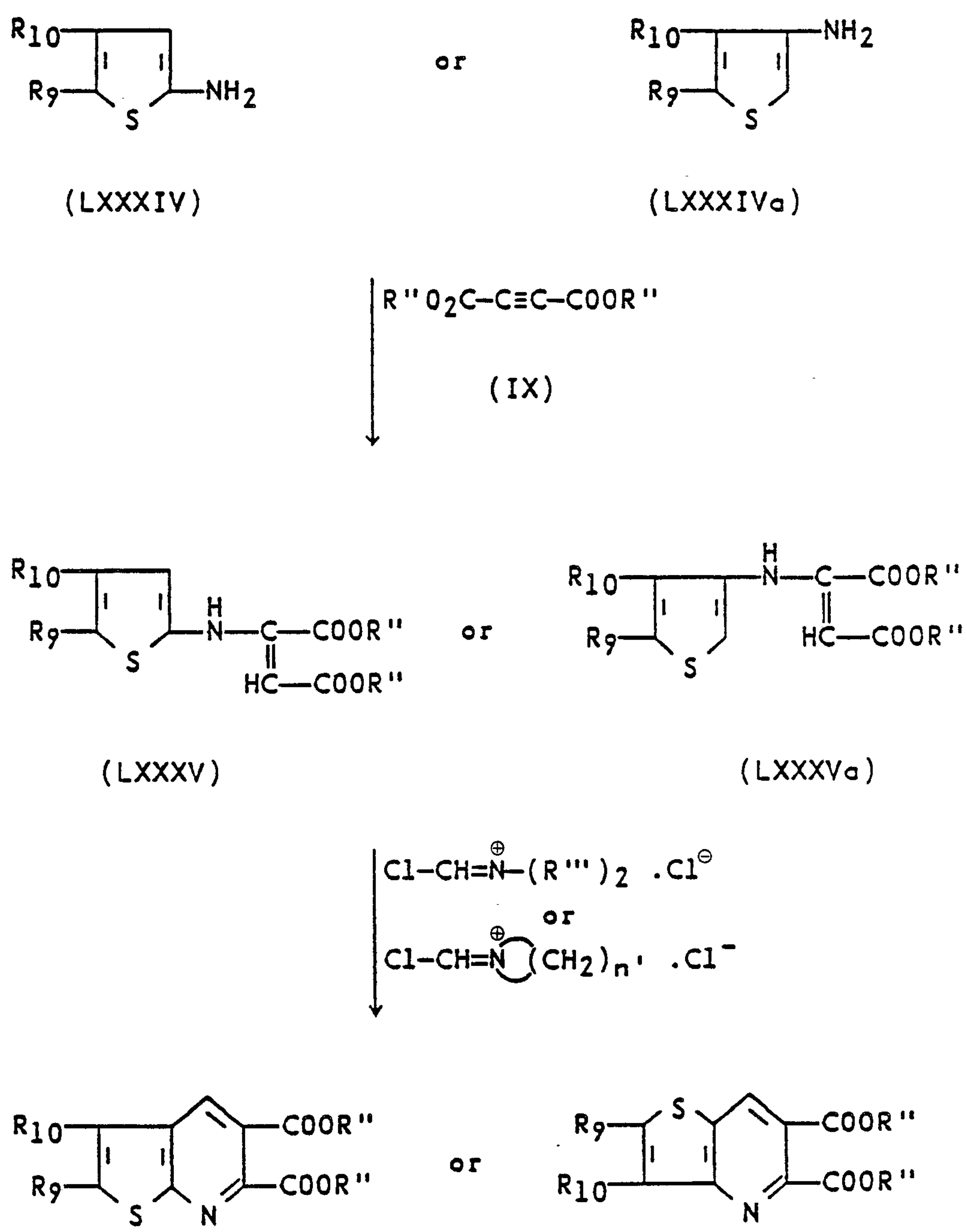


(LXXXIV) or (LXXXIVa)

(LXXXV) or (LXXXVa)

(LXXI) or (LXXIa)

FLOW DIAGRAM (VII)

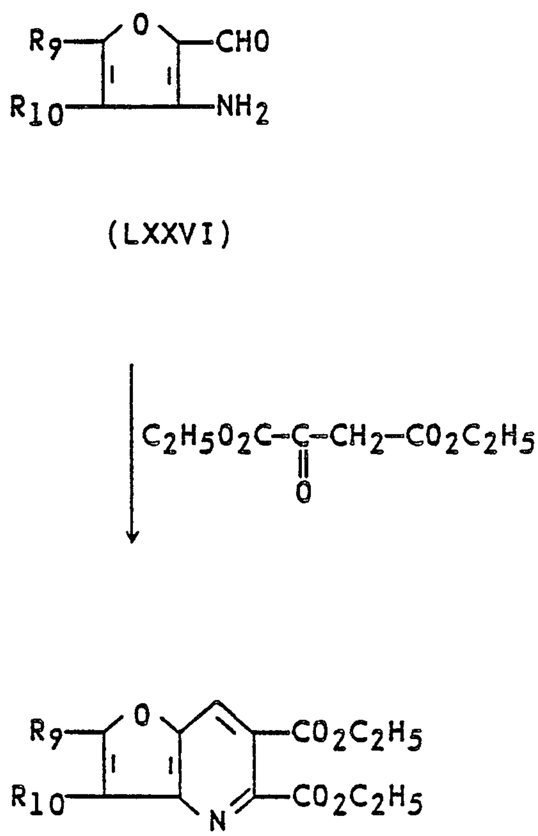

(LXXVI)

FLOW DIAGRAM (VIII)

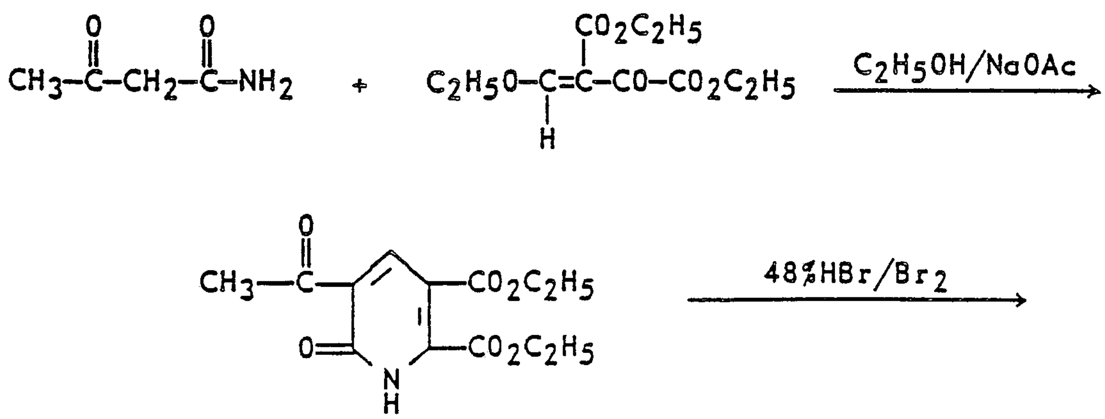


(LXXVII)

FLOW DIAGRAM (VIII) (Continued)

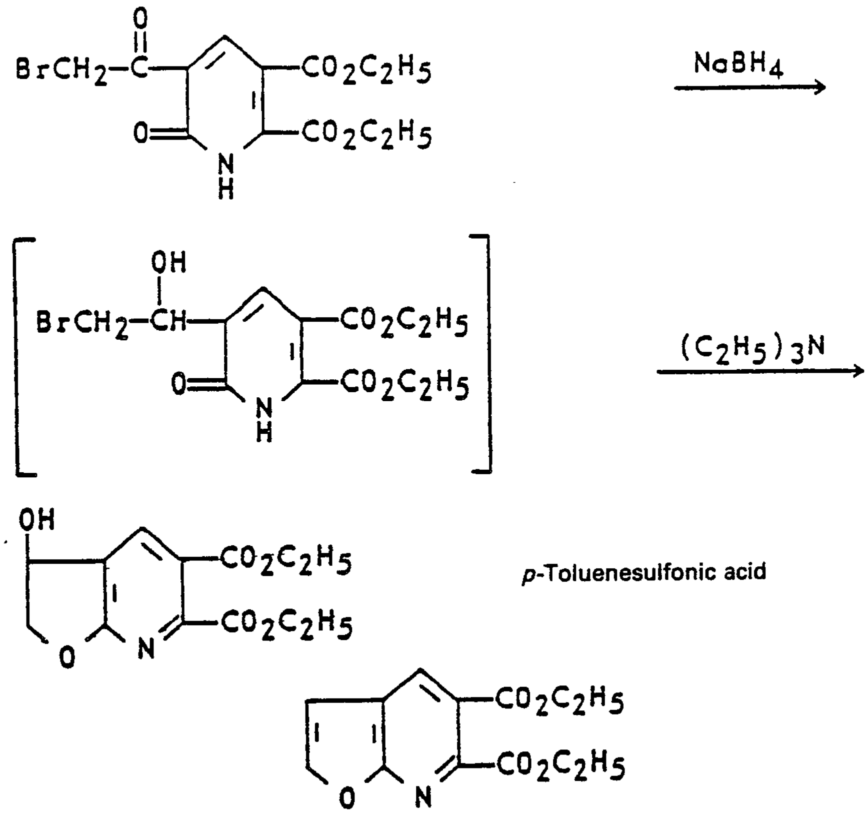


Substituents represented by $R_9$ and $R_{10}$ in formula (Va), (VIIa), (LXXV) and (LXXVa) compounds of the present invention may be prepared either by using the appropriately substituted starting material for the preparation of formula (LXXI) and (LXXIa) thieno- and furopyridine-5,6-dicarboxylic acid esters or by electrophilic substitution (halogenation, nitration, sulfonation, etc.) directly upon formula (LXXI) or (LXXIa) diesters or Formula (Va) or (VIIa) final products, wherein at least one of Y or Z is hydrogen. These substituted formula (LXXI), (LXXIa), (Va) and (VIIa) compounds then may be used as starting materials for additional $R_9$ and $R_{10}$ substitution by displacement, reduction, oxidation, etc. Representative substituted (LXXI) and (LXXIa) compounds which may be prepared by these procedures are as illustrated below.

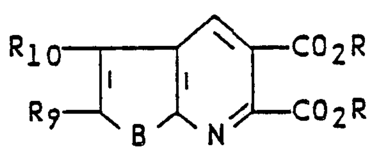

and

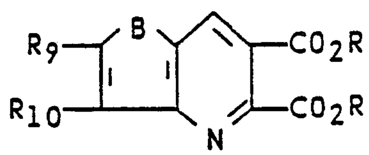

(LXXI) (LXXIa)

| B | $R_9$ | $R_{10}$ | R |
|---|---|---|---|
| S | H | H | $CH_3$ |
| S | H | Br | $CH_3$ |
| S | $CH_3$ | H | $CH_3$ |
| S | H | Cl | $CH_3$ |
| S | Cl | Cl | $CH_3$ |
| S | H | I | $CH_3$ |
| S | H | $NO_2$ | $CH_3$ |
| S | Br | Br | $CH_3$ |

| B | R9 | R10 | R |
|---|---|---|---|
| S | $CH_3$ | Cl | $CH_3$ |
| S | H | $CH_3$ | $CH_3$ |
| S | Cl | H | $CH_3$ |
| S | $CH_3$ | $CH_3$ | $CH_3$ |
| S | H | CN | $CH_3$ |
| S | H | $OCH_3$ | $CH_3$ |
| S | H | $N(CH_3)_2$ | $CH_3$ |
| S | H | $SCH_3$ | $CH_3$ |
| S | H | $OCF_2H$ | $CH_3$ |
| O | H | H | $C_2H_5$ |
| O | H | Br | $CH_3$ |
| O | H | Cl | $CH_3$ |
| O | $CH_3$ | H | $CH_3$ |
| O | $CH_3$ | H | $C_2H_5$ |
| O | H | $CH_3$ | $CH_3$ |
| O | $C_2H_5$ | H | $CH_3$ |
| O | H | $C_2H_5$ | $CH_3$ |
| O | $CH_3$ | $CH_3$ | $CH_3$ |
| S | $-(CH_2)_3-$ | | $CH_3$ |
| S | $-(CH_2)_4-$ | | $CH_3$ |
| S | $-(CH)_4-$ | | $CH_3$ |
| S | $C_6H_5$ | H | $CH_3$ |
| O | $C_6H_5$ | H | $CH_3$ |
| S | H | $SO_2N(CH_3)_2$ | $CH_3$ |
| S | H | $OC_6H_5$ | $CH_3$ |
| O | H | $OC_6H_5$ | $CH_3$ |
| O | $CF_3$ | H | $CH_3$ |

Additionally, novel herbicidal 2,3-dihydrothieno[2,3-*b*] and [3,2-*b*]pyridine compounds may be obtained by starting the sequence in Flow Diagram (VI) above with a dihydrothiophenimine hydrochloride. Novel herbicidal 2,3-dihydro furo[2,3-*b*] and [3,2-*b*]pyridines may be prepared by catalytic reduction of the formula (Va) or (VIIa) (2-imidazolin-2-yl) product, or (LXXI) and (LXXIa) furo[2,3-*b*] and [3,2-*b*]pyridine-5,6-diesters as for example with hydrogen and palladium on carbon, provided that $R_9$ and $R_{10}$ are substituents which are not reduced by such a procedure. This then provides novel 2,3-dihydro herbicidal compounds illustrated below.

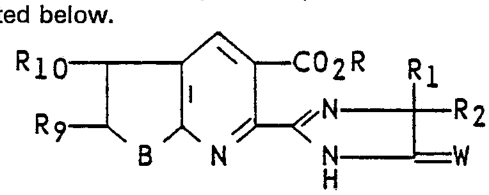

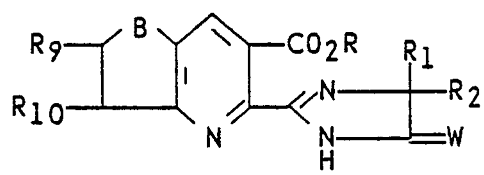

wherein $R_9$, $R_{10}$, B, W, $R_1$, $R_2$ and $R_B$ are as described for (Va) and (VIIA).

The formula I dihydroimidazopyrrolopyridines and derivatives of the present invention are highly effective preemergence and postemergence herbicidal agents, useful for the control of a wide variety of undesirable monocotyledonous and dicotyledonous plant species. Surprisingly, it has also been found that these formula I compounds are very active against a wide variety of weed species but well tolerated by a number of crops including: graminaceous crops such as sunflower, corn, rice, turf, and wheat; leguminous crops such as soybeans and other crops including cotton. While herbicidal selectivity of the formula I compounds of this invention may vary with compound structure from crop to crop, the presence of the dihydroimidazopyrrolopyridine function, which is unique to all of the formula I compounds of this invention, appears to impart significant herbicidal selectivity to said compounds. This selectivity thus permits application of the active compounds to newly planted fields or to maturing crops for control of undesirable grasses and broadleaf weeds in the presence of said crops.

It is also surprising to find that the compounds of this invention, frequently exhibit plant growth regulating activity when employed at non-herbicidal rates of application.

In practice, the formula I dihydroimidazopyrrolopyridines and derivatives thereof may be applied to the foliage of undesirable monocotyledonous or dicotyledonous plants or to soil containing seeds or other propagating organs of said plants such as tubers, rhizomes or stolons, at ranges generally between about 0.032 and 4.0 kg/ha, and preferably between about 0.063 and 2.0 kg/ha, although rates as high as 8.0 kg/ha may be used if desired.

Effective plant growth regulating activity such as dwarfing, antilodging, increased branching, increased tillering and the like, is generally obtained when the above-said formula I compounds are applied to crops at rates below herbicidal rates. Obviously, this rate will vary from compound to compound.

The formula I compounds of the present invention may be applied to the foliage of plants or to soil containing seeds or other propagating organs thereof, in the form of a liquid spray, as a ULV concentrate or as a solid formulation.

Formula I compounds may also be prepared as wettable powders, flowable concentrates, emulsifiable concentrates, granular formulations or the like.

A typical emulsifiable concentrate can be prepared by dissolving about 5 to 25% by weight of the active ingredient in about 65 to 90% by weight of N-methylpyrrolidone, isophorone, butyl cellosolve, methylacetate or the like and dispersing therein about 5 to 10% by weight of a nonionic surfactant such as an alkylphenoxy polyethoxy alcohol. This concentrate is dispersed in water for application as a liquid spray or it may be applied directly as an ultra low volume concentrate in the form of discrete droplets having a mass median diameter between about 17 and 150 microns particle size.

Wettable powders can be prepared by grinding together about 20 to 45% by weight of a finely divided carrier such as kaolin, bentonite, diatomaceous earth, attapulgite, or the like, 45 to 80% by weight of the active compound, 2 to 5% by weight of a dispersing agent such as sodium lignosulfonate, and 2 to 5% by weight of a nonionic surfactant, such as octylphenoxy polyethoxy ethanol, nonylphenoxy polyethoxy ethanol or the like.

A typical flowable liquid can be prepared by admixing about 40% by weight of the active ingredient with about 2% by weight of a gelling agent such as bentonite, 3% by weight of a dispersing agent such as sodium lignosulfonate, 1% by weight of polyethylene glycol and 54% by weight of water.

When the compounds of the invention are to be used as herbicides where soil treatments are involved, the compounds may be prepared and applied as granular products. Preparation of the granular product can be achieved by dissolving the active compound in a solvent such as methylene chloride, N-methylpyrrolidone or the like and spraying the thus-prepared solution on a granular carrier such as corncob grits, sand, attapulgite, kaolin or the like.

The granular product thus-prepared generally comprises about 3 to 20% by weight of the active ingredient and about 97 to 80% by weight of the granular carrier.

In order to facilitate a further understanding of the invention, the following examples are presented primarily for the purpose of illustrating certain more specific details thereof. The invention is not to be deemed limited thereby except as defined in the claims. Unless otherwise noted, all parts are by weight.

Example 1

Preparation of 7-ethyl-1,9bα(and β)-dihydro-3α-isopropyl-3-methyl-5*H*-imidazo[1′,2′:1,2]pyrrolo-[3,4-*b*]pyridine-2(3*H*),5-dione

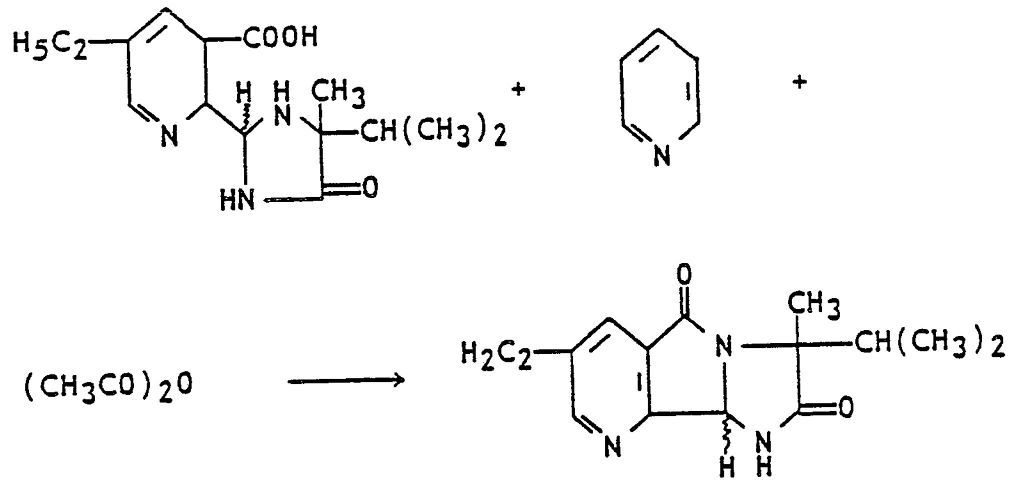


A suspension of 1 g of *cis*- and *trans*-5-ethyl-2-(4-isopropyl-4-methyl-5-oxo-2-imidazolidinyl)-nicotinic acid and 0.4 mL pyridine and 0.5 mL acetic anhydride in 10 mL acetonitrile is warmed to about 50°C until a clear solution is obtained. Upon cooling the solution to room temperature, a solid crystallizes. The solid is collected by filtration and recrystallized from ethyl acetate to give analytically pure 7-ethyl-1,9bα(and β)-dihydro-3α-isopropyl-3-methyl-5*H*-imidazo[1′,2′:1,2]pyrrolo[3,4-*b*]pyridine-2(3*H*),5-dione, mp 170—177°C.

Example 2

Preparation of 3,7-dimethyl-1,9bβ-dihydro-3α-isopropyl-3,5*H*-imidazo[1′,2′:1,2]pyrrolo[3,4-*b*]pyridine-2(3*H*),5-dione

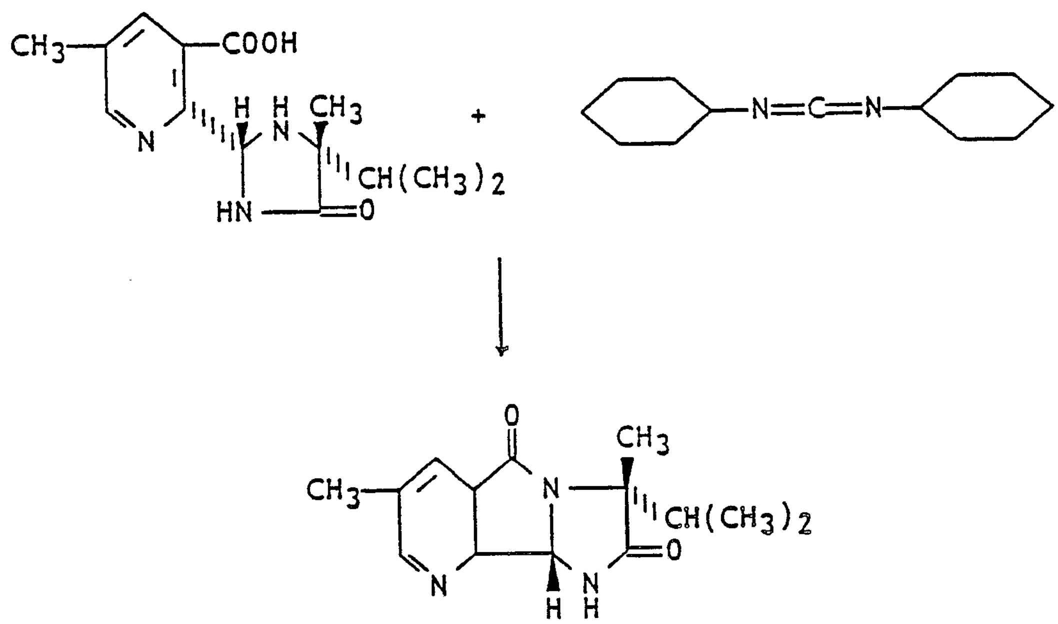

A mixture containing 1.08 g of *cis*-2-(4-isopropyl-4-methyl-5-oxo-2-imidazolidinyl)-5-methyl-nicotinic acid and 0.89 g of N,N′-dicyclohexylcarbodiimide in 25 mL methylene chloride is stirred at room temperature for 18 hours. The mixture is filtered and concentrated *in vacuo*. The crystalline residue is recrystallized from methylene chloride to give analytically pure 3,7-dimethyl-1,9bβ-dihydro-3α-isopropyl-3,5*H*-imidazo-[1′,2′:1,2]pyrrolo[3,4-*b*]pyridine-2(3*H*),5-dione, mp 191—193°C.

Example 3

Preparation of 1,9bα(and β)-dihydro-3α-isopropyl-3-methyl-5*H*-imidazo[1',2':1,2]pyrrolo[3,4-*b*]pyridine-2(3*H*),5-dione

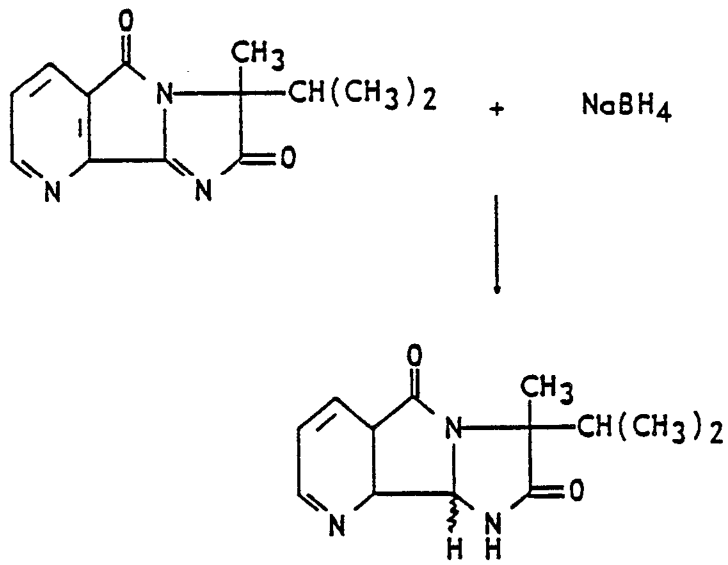

To a suspension of 1.7 g sodium borohydride in 120 mL absolute ethanol cooled to 0°C is added dropwise a solution of 10.8 g 3-isopropyl-3-methyl-5*H*-imidazo[1',2':1,2]pyrrolo[3,4-*b*]pyridine-2(3*H*),5-dione in 120 mL dry tetrahydrofuran, maintaining the temperature between 0 and 5°C. The mixture is stirred at room temperature for two hours and added to ice water. The aqueous mixture is acidified to pH 3 with concentrated sulfuric acid and extracted with methylene chloride. Extracts are dried and concentrated *in vacuo* to give a solid. The solid is washed with anhydrous ether and air dried to give 1,9*b*α(and β)-dihydro-3α-isopropyl-3-methyl-5*H*-imidazo[1',2':1,2]pyrrolo[3,4-*b*]pyridine-2(3*H*),5-dione, mp 170°C (dec).

Example 4

Preparation of formula (I) dihydroimidazopyrrolopyridines or derivatives thereof

Following one of the procedures described in Examples 1, 2 or 3, the compounds of formula (I), reported in Table I below, were prepared.

TABLE I

Formula (I) compounds of the invention having the *cis* and/or *trans* structure illustrated below

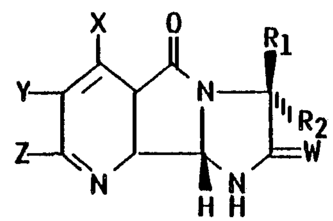

*cis*

and/or

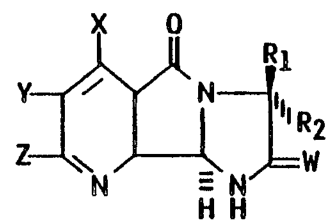

*trans*

| (3RS) | (3R) | $R_1$ | $R_2$ | X | Y | Z | W | cis | trans | mp°C |
|---|---|---|---|---|---|---|---|---|---|---|
| ✓ | | $CH_3$ | $C_2H_5$ | H | H | H | O | 36 | 64 | 192.0 - 107.0 |
| ✓ | | $CH_3$ | $CH(CH_3)_2$ | H | H | H | O | 25 | 75 | 170.0 (dec) |
| ✓ | | $CH_3$ | $CH(CH_3)_2$ | H | $CH_3$ | H | O | ✓ | | 191.0 - 193.0 |
| | ✓ | $CH_3$ | $CH(CH_3)_2$ | H | H | H | O | ✓ | | 200.0 - 205.0 |
| ✓ | | $CH_3$ | $CH(CH_3)_2$ | H | H | H | O | ✓ | | 206.0 - 217.5 |
| ✓ | | $CH_3$ | $CH(CH_3)_2$ | H | H | $CH_3$ | O | ✓ | | 202.0 - 217.0 |

TABLE I (Continued)

| (3RS) | (3R) | $R_1$ | $R_2$ | X | Y | Z | W | cis | trans | mp°C |
|---|---|---|---|---|---|---|---|---|---|---|
| ✓ | | $CH_3$ | $CH(CH_3)_2$ | H | H | $CH_2$=CH-$CH_2$O | O | ✓ | | 185.0 - 187.0 |
| ✓ | | $CH_3$ | $CH(CH_3)_2$ | H | H | H | O | | ✓ | 231.0 - 234.0 |
| ✓ | | $CH_3$ | $CH(CH_3)_2$ | H | $C_2H_5$ | H | O | 50 | 50 | 170.0 - 177.0 |
| ✓ | | $CH_3$ | $CH(CH_3)_2$ | H | H | $CH_3O$ | O | ✓ | | 215.0 - 219.0 |
| ✓ | | $CH_3$ | $CH(CH_3)_2$ | H | H | Cl | O | ✓ | | 208.0 - 222.0 |
| ✓ | | $CH_3$ | $CH(CH_3)_2$ | H | $C_2H_5$ | H | O | ✓ | | 200.0 - 203.0 |
| ✓ | | $CH_3$ | $CH(CH_3)_2$ | H | $C_2H_5$ | H | O | | ✓ | 220.0 - 222.0 |
| ✓ | | $CH_3$ | $CH(CH_3)_2$ | H | H | H | S | ✓ | | 144.0 - 146.0 |
| ✓ | | $CH_3$ | $CH(CH_3)_2$ | H | $CH_3$ | H | S | ✓ | | 225.0 - 230.0 |
| ✓ | | $CH_3$ | $CH(CH_3)_2$ | H | $CH_3$ | H | S | | ✓ | 243.0 - 249.0 |
| ✓ | | $CH_3$ | $CH(CH_3)_2$ | H | -CH=CH-CH=CH- | | O | | ✓ | 229.0 - 251.0 |
| ✓ | | $CH_3$ | $CH(CH_3)_2$ | H | $CH_3O$- | H | O | ✓ | | 202.0 - 204.0 |

TABLE I (Continued)

| (3RS) | (3R) | $R_1$ | $R_2$ | X | Y | Z | W | cis | trans | mp°C |
|---|---|---|---|---|---|---|---|---|---|---|
| ✓ | | $CH_3$ | $CH(CH_3)_2$ | H | Cl | H | O | ✓ | | 209.0 - 211.0 |
| ✓ | | $CH_3$ | $CH(CH_3)_2$ | H | H | H | S | | ✓ | 252.0 - 256.0 |
| ✓ | | $CH_3$ | $CH(CH_3)_2$ | H | $CH_3$ | H | O | 33 | 66 | 170.0 - 183.0 |
| | ✓ | $CH_3$ | $CH(CH_3)_2$ | H | $CH_3$ | H | O | 26 | 74 | 160.0 - 163.0 |
| ✓ | | $CH_3$ | $CH(CH_3)_2$ | H | $OCH_2CH=CH_2$ | H | O | ✓ | | 189.0 - 191.0 |
| ✓ | | $CH_3$ | $CH(CH_3)_2$ | H | $CH_3$ | H | O | | ✓ | 208.0 - 209.0 |
| | ✓ | $CH_3$ | $CH(CH_3)_2$ | H | $CH_3$ | H | O | | ✓ | 194.5 - 195.5 |
| | ✓ | $CH_3$ | $CH(CH_3)_2$ | H | $CH_3$ | H | S | ✓ | | 227.0 - 229.0 |
| ✓ | | $CH_3$ | $CH(CH_3)_2$ | H | H | 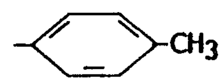 | O | ✓ | | 241.0 - 243.0 |
| ✓ | | $CH_3$ | $CH(CH_3)_2$ | H | H | 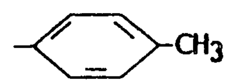 | O | | ✓ | 271.0 - 272.0 |
| ✓ | | $CH_3$ | $CH(CH_3)_2$ | H | H | $N(CH_3)_2$ | O | ✓ | | 250.0 - 253.0 |

Example 4-A

Preparation of 3-isopropyl-3-methyl-5*H*-imidazo[1′,2′:1,2]pyrrolo[3,4-*b*]pyridine-2(3*H*),5-dione

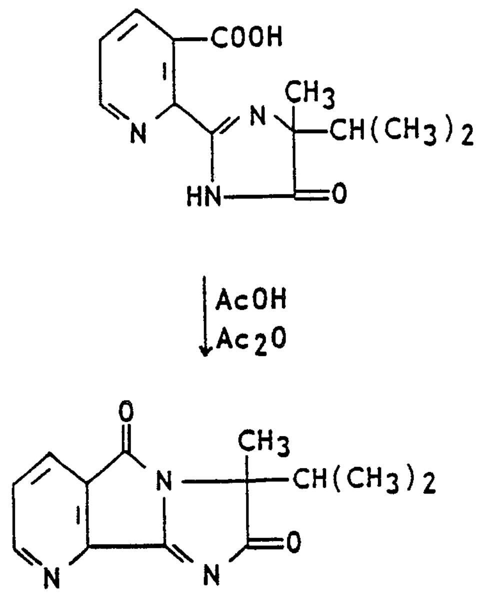


A stirred mixture of 123.2 g acid in 300 mL acetic acid and 89 mL acetic anhydride is heated at reflux for four hours. The mixture is concentrated *in vacuo* and the residue dissolved in toluene and again concentrated. The residue is slurried in hexane, filtered and washed several times with hexane to give a 90% of product, mp 105—112°C. NMR and Gc analysis indicates this material to consist of 80—87% of the desired 2,5-dione and the balance of the material being the corresponding 3,5-dione.

Example 4-B

Preparation of 1,9b-dihydro-3a-isopropyl-3-methyl-9bα-propoxy-5*H*-imidazo[1′,2′:1,2]pyrrolo-[3,4-*b*]pyridine-2(3*H*),5-dione

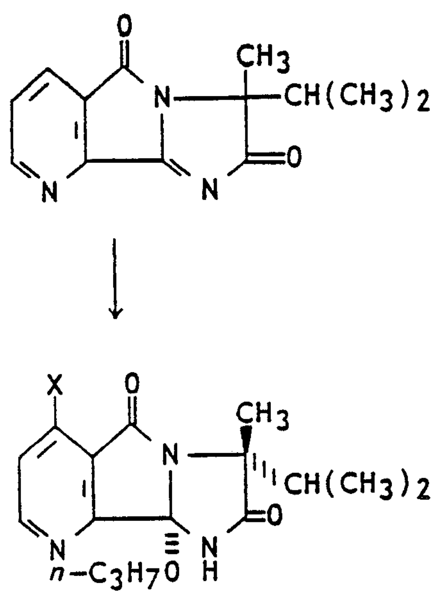

The solution containing 54 g dione in 200 mL *n*-propanol is heated at reflux for one hour. The solution is slowly cooled to 0°C and after one hour, the white crystals removed by filtration. The solids are washed with hexane and dried to give 41.6 of the 9*b*α-propoxy derivative mp 129—132°C. This material can be recrystallized from ether-hexane to give analytically pure material mp 135—137.5°C. This product can also be obtained by running the reaction at room temperature.

Using essentially the same conditions but utilizing the appropriate nucliophile and the appropriate 2,5-dione, the following dihydro derivatives are prepared.

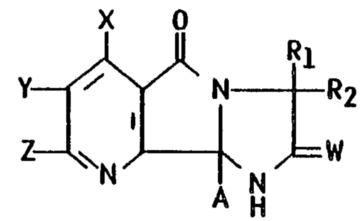

| $R_1$ | $R_2$ | W | A | X | Y | Z | mp°C | |
|---|---|---|---|---|---|---|---|---|
| $CH_3$ | $C_2H_5$ | O | $OCH_2C_6H_5$ | H | H | H | 135 - 139 | cis |
| $CH_3$ | $C_2H_5$ | O | $OCH_2C_6H_5$ | H | H | H | 135 - 145 | trans |
| $CH_3$ | $C_2H_5$ | O | $OCH_2CH=CH_2$ | H | H | H | 145 - 148 | trans |
| $CH_3$ | $CH(CH_3)_2$ | O | $OCH_3$ | H | H | H | 130 - 139 | trans |
| $CH_3$ | $CH(CH_3)_2$ | O | $OC_3H_6$-n | H | H | H | 135 - 137.5 | trans |
| $CH_3$ | $CH(CH_3)_2$ | O | $NHCH_2C{\equiv}CH$ | H | H | H | 168 - 169 | trans |
| $CH_3$ | $CH(CH_3)_2$ | O | $OCH_2$- 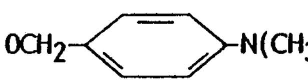 -$N(CH_3)_2$ | H | H | H | 163 - 164.5 | trans |
| $CH_3$ | $CH(CH_3)_2$ | O | $NH_2$ | H | H | H | 165 - 167 | trans |
| $CH_3$ | $CH(CH_3)_2$ | O | $OCH_2C_6H_5$ | H | H | H | 133 - 135 | trans |
| $CH_3$ | $CH(CH_3)_2$ | O | $C_2H_5$ | H | H | H | 142 - 144 | trans |
| $CH_3$ | $CH(CH_3)_2$ | O | $OCH_3$ | H | $-(CH_2)_5-$ | | 173 - 175 | trans |
| $CH_3$ | $CH(CH_3)_2$ | S | $OCH_3$ | H | H | H | 161 - 163 | trans |
| $CH_3$ | $CH(CH_3)_2$ | O | $SCH_3$ | H | H | H | 147 - 149 | |
| $Ch_3$ | $CH(CH_3)_2$ | S | $OCH_3$ | H | -CH=CH-CH=CH- | | 232 - 233 | |
| $CH_3$ | $CH(CH_3)_2$ | S | OH | H | H | H | 162 - 165 | |

Example 5

Postemergence herbicidal evaluation of test compounds

The postemergence herbicidal activity of the compounds of the present invention is demonstrated by the following tests, wherein a variety of monocotyledonous and dicotyledonous plants are treated with test compounds dispersed in aqueous acetone mixtures. In the tests, seedling plants are grown in jiffy flats for about two weeks. The test compounds are dispersed in 50/50 acetone/water mixtures containing 0.5% TWEEN® 20, a polyoxyethylene sorbitan monolaurate surfactant of Atlas Chemical Industries, in sufficient quantities to provide the equivalent of about .016 to 10.0 kg per hectare of active compound when applied to the plants through a spray nozzle operating at 40 psig for a predetermined time. After spraying, the plants are placed on greenhouse benches and are cared for in the usual manner, commensurate with conventional greenhouse practices. From four to five weeks after treatment, the seedling plants, are examined and rated according to the rating system provided below. The data obtained are recorded in Table V below.

| Rating System | % Difference in Growth from the Check |
|---|---|
| 0 — No Effect | 0 |
| 1 — Possible effect | 1—10 |
| 2 — Slight effect | 11—25 |
| 3 — Moderate effect | 26—40 |
| 5 — Definite injury | 41—60 |
| 6 — Herbicidal effect | 61—75 |
| 7 — Good herbicidal effect | 76—90 |
| 8 — Approaching complete kill | 91—99 |
| 9 — Complete kill | 100 |
| 4 — Abnormal growth, that is, a definite physiological malformation but with an over-all effect less than a 5 on the rating scale. | |

In most cases the data are for a single test, but in several instances, they are average values obtained from more than one test.

Plant Species Used

| | |
|---|---|
| Barnyardgrass | (*Echinochloa crusgalli*) |
| Green foxtail | (*Setaria viridis*) |
| Purple Nutsedge | (*Cyperus rotundus* L.) |
| Ragweed | (*Ambrosia* artemisiifolia, L.) |
| Quackgrass | (*Agropyron repens*) |
| Field Bindweed | (*Convolvulus arvensis* L.) |
| Morningglory | (*Ipomoea purpurea*) |
| Velvetleaf | (*Abutilon theophrasti*) |
| Cotton | (*Gossypium hirsutum*, L.) |
| Corn | (*Zea mays*) |
| Soybean | (*Glycine max*) Bragg |
| Soybean | (*Glycine max*) Williams |
| Wheat | (*Triticum aestivum*) |
| Rice | (*Oryza sativa*) Nato |

## TABLE V

POST-EMERGENCE TESTS -- RATES IN KG/HA

| Compound | RATE | BARNY ARDGR | FOXTA IL SP | P NUT SEDGE | QUACK GRASS | FLD B INDWD | MRNGL RY SP | RAGWE ED | VELVE TLEAF | CORN FIELD | COTTO N | RICE, NATO | SOYBE AN BR | SOYBE AN HI | S WHE AT ER |
|---|---|---|---|---|---|---|---|---|---|---|---|---|---|---|---|
| 3α-Ethyl-1,9bα(and β)-dihydro-3-methyl-5*H*-imidazo[1',2':1,2]pyrrolo[3,4-*b*]pyridine-2(3*H*),5-dione | 10.000 | 7.0 | | 0.0 | | | 2.0 | | 8.0 | | | | | | |
| | 1.000 | 0.0 | | 0.0 | | | 4.0 | 1.0 | 7.0 | 0.0 | 0.0 | 4.0 | | 0.0 | |
| | .500 | 0.0 | | 0.0 | | | 8.0 | 0.0 | 7.0 | 0.0 | 0.0 | 0.0 | | 0.0 | |
| | .250 | 0.0 | | 0.0 | | | 6.0 | 0.0 | 7.0 | 0.0 | 0.0 | 0.0 | | 0.0 | |
| | .125 | 0.0 | | 0.0 | | | 1.0 | 0.0 | 6.0 | 0.0 | 0.0 | 0.0 | | 0.0 | |
| | .063 | 0.0 | | 0.0 | | | 4.0 | 0.0 | 6.0 | 0.0 | 0.0 | 0.0 | | 0.0 | |
| 1,9α(and β)-dihydro-3α-isopropyl-3-methyl-5*H*-imidazo[1',2':1,2]pyrrolo-[3,4-*b*]pyridine-2(3*H*),5-dione | 2.000 | | | | | | | | | 9.0 | 9.0 | 9.0 | | 9.0 | 9.0 |
| | 1.000 | 9.0 | | 8.0 | 9.0 | 9.0 | 9.0 | 9.0 | 9.0 | 8.0 | 9.0 | 7.5 | | 8.5 | 9.0 |
| | .500 | 8.0 | | 7.0 | 9.0 | 9.0 | 9.0 | 8.0 | 9.0 | 7.5 | 9.0 | 5.0* | | 8.5 | 9.0 |
| | .250 | 2.0 | | 2.0 | 8.0 | 9.0 | 9.0 | 8.0 | 9.0 | 5.0 | 9.0 | 5.0* | | 8.0 | 8.5 |
| | .125 | 1.0 | | 0.0 | 2.0 | 9.0 | 8.0 | 8.0 | 9.0 | 1.5 | 8.0 | 4.0* | | 8.0 | 8.0 |
| | .063 | 0.0 | | 0.0 | 2.0 | 9.0 | 8.0 | 1.0 | 8.0 | 0.5 | 7.0 | 3.0* | | 6.5 | 6.0 |
| | .032 | 0.0 | | 0.0 | | 3.0 | 4.0 | 0.0 | 5.0 | 0.0 | 3.0 | 0.0 | | 1.0 | 1.0 |
| 1,9bβ-Dihydro-3α-isopropyl-3,7-dimethyl-5*H*-imidazo-[1',2':1,2]pyrrolo[3,4-*b*]-pyridine-2(3*H*),5-dione | 1.000 | 9.0 | 9.0 | 8.0 | 9.0 | 8.0 | 8.0 | 9.0 | 9.0 | 2.0 | 7.0 | 9.0 | 4.0 | 3.0 | 7.0 |
| | .500 | 9.0 | 9.0 | 8.0 | | 8.0 | 7.0 | 8.0 | 9.0 | 2.0 | 7.0 | 9.0 | 2.0 | 2.0 | 3.0 |
| | .250 | 9.0 | 9.0 | 8.0 | 8.0 | 8.0 | 4.0 | 8.0 | 8.0 | 1.0 | 3.0 | 7.0 | 2.0 | 2.0 | 2.0 |
| | .125 | 8.0 | 9.0 | 4.0 | 4.0 | 6.0 | 1.0 | 8.0 | 6.0 | 1.0 | | 7.0 | 2.0 | 1.0 | 2.0 |
| | .063 | 4.0 | 8.0 | 1.0 | 2.0 | 3.0 | 0.0 | 6.0 | 6.0 | 1.0 | 2.0 | 5.0 | 1.0 | 0.0 | 1.0 |
| | .032 | 0.0 | 4.0 | 0.0 | 0.0 | 0.0 | 0.0 | 4.0 | 2.0 | 1.0 | 2.0 | 3.0 | 1.0 | 0.0 | 1.0 |
| (*R*)-(+)-1,9bα-Dihydro-3β-isopropyl-3-methyl-5*H*-imidazo[1',2':1,2]pyrrolo-[3,4-*b*]pyridine-2(3*H*),5-dione | 1.000 | 9.0 | 9.0 | 9.0 | 9.0 | 9.0 | 9.0 | 9.0 | 9.0 | 9.0 | 9.0 | 8.0 | 9.0 | 9.0 | 9.0 |
| | .500 | 9.0 | 9.0 | 9.0 | 9.0 | 9.0 | 9.0 | 9.0 | 9.0 | 9.0 | 9.0 | 8.0 | 9.0 | 9.0 | 9.0 |
| | .250 | 9.0 | 9.0 | 9.0 | 9.0 | 8.0 | 9.0 | 8.0 | 9.0 | 8.0 | 9.0 | 8.0 | 8.0 | 9.0 | 9.0 |
| | .125 | 9.0 | 9.0 | 9.0 | 9.0 | 9.0 | 9.0 | 8.0 | 9.0 | 8.0 | 9.0 | 7.0 | 8.0 | 9.0 | 9.0 |
| | .063 | 8.0 | 9.0 | 8.0 | 9.0 | 8.0 | 9.0 | 8.0 | 9.0 | 8.0 | 8.0 | 6.0 | 8.0 | 8.0 | 9.0 |
| | .032 | 7.0 | 8.0 | 7.0 | 9.0 | 7.0 | 9.0 | 7.0 | 7.0 | 2.0 | 8.0 | 4.0 | 8.0 | 8.0 | 8.0 |

TABLE V (Continued)

POST-EMERGENCE TESTS -- RATES IN KG/HA

| Compound | RATE | BARNY ARDGR | FOXTA IL SP | P NUT SEDGE | QUACK GRASS | FLD B INDWD | MRNGL RY SP | RAGWE ED | VELVE TLEAF | CORN FIELD | COTTO N | RICE, NATO | SOYBE AN BR | SOYBE AN WI | S WHE AT ER |
|---|---|---|---|---|---|---|---|---|---|---|---|---|---|---|---|
| (±)-1,9bβ-Dihydro-3α-isopropyl-3-methyl-5*H*-imidazo[1′,2′:1,2]pyrrolo[3,4-*b*]pyridine-2(3*H*),5-dione | 1.000 | 9.0 | 9.0 | 9.0 | 9.0 | 9.0 | 9.0 | 9.0 | 9.0 | 9.0 | 9.0 | 8.0 | 9.0 | 8.0 | 9.0 |
| | .500 | 9:0 | 9.0 | 8.0 | 9.0 | 9.0 | 9.0 | 9.0 | 9.0 | 9.0 | 9.0 | 9.0 | 8.0 | 8.0 | 9.0 |
| | .250 | 9.0 | 9.0 | 8.0 | 9.0 | 9.0 | 9.0 | 8.0 | 8.0 | 9.0 | 9.0 | 8.0 | 9.0 | 8.0 | 9.0 |
| | .125 | 8.0 | 9.0 | 7.0 | 9.0 | 8.0 | 9.0 | 7.0 | 8.0 | 9.0 | 8.0 | 5.0 | 8.0 | 8.0 | 8.0 |
| | .063 | 8.0 | 8.0 | 6.0 | 9.0 | 8.0 | 9.0 | 6.0 | 8.0 | 3.0 | 7.0 | 4.0 | 8.0 | 8.0 | 8.0 |
| | .032 | 6.0 | 8.0 | 2.0 | | 7.0 | 9.0 | 3.0 | 7.0 | 2.0 | 7.0 | 2.0 | 7.0 | 6.0 | 6.0 |
| 1,9bβ-Dihydro-3α-isopropyl-3,8-dimethyl-5*H*-imidazo[1′,2′:1,2]pyrrolo[3,4-*b*]pyridine-2(3*H*),5-dione | 1.000 | 9.0 | 9.0 | 8.0 | 9.0 | 9.0 | 8.0 | 9.0 | 7.0 | 8.0 | 8.0 | 9.0 | 7.0 | 4.0 | 9.0 |
| | .500 | 9.0 | 9.0 | 8.0 | 9.0 | 8.0 | 7.0 | 8.0 | 7.0 | 7.0 | 8.0 | 9.0 | 6.0 | 3.0 | 8.0 |
| | .250 | 9.0 | 9.0 | 8.0 | 4.0 | 8.0 | 7.0 | 8.0 | 3.0 | 5.0 | 8.0 | 9.0 | 3.0 | 3.0 | 3.0 |
| | .125 | 8.0 | 9.0 | 7.0 | 4.0 | 5.0 | 2.0 | 6.0 | 1.0 | 3.0 | 8.0 | 9.0 | 3.0 | 3.0 | 4.0 |
| | .063 | 4.0 | 9.0 | 4.0 | 3.0 | 2.0 | 1.0 | 4.0 | 0.0 | 2.0 | 6.0 | 7.0 | 2.0 | 2.0 | 2.0 |
| | .032 | 0.0 | 4.0 | 0.0 | 0.0 | 0.0 | 0.0 | 2.0 | 0.0 | 2.0 | 2.0 | 4.0 | 2.0 | 2.0 | 2.0 |
| 8-(Allyloxy)-1,9bβ-dihydro-3α-isopropyl-3-methyl-5*H*-imidazo[1′,2′:1,2]pyrrolo[3,4-*b*]pyridine-2(3*H*),5-dione | | | | | | | | | | | | | | | |
| 1,9bα-Dihydro-3α-isopropyl-3-methyl-5*H*-imidazo[1′,2′:1,2]pyrrolo[3,4-*b*]pyridine-2(3*H*),5-dione | 1.000 | 9.0 | 9.0 | 7.0 | 9.0 | 9.0 | 9.0 | 9.0 | 9.0 | 7.0 | 8.0 | 6.0 | 9.0 | 8.0 | 9.0 |
| | .500 | 9.0 | 9.0 | 7.0 | 9.0 | 9.0 | 9.0 | 9.0 | 9.0 | 7.0 | 8.0 | 6.0 | 8.0 | 8.0 | 9.0 |
| | .250 | 9.0 | 9.0 | 4.0 | 9.0 | 9.0 | 9.0 | 8.0 | 8.0 | 7.0 | 8.0 | 5.0 | 8.0 | 8.0 | 9.0 |
| | .125 | 9.0 | 9.0 | 2.0 | 9.0 | 8.0 | 9.0 | 8.0 | 8.0 | 7.0 | 6.0 | 3.0 | 8.0 | 7.0 | 9.0 |
| | .063 | 9.0 | 9.0 | 2.0 | 9.0 | 8.0 | 9.0 | 8.0 | 8.0 | 7.0 | 7.0 | 3.0 | 7.0 | 7.0 | 8.0 |
| | .032 | 8.0 | 9.0 | 0.0 | 8.0 | 8.0 | 8.0 | 6.0 | 8.0 | 3.0 | 7.0 | 2.0 | 7.0 | 6.0 | 8.0 |

TABLE V (Continued)

POST-EMERGENCE TESTS -- RATES IN KG/HA

| Compound | RATE | BARNY ARDGR | FOXTA IL SP | P NUT SEDGE | QUACK GRASS | FLD B INDWD | MRIGL RY SP | RAGWE ED | VELVE TLEAF | CORN FIELD | COTTO N | RICE, NATO | SOYBE AN BR | SOYBE AN WI | S WHE AT ER |
|---|---|---|---|---|---|---|---|---|---|---|---|---|---|---|---|
| 7-Ethyl-1,9bα(and β)-dihydro-3α-isopropyl-3-methyl-5*H*-imidazo[1′,2′:1,2]pyrrolo-[3,4-*b*]pyridine-2(3*H*),5-dione | 1.000 | 9.0 | 9.0 | 4.0 | 8.0 | 9.0 | 5.5 | 8.5 | 8.0 | 0.5 | 3.0 | 6.0 | 0.0 | 0.0 | 2.0 |
| | .500 | 9.0 | 9.0 | 1.5 | 8.0 | 9.0 | 4.0 | 8.0 | 3.5 | 0.5 | 2.0 | 6.0 | 0.0 | 0.0 | 1.0 |
| | .250 | 8.5 | 7.0 | 0.0 | 2.0 | 6.0 | 3.0 | 5.0 | 3.0 | 0.5 | 1.0 | 6.0 | 0.0 | 0.0 | 1.0 |
| | .125 | 5.5 | 4.0 | 0.0 | 2.0 | 2.0 | 2.0 | 2.0 | 1.5 | 0.5 | 0.0 | 4.0 | 0.0 | 0.0 | 0.0 |
| | .063 | 1.5 | 1.0 | 0.0 | 2.0 | 1.0 | 0.0 | 0.0 | 0.0 | 0.5 | 0.0 | 3.0 | 0.0 | 0.0 | 0.0 |
| | .032 | 1.0 | 0.0 | 0.0 | 2.0 | 1.0 | 0.0 | 0.0 | 0.0 | 0.5 | 0.0 | 2.0 | 0.0 | 0.0 | 0.0 |
| 1,9bβ-Dihydro-3α-isopropyl-8-methoxy-3-methyl-5*H*-imidazo[1′,2′:1,2]pyrrolo-[3,4-*b*]pyridine-2(3*H*),5-dione | 1.000 | 8.0 | 9.0 | 8.0 | 8.0 | 9.0 | 8.0 | 8.0 | 2.0 | 2.0 | 7.0 | 8.0 | 4.0 | 4.0 | 2.0 |
| | .500 | 8.0 | 7.0 | 7.0 | 3.0 | 6.0 | 7.0 | 6.0 | 0.0 | 2.0 | 5.0 | 7.0 | 3.0 | 3.0 | 2.0 |
| | .250 | 4.0 | 5.0 | 7.0 | 2.0 | 5.0 | 7.0 | 2.0 | 0.0 | 1.0 | 2.0 | 6.0 | 3.0 | 2.0 | 0.0 |
| | .125 | 2.0 | 3.0 | 2.0 | 0.0 | 2.0 | 7.0 | 0.0 | 0.0 | 1.0 | 2.0 | 5.0 | 3.0 | 2.0 | 0.0 |
| | .063 | 1.0 | 1.0 | 1.0 | 0.0 | 2.0 | 3.0 | 0.0 | 0.0 | 1.0 | 2.0 | 5.0 | 2.0 | 1.0 | 0.0 |
| | .032 | 1.0 | 0.0 | 1.0 | 0.0 | 0.0 | 0.0 | 0.0 | 0.0 | 1.0 | 1.0 | 2.0 | 2.0 | 1.0 | 0.0 |
| 1,9bα-Dihydro-3α-isopropyl-3-methyl-5*H*-imidazo-[1′,2′:1,2]pyrrolo[3,4-*b*]-quinoline-2(3*H*),5-dione | 1.000 | 3.0 | 4.0 | 2.0 | 0.0 | 0.0 | 2.0 | | 0.0 | 0.0 | 6.0 | 4.0 | 0.0 | 0.0 | 0.0 |
| | .500 | 2.0 | 2.0 | 0.0 | 0.0 | 0.0 | 0.0 | 0.0 | 0.0 | 0.0 | 6.0 | 2.0 | 0.0 | 0.0 | 0.0 |
| | .250 | 1.0 | 2.0 | 0.0 | 0.0 | 0.0 | 0.0 | | 0.0 | 0.0 | 4.0 | 0.0 | 0.0 | 0.0 | 0.0 |
| | .125 | 0.0 | 0.0 | 0.0 | 0.0 | 0.0 | 0.0 | | 0.0 | 0.0 | 3.0 | 0.0 | 0.0 | 0.0 | 0.0 |
| | .063 | 0.0 | 0.0 | 0.0 | 0.0 | 0.0 | 0.0 | 0.0 | 0.0 | 0.0 | 3.0 | 0.0 | 0.0 | 0.0 | 0.0 |
| | .032 | 0.0 | 0.0 | 0.0 | 0.0 | 0.0 | 0.0 | 0.0 | 0.0 | 0.0 | 2.0 | 0.0 | 0.0 | 0.0 | 0.0 |
| 8-Chloro-1,9β-dihydro-3α-isopropyl-3-methyl-5*H*-imidazo[1′,2′:1,2]-pyrrolo[3,4-*b*]pyridine-2(3*H*),5-dione | 1.000 | 4.0 | 9.0 | 7.0 | 9.0 | 4.0 | 7.0 | 9.0 | 4.0 | 2.0 | 4.0 | 5.0 | 3.0 | 3.0 | 5.0 |
| | .500 | 2.0 | 8.0 | 3.0 | 6.0 | 1.0 | 5.0 | 8.0 | 2.0 | 2.0 | 2.0 | 4.0 | 2.0 | 2.0 | 4.0 |
| | .250 | 1.0 | 6.0 | 3.0 | 6.0 | 1.0 | 3.0 | 8.0 | 0.0 | 1.0 | 1.0 | 2.0 | 1.0 | 2.0 | 2.0 |
| | .125 | 0.0 | 6.0 | 1.0 | 2.0 | 0.0 | 1.0 | 7.0 | 0.0 | 0.0 | 0.0 | 1.0 | 1.0 | 1.0 | 1.0 |
| | .063 | 0.0 | 0.0 | 0.0 | 1.0 | 0.0 | 0.0 | 4.0 | 0.0 | 0.0 | 0.0 | 1.0 | 0.0 | 0.0 | 0.0 |
| | .032 | 0.0 | 0.0 | 0.0 | 0.0 | 0.0 | 0.0 | 0.0 | 0.0 | 0.0 | 0.0 | 1.0 | 0.0 | 0.0 | 0.0 |

Example 6

Preemergence herbicidal evaluation of test compounds

The preemergence herbicidal activity of the compounds of the present invention is exemplified by the following tests in which the seeds of a variety of monocotyledonous and dicotyledonous plants are separately mixed with potting soil and planted on top of approximately one inch of soil in separate pint cups. After planting, the cups are sprayed with the selected aqueous acetone solution containing test compound in sufficient quantity to provide the equivalent of about 0.016 to 10.0 kg per hectare of test compound per cup. The treated cups are then placed on greenhouse benches, watered and cared for in accordance with conventional greenhouse procedures. From four to five weeks after treatment, the tests are terminated and each cup is examined and rated according to the rating system set forth above. The herbicidal proficiency of the active ingredients of the present invention is evident from the test results which are recorded in Table VI below. Where more than one test is involved for a given compound, the data are averaged.

TABLE VI

PRE-EMERGENCE TESTS -- RATES IN KG/HA

| Compound | RATE | BARNY ARDGR | FOXTA IL SP | P NUT SEDGE | QUACK GRASS | FLD B INDWD | MRNGL RY SP | RAGWE ED | VELVE TLEAF | CORN FIELD | COTTO N | RICE, NATO | SOYBE AN BR | SOYBE AN HI | S WHE AT ER |
|---|---|---|---|---|---|---|---|---|---|---|---|---|---|---|---|
| 3α-Ethyl-1,9bα(and β)-dihydro-3-methyl-5*H*-imidazo-[1',2':1,2]pyrrolo[3,4-*b*]-pyridine-2(3*H*),5-dione | 10.000 | 8.0 | | 8.0 | | | 8.0 | 8.0 | 8.0 | | | | | | |
| | 1.000 | 3.0 | | 6.0 | | | 8.0 | 7.0 | 8.0 | 0.0 | 3.0 | 5.0 | | 0.0 | |
| | .500 | 0.0 | | 0.0 | | | 8.0 | 6.0 | 7.0 | 0.0 | 0.0 | 3.0 | | 0.0 | |
| | .250 | 0.0 | | 0.0 | | | 3.0 | 3.0 | 7.0 | 0.0 | 0.0 | 0.0 | | 0.0 | |
| | .125 | 0.0 | | 0.0 | | | 0.0 | 0.0 | 5.0 | 0.0 | 0.0 | 0.0 | | 0.0 | |
| | .063 | 0.0 | | 0.0 | | | 0.0 | 0.0 | 0.0 | 0.0 | 0.0 | 0.0 | | 0.0 | |
| | .032 | 0.0 | | 0.0 | | | 0.0 | 0.0 | 0.0 | 0.0 | 0.0 | 0.0 | | 0.0 | |
| 1,9bα(and β)-dihydro-3α-isopropyl-3-methyl-5*H*-imidazo[1',2':1,2]pyrrolo-[3,4-*b*]pyridine-2(3*H*),5-dione | 8.000 | 9.0 | | 9.0 | | | 9.0 | 8.0 | 8.0 | | | | | | |
| | 2.000 | 9.0 | | 9.0 | 9.0 | 9.0 | 9.0 | 8.0 | 9.0 | 9.0 | 8.0 | 9.0 | 8.0 | 8.0 | 9.0 |
| | 1.000 | 9.0 | | 9.0 | 9.0 | 9.0 | 8.0 | 8.0 | 9.0 | 9.0 | 8.0 | 9.0 | 8.0 | 7.0 | 9.0 |
| | .500 | 8.0 | | 9.0 | 9.0 | 9.0 | 8.5 | 8.5 | 8.5 | 7.5 | 8.5 | 9.0 | 8.0 | 8.0 | 9.0 |
| | .250 | 8.0 | | 8.0 | 8.0 | 9.0 | 8.5 | 8.0 | 8.5 | 6.5 | 7.5 | 8.0 | 7.0 | 6.5 | 8.0 |
| | .125 | 7.0 | | 5.5 | 7.0 | 9.0 | 8.5 | 6.5 | 8.5 | 3.5 | 6.0 | 7.5 | 5.0 | 5.5 | 7.0 |
| | .063 | 3.0 | | 2.5 | 4.0 | 8.5 | 5.5 | 3.0 | 8.0 | 2.0 | 2.5 | 4.5 | 2.0 | 2.5 | 5.0 |
| | .032 | 1.0 | | 1.0 | 2.0 | 6.0 | 0.5 | 1.0 | 5.5 | 0.0 | 3.5 | 2.0 | 0.0 | 0.0 | 3.0 |
| | .016 | 0.0 | | 0.0 | | 9.0 | 0.0 | 0.0 | 4.0 | 0.0 | 3;0 | 3.0 | | 0.0 | 0.0 |
| 1,9bβ-Dihydro-3α-isopropyl-3,7-dimethyl-5*H*-imidazo-[1',2':1,2]pyrrolo[3,4-*b*]-pyridine-2(3*H*),5-dione | 1.000 | 9.0 | | 9.0 | 9.0 | 9.0 | 8.0 | 9.0 | 9.0 | | | | | | |
| | .750 | 9.0 | | 9.0 | 8.0 | 9.0 | 9.0 | 9.0 | 9.0 | | | | | | |
| | .500 | 9.0 | 9.0 | 9.0 | 8.5 | 9.0 | 8.0 | 9.0 | 9.0 | 4.0 | 8.0 | 9.0 | 9.0 | 4.0 | 9.0 |
| | .250 | 9.0 | 9.0 | 8.0 | 8.5 | 8.5 | 8.0 | 8.5 | 9.0 | 3.0 | 8.0 | 9.0 | 2.0 | 3.0 | 5.0 |
| | .125 | 8.5 | 9.0 | 7.5 | 4.0 | 9.0 | 4.0 | 8.0 | 8.5 | 2.0 | 4.0 | 8.0 | 2.0 | 2.0 | 5.0 |
| | .063 | 8.0 | 6.0 | 3.0 | 4.0 | 7.0 | 1.0 | 8.0 | 3.0 | 2.0 | 3.0 | 6.0 | 2.0 | 2.0 | 2.0 |
| | .032 | 3.0 | 4.0 | 2.0 | 2.0 | 3.0 | 0.0 | 2.0 | 1.0 | 2.0 | 2.0 | 2.0 | 2.0 | 2.0 | 1.0 |
| | .016 | 0.0 | 0.0 | 1.0 | 2.0 | 0.0 | 0.0 | 0.0 | 0.0 | 2.0 | 2.0 | 2.0 | 2.0 | 2.0 | 1.0 |
| (*R*)-(+)-1,9bα-Dihydro-3β-isopropyl-3-methyl-5*H*-imidazo[1',2':1,2]pyrrolo-[3,4-*b*]pyridine-2(3*H*),5-dione | .500 | 9.0 | 9.0 | 9.0 | 9.0 | 9.0 | 9.0 | 9.0 | 9.0 | 9.0 | 9.0 | 9.0 | 9.0 | 9.0 | 9.0 |
| | .250 | 9.0 | 9.0 | 9.0 | 9.0 | 9.0 | 9.0 | 9.0 | 9.0 | 9.0 | 9.0 | 9.0 | 9.0 | 8.0 | 9.0 |
| | .125 | 9.0 | 9.0 | 9.0 | 9.0 | 9.0 | 9.0 | 9.0 | 9.0 | 9.0 | 9.0 | 9.0 | 8.0 | 7.0 | 9.0 |
| | .063 | 8.0 | 9.0 | 8.0 | 9.0 | 7.0 | 9.0 | 8.0 | 8.0 | 9.0 | 9.0 | 6.0 | 7.0 | 7.0 | 9.0 |
| | .032 | 3.0 | 9.0 | 7.0 | 9.0 | 7.0 | 8.0 | 3.0 | 7.0 | 7.0 | 7.0 | 5.0 | 6.0 | 4.0 | 9.0 |
| | .016 | 0.0 | 4.0 | 3.0 | 8.0 | 6.0 | 7.0 | 0.0 | 7.0 | 2.0 | 2.0 | 4.0 | 3.0 | 3.0 | 7.0 |

TABLE VI (Continued)

PRE-EMERGENCE TESTS -- RATES IN KG/HA

| Compound | RATE | BARNY ARDGR | FOXTA IL SP | P NUT SEDGE | QUACK GRASS | FLD B INDWD | MRNGL RY SP | RAGWE ED | VELVE TLEAF | CORN FIELD | COTTO N | RICE, NATO | SOYBE AN BR | SOYBE AN WI | S WHE AT ER |
|---|---|---|---|---|---|---|---|---|---|---|---|---|---|---|---|
| (±)-1,9bβ-Dihydro-3α-isopropyl-3-methyl-5*H*-imidazo[1′,2′:1,2]pyrrolo[3,4-*b*]pyridine-2(3*H*),5-dione | .500 | 9.0 | 9.0 | 9.0 | 9.0 | 9.0 | 9.0 | 9.0 | 9.0 | 9.0 | 9.0 | 9.0 | 9.0 | 8.0 | 9.0 |
| | .250 | 9.0 | 9.0 | 7.0 | 9.0 | 9.0 | 9.0 | 9.0 | 9.0 | 9.0 | 9.0 | 9.0 | 8.0 | 8.0 | 9.0 |
| | .125 | 8.0 | 9.0 | 9.0 | 9.0 | 9.0 | 9.0 | 8.0 | 9.0 | 9.0 | 9.0 | 6.0 | 8.0 | 7.0 | 9.0 |
| | .063 | 8.0 | 9.0 | 7.0 | 8.0 | 9.0 | 8.0 | 7.0 | 8.0 | 7.0 | 8.0 | 4.0 | 3.0 | 6.0 | 8.0 |
| | .032 | 3.0 | 8.0 | 4.0 | 8.0 | 9.0 | 8.0 | 3.0 | 9.0 | 5.0 | 4.0 | 3.0 | 7.0 | 2.0 | 7.0 |
| | .016 | 0.0 | 4.0 | 2.0 | 7.0 | 8.0 | 2.0 | 2.0 | 6.0 | 3.0 | 3.0 | 2.0 | 7.0 | 2.0 | 7.0 |
| 1,9bβ-Dihydro-3α-isopropyl-3,8-dimethyl-5*H*-imidazo[1′,2′:1,2]pyrrolo[3,4-*b*]pyridine-2(3*H*),5-dione | .500 | 9.0 | 9.0 | 9.0 | 9.0 | 9.0 | 8.0 | 9.0 | 9.0 | 9.0 | 9.0 | 9.0 | 4.0 | 4.0 | 9.0 |
| | .250 | 8.0 | 9.0 | 8.0 | 9.0 | 8.0 | 7.0 | 9.0 | 8.0 | 3.0 | 9.0 | 9.0 | 2.0 | 3.0 | 9.0 |
| | .125 | 0.0 | 9.0 | 8.0 | 9.0 | 8.0 | 7.0 | 8.0 | 7.0 | 4.0 | 5.0 | 6.0 | 2.0 | 2.0 | 8.0 |
| | .063 | 0.0 | 7.0 | 6.0 | 7.0 | 5.0 | 4.0 | 8.0 | 5.0 | 3.0 | 4.0 | 4.0 | 1.0 | 2.0 | 3.0 |
| | .032 | 0.0 | 4.0 | 4.0 | 3.0 | 5.0 | 0.0 | 3.0 | 3.0 | 2.0 | 2.0 | 3.0 | 1.0 | 2.0 | 2.0 |
| 8-(Allyloxy)-1,9bβ-dihydro-3α-isopropyl-3-methyl-5*H*-imidazo[1′,2′:1,2]pyrrolo-[3,4-*b*]pyridine-2(3*H*),5-dione | .500 | 2.0 | 4.0 | 3.0 | 2.0 | 9.0 | 6.0 | 4.0 | 2.0 | 2.0 | 0.0 | 0.0 | 1.0 | | 0.0 |
| | .250 | 1.0 | 0.0 | 1.0 | 1.0 | 1.0 | 2.0 | 2.0 | 0.0 | 0.0 | 0.0 | 0.0 | 1.0 | 0.0 | 0.0 |
| 1,9bα-Dihydro-3α-isopropyl-3-methyl-5*H*-imidazo-[1′,2′:1,2]pyrrolo[3,4-*b*]-pyridine-2(3*H*),5-dione | .500 | 9.0 | 9.0 | 9.0 | 9.0 | 9.0 | 9.0 | 9.0 | 9.0 | 9.0 | 9.0 | 8.0 | 9.0 | 8.0 | 9.0 |
| | .250 | 9.0 | 9.0 | 7.0 | 9.0 | 9.0 | 9.0 | 9.0 | 9.0 | 9.0 | 9.0 | 7.0 | 9.0 | 8.0 | 9.0 |
| | .125 | 9.0 | 9.0 | 6.0 | 9.0 | 9.0 | 9.0 | 9.0 | 9.0 | 9.0 | 8.0 | 6.0 | 6.0 | 7.0 | 9.0 |
| | .063 | 9.0 | 9.0 | 4.0 | 8.0 | 9.0 | 8.0 | 8.0 | 9.0 | 4.0 | 4.0 | 3.0 | 4.0 | 4.0 | 8.0 |
| | .032 | 7.0 | 8.0 | 2.0 | 7.0 | 7.0 | 8.0 | 8.0 | 9.0 | 3.0 | 3.0 | 3.0 | 5.0 | 2.0 | 7.0 |
| | .016 | 2.0 | 7.0 | 0.0 | 7.0 | 7.0 | 7.0 | 7.0 | 7.0 | 2.0 | 2.0 | 2.0 | | 2.0 | 7.0 |

TABLE VI (Continued)

PRE-EMERGENCE TESTS -- RATES IN KG/HA

| Compound | RATE | BARNY ARDGR | FOXTA IL SP | P NUT SEDGE | QUACK GRASS | FLD B INDWD | MRNGL RY SP | RAGWE ED | VELVE TLEAF | CORN FIELD | COTTO N | RICE, NATO | SOYBE AN BR | SOYBE AN WI | S WHE AT ER |
|---|---|---|---|---|---|---|---|---|---|---|---|---|---|---|---|
| 7-Ethyl-1,9bα(and β)-dihydro-3α-isopropyl-3-methyl-5*H*-imidazo[1′,2′:1,2]pyrrolo-[3,4-*b*]pyridine-2(3*H*),5-dione | 1.000 | 9.0 | | 8.0 | 9.0 | 9.0 | 9.0 | 9.0 | 9.0 | | | | | | |
| | .500 | 8.5 | 9.0 | 8.0 | 9.0 | 9.0 | 7.5 | 9.0 | 7.5 | 3.0 | 4.0 | 9.0 | 0.0 | 1.0 | 7.0 |
| | .250 | 7.0 | 9.0 | 5.5 | 8.0 | 8.5 | 2.5 | 7.0 | 7.0 | 2.0 | 2.0 | 9.0 | 0.0 | 1.0 | 2.0 |
| | .125 | 6.0 | 9.0 | 3.0 | 5.0 | 8.0 | 0.5 | 4.5 | 4.0 | 2.0 | 0.0 | 6.0 | 0.0 | 0.0 | 0.0 |
| | .063 | 2.0 | 6.0 | 0.5 | 2.0 | 5.0 | 0.0 | 0.0 | 0.0 | 1.0 | 0.0 | 3.0 | 0.0 | 0.0 | 0.0 |
| | .032 | 0.0 | 2.0 | 0.0 | | 0.0 | 0.0 | 0.0 | 0.0 | 1.0 | 0.0 | 2.0 | 0.0 | 0.0 | 0.0 |
| | .016 | 0.0 | 0.0 | 0.0 | 0.0 | 0.0 | 0.0 | 0.0 | 0.0 | 1.0 | 0.0 | 2.0 | 0.0 | 0.0 | 0.0 |
| 1,9bβ-Dihydro-3α-isopropyl-8-methoxy-3-methyl-5*H*-imidazo[1′,2′:1,2]pyrrolo-[3,4-*b*]pyridine-2(3*H*),5-dione | .500 | 9.0 | 9.0 | 9.0 | 9.0 | 9.0 | 9.0 | 9.0 | 7.0 | 7.0 | 8.0 | 6.0 | 5.0 | 4.0 | 7.0 |
| | .250 | 8.0 | 9.0 | 6.0 | 7.0 | 7.0 | 8.0 | 8.0 | 4.0 | 5.0 | 6.0 | 4.0 | | 3.0 | 7.0 |
| | .125 | 8.0 | 6.0 | 7.0 | 7.0 | 8.0 | 7.0 | 5.0 | 2.0 | 3.0 | 5.0 | 3.0 | 1.0 | 1.0 | 5.0 |
| | .063 | 2.0 | 6.0 | 4.0 | 2.0 | 6.0 | 6.0 | 2.0 | 1.0 | 3.0 | 2.0 | 2.0 | 0.0 | 1.0 | 2.0 |
| | .032 | 0.0 | 0.0 | 1.0 | 0.0 | 3.0 | 3.0 | 0.0 | 0.0 | 2.0 | 2.0 | 2.0 | 0.0 | 0.0 | 1.0 |
| | .016 | 0.0 | 0.0 | 0.0 | 0.0 | 0.0 | 0.0 | 0.0 | 0.0 | 2.0 | 2.0 | 1.0 | 0.0 | 0.0 | 1.0 |
| 1,9bα-Dihydro-3α-isopropyl-3-methyl-5*H*-imidazo-[1′,2′:1,2]pyrrolo[3,4-*b*]-quinoline-2(3*H*),5-dione | .500 | 8.0 | 5.0 | 6.0 | 0.0 | 4.0 | 0.0 | 7.0 | 4.0 | 0.0 | 0.0 | 0.0 | 0.0 | 0.0 | 0.0 |
| | .250 | 7.0 | 5.0 | 2.0 | 0.0 | 3.0 | 0.0 | 7.0 | 3.0 | 0.0 | 0.0 | 0.0 | 0.0 | 0.0 | 0.0 |
| | .125 | 0.0 | 5.0 | 0.0 | 0.0 | 0.0 | 0.0 | 2.0 | 0.0 | 0.0 | 0.0 | 0.0 | 0.0 | 0.0 | 0.0 |
| | .063 | 0.0 | 0.0 | 0.0 | 0.0 | 0.0 | 0.0 | 0.0 | 0.0 | 0.0 | 0.0 | 0.0 | 0.0 | 0.0 | 0.0 |
| | .032 | 0.0 | 0.0 | 0.0 | 0.0 | 0.0 | 0.0 | 0.0 | 0.0 | 0.0 | 0.0 | 0.0 | 0.0 | 0.0 | 0.0 |
| | .016 | 0.0 | 0.0 | 0.0 | 0.0 | 0.0 | 0.0 | 0.0 | 0.0 | 0.0 | 0.0 | 0.0 | 0.0 | 0.0 | 0.0 |
| 8-Chloro-1,9β-dihydro-3α-isopropyl-3-methyl-5*H*-imidazo[1′,2′:1,2]-pyrrolo[3,4-*b*]pyridine-2(3*H*),5-dione | .500 | 7.0 | 9.0 | 9.0 | 9.0 | 9.0 | 7.0 | 9.0 | 5.0 | 6.0 | 2.0 | 4.0 | 0.0 | 1.0 | 9.0 |
| | .250 | 4.0 | 9.0 | 7.0 | 9.0 | 9.0 | 7.0 | 9.0 | 3.0 | 3.0 | 1.0 | 2.0 | 0.0 | 1.0 | 9.0 |
| | .125 | 1.0 | 4.0 | 6.0 | 7.0 | 4.0 | 5.0 | 8.0 | 2.0 | 2.0 | 1.0 | 2.0 | 0.0 | 0.0 | 7.0 |
| | .063 | 0.0 | 1.0 | 2.0 | 7.0 | 0.0 | 2.0 | 3.0 | 0.0 | 2.0 | 0.0 | 1.0 | 0.0 | 0.0 | 4.0 |
| | .032 | 0.0 | 0.0 | 1.0 | 6.0 | 0.0 | 1.0 | 0.0 | 0.0 | 1.0 | 0.0 | 1.0 | 0.0 | 0.0 | 2.0 |
| | .016 | 0.0 | 0.0 | 0.0 | 3.0 | 0.0 | 0.0 | 0.0 | 0.0 | 0.0 | 0.0 | 1.0 | 0.0 | 0.0 | 2.0 |

TABLE VI (Continued)

PRE-EMERGENCE TESTS -- RATES IN KG/HA

| Compound | RATE | BARNY ARDGR | FOXTA IL SP | P NUT SEDGE | QUACK GRASS | FLD B INDWD | MRNGL RY SP | RAGWE ED | VELVE TLEAF | CORN FIELD | COTTO N | RICE, NATO | SOYBE AN BR | SOYBE AN WI | S WHE AT ER |
|---|---|---|---|---|---|---|---|---|---|---|---|---|---|---|---|
| 7-Ethyl-1,9bβ-dihydro-3α-isopropyl-3-methyl-5*H*-imidazo[1',2':1,2]pyrrolo-[3,4-*b*]pyridine-2(3*H*),5-dione | .500 | 9.0 | 9.0 | 8.0 | 2.0 | 9.0 | 7.0 | 9.0 | 8.0 | 3.0 | 2.0 | 9.0 | 0.0 | 0.0 | 8.0 |
| | .250 | 9.0 | 9.0 | 7.0 | 1.0 | 9.0 | 7.0 | 7.0 | 8.0 | 2.0 | 1.0 | 9.0 | 0.0 | 0.0 | 8.0 |
| | .125 | 8.0 | 9.0 | 6.0 | 0.0 | 7.0 | 4.0 | 4.0 | 6.0 | 1.0 | 0.0 | 7.0 | 0.0 | 0.0 | 2.0 |
| | .063 | 7.0 | 3.0 | 1.0 | 0.0 | 4.0 | 0.0 | 2.0 | 3.0 | 0.0 | 0.0 | 6.0 | 0.0 | 0.0 | 0.0 |
| | .032 | 2.0 | 3.0 | 0.0 | 0.0 | 1.0 | 0.0 | 0.0 | 1.0 | 0.0 | 0.0 | 2.0 | 0.0 | 0.0 | 0.0 |
| | .016 | 0.0 | 0.0 | 0.0 | 0.0 | 0.0 | 0.0 | 0.0 | 0.0 | 0.0 | 0.0 | 2.0 | 0.0 | 0.0 | 0.0 |
| 7-Ethyl-1,9bα-dihydro-3α-isopropyl-3-methyl-5*H*-imidazo[1',2':1,2]-pyrrolo[3,4-*b*]pyridine-2(3*H*),5-dione | .500 | 9.0 | 9.0 | 7.0 | 0.0 | 7.0 | 8.0 | 9.0 | 8.0 | 2.0 | | 9.0 | 0.0 | 2.0 | 8.0 |
| | .250 | 9.0 | 9.0 | 6.0 | 0.0 | 6.0 | 7.0 | 9.0 | 7.0 | 2.0 | 2.0 | 7.0 | 0.0 | 1.0 | 3.0 |
| | .125 | 9.0 | 9.0 | 3.0 | 0.0 | 4.0 | 2.0 | 4.0 | 3.0 | 2.0 | 1.0 | 7.0 | 0.0 | 0.0 | 1.0 |
| | .063 | 4.0 | 6.0 | 1.0 | 0.0 | 1.0 | 0.0 | 2.0 | 0.0 | 1.0 | 0.0 | 6.0 | 0.0 | 0.0 | 0.0 |
| | .032 | 1.0 | 2.0 | 1.0 | 0.0 | 0.0 | 0.0 | 0.0 | 0.0 | 1.0 | 0.0 | 3.0 | 0.0 | 0.0 | 0.0 |
| | .016 | 0.0 | 0.0 | 1.0 | 0.0 | 0.0 | 0.0 | 0.0 | 0.0 | 1.0 | 0.0 | 2.0 | 0.0 | 0.0 | 0.0 |
| 1,9bβ-Dihydro-3α-isopropyl-3-methyl-2-thio-5*H*-imidazo[1',2':1,2]-pyrrolo[3,4-*b*]pyridine-2(3*H*),5-dione | .500 | 9.0 | 9.0 | 9.0 | 9.0 | 9.0 | 8.0 | 7.0 | 9.0 | 9.0 | 7.0 | 9.0 | 7.0 | 7.0 | 9.0 |
| | .250 | 7.0 | 8.0 | 9.0 | 9.0 | 9.0 | 8.0 | | 8.0 | 9.0 | 7.0 | 8.0 | 7.0 | 7.0 | 9.0 |
| | .125 | 6.0 | 8.0 | 8.0 | 7.0 | 7.0 | 6.0 | 2.0 | 7.0 | 9.0 | 3.0 | 6.0 | 5.0 | 4.0 | 9.0 |
| | .063 | 1.0 | 2.0 | 2.0 | 7.0 | 6.0 | 2.0 | 1.0 | 6.0 | 6.0 | 1.0 | 2.0 | 3.0 | 2.0 | 4.0 |
| | .032 | 0.0 | 1.0 | 0.0 | 2.0 | 0.0 | 0.0 | 0.0 | 3.0 | 3.0 | 0.0 | 1.0 | 1.0 | 1.0 | 2.0 |
| | .016 | 0.0 | 0.0 | 0.0 | 0.0 | 0.0 | 0.0 | 0.0 | 1.0 | 1.0 | 0.0 | 0.0 | 0.0 | 0.0 | 0.0 |

**Claims for the Contracting States: BE CH DE FR IT LI NL SE**

1. A compound having the structure:

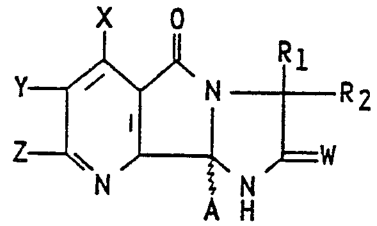

wherein

$R_1$ and $R_2$ each represent $C_1$—$C_3$ alkyl or cyclopropyl, with the proviso that the sum of the number of carbon atoms in $R_1$ and $R_2$ is 2 to 5; and when $R_1$ and $R_2$ are taken together with the carbon to which they are attached, they may form a $C_3$—$C_6$ cycloalkyl ring optionally substituted with methyl;

W is oxygen or sulfur;

A is hydrogen, hydroxyl, $C_3$—$C_6$ alkenyloxy, $C_3$—$C_6$ alkynyloxy, $C_1$—$C_6$ alkylthio, $NR_{13}R_{14}$ or $C_1$—$C_6$ alkoxy optionally substituted with phenyl, halophenyl, $C_1$—$C_3$ alkylphenyl, $C_1$—$C_3$ alkoxyphenyl or di-$C_1$—$C_3$ alkylaminophenyl;

$R_{13}$ is hydrogen, $C_1$—$C_4$ alkyl optionally substituted with phenyl, halophenyl, $C_1$—$C_3$ alkylphenyl or $C_1$—$C_3$ alkoxyphenyl;

$R_{14}$ is hydrogen or $C_1$—$C_4$ alkyl;

X is hydrogen, halogen or methyl;

Y and Z are each hydrogen, halogen, $C_1$—$C_6$ alkyl, $C_1$—$C_4$ hydroxyalkyl, $C_1$—$C_6$ alkoxy, $C_1$—$C_4$ alkylthio, phenoxy, $C_1$—$C_4$ haloalkyl, $OCF_2CHF_2$, $OCF_3$, $OCHF_2$, nitro, cyano, $NR_4R_5$, $C_3$—$C_8$ straight or branched alkenyloxy optionally substituted with one to three halogens, $C_3$—$C_8$ straight or branched alkynyloxy optionally substituted with one to three halogens, or phenyl optionally substituted with one $C_1$—$C_4$ alkyl, $C_1$—$C_4$ alkoxy or halogen;

$R_4$ is hydrogen or $C_1$—$C_4$ alkyl;

$R_5$ is $C_1$—$C_4$ alkyl;

and, when taken together, Y and Z may form a ring in which YZ is represented by

(1) the structure: $—(CH_2)_n—$, where n is an integer of 2, 3 or 4, provided that X is hydrogen; or

(2) by the structure:

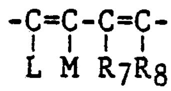

where, L, M, $R_7$ and $R_8$ each represent hydrogen, halogen, $C_1$—$C_4$ alkyl, $C_1$—$C_4$ alkoxy, $C_1$—$C_4$ alkylthio, $C_1$—$C_4$ alkylsulfonyl, $C_1$—$C_4$ haloalkyl, $NO_2$, CN, phenyl, phenoxy, amino, $OCF_3$, $OCHF_2$, $OCF_2CHF_2$, $C_1$—$C_4$ alkylamino, dialkyl($C_1$—$C_4$)amino, chlorophenyl, methylphenyl, $C_3$—$C_8$ straight or branched alkenyloxy optionally substituted with one to three halogens, $C_3$—$C_8$ straight or branched alkynyloxy optionally substituted with one to three halogens, or phenoxy substituted with one Cl, $CF_3$, $NO_2$ or $CH_3$ group, with the proviso that only one of L, M, $R_7$ or $R_8$ may represent a substituent other than hydrogen, halogen, $C_1$—$C_4$ alkyl or $C_1$—$C_4$ alkoxy; and when A is OH at least one of L, M, $R_7$, and $R_8$ is other than hydrogen; or

(3) by the structures:

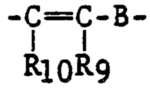, 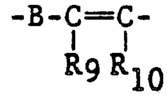, 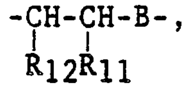, or 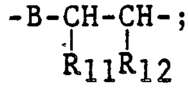;

where B is oxygen or sulfur; $R_9$ and $R_{10}$ each represent hydrogen, halogen, phenyl, or $C_1$—$C_4$ alkyl; $R_{11}$ and $R_{12}$ each represent hydrogen, $C_1$—$C_4$ alkyl or phenyl;

and when $R_1$ and $R_2$ are not the same, the *cis*- or *trans*-isomers or mixtures thereof or the optical isomers (*cis*- or *trans*- or mixtures thereof).

2. A compound according to Claim 1, wherein A, $R_1$, $R_2$, W and X are as defined in said Claim 1 above; Y and Z each, independently, represent hydrogen, halogen, $C_1$—$C_6$ alkyl, $C_1$—$C_6$ alkoxy, CN, $NO_2$, $OCF_3$, $OCHF_2$, $OCF_2CHF_2$, phenoxy, $C_1$—$C_4$ haloalkyl, $C_1$—$C_4$ alkylthio, $C_1$—$C_4$ hydroxyalkyl, $NR_4R_5$, $C_3$—$C_8$ straight or branched alkenyloxy optionally substituted with one to three halogens, $C_3$—$C_8$ straight or branched alkynyloxy optionally substituted with one to three halogens, or phenyl optionally substituted with one $C_1$—$C_4$ alkyl, $C_1$—$C_4$ alkoxy or halogen; $R_4$ is hydrogen or $C_1$—$C_4$ alkyl; $R_5$ is $C_1$—$C_4$ alkyl; and when taken

together Y and Z may form a ring in which YZ are represented by the structure: $-(CH_2)_n-$, where n is an integer of 2, 3 or 4 provided that X is hydrogen; and when $R_1$ and $R_2$ are not the same, the *cis*- or *trans*-isomers or mixtures thereof or the optical isomers (*cis*- or *trans*- or mixtures thereof).

3. A method for preparing a compound having the structure:

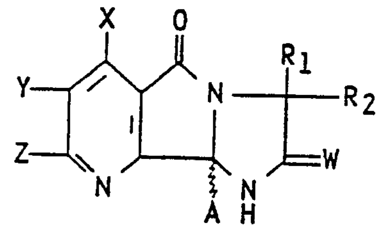

wherein $R_1$, $R_2$, A, W, X, Y and Z are as defined in Claim 1, comprising reacting a compound having the formula:

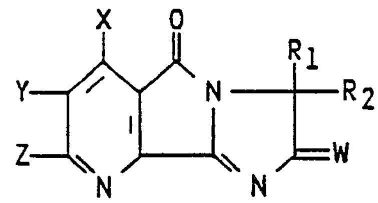

wherein $R_1$, $R_2$, W, X, Y and Z are as described above, with at least one equivalent of a nucleophile having the formula: AH, wherein A is as described above.

4. A method for the preparation of a compound having the structure:

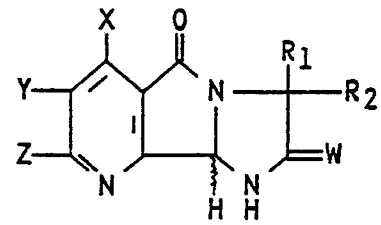

wherein $R_1$, $R_2$, W, X, Y and Z are as defined in Claim 1, comprising reacting a compound having the structure:

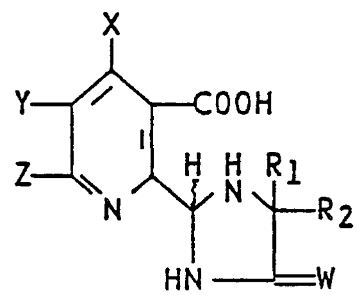

wherein $R_1$, $R_2$, W, X, Y and Z are as described above, with at least one molar equivalent of acetic anhydride in the presence of pyridine and acetonitrile, whereby the desired product is formed.

5. A method for the preparation of a compound having the formula:

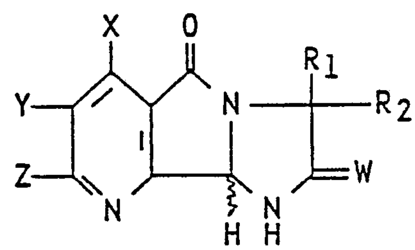

wherein $R_1$, $R_2$, W, X, Y and Z are as defined in Claim 1, comprising reacting a compound of the formula:

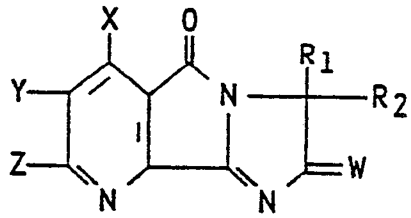

wherein X, Y, Z, $R_1$, $R_2$ and W, are as described above, with at least an equimolar amount of sodium borohydride or lithium borohydride, whereby the desired product is formed.

6. A method for the control of undesirable monocotyledonous and dicotyledonous plant species comprising: applying to the foliage of said plants or to soil containing seeds or other propagating organs thereof, a herbicidally effective amount of a compound having the structure:

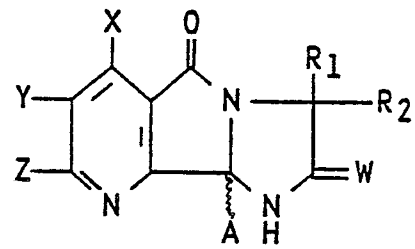

wherein

$R_1$ and $R_2$ each represent $C_1$—$C_3$ alkyl or cyclopropyl, with the proviso that the sum of the number of carbon atoms in $R_1$ and $R_2$ is 2 to 5; and when $R_1$ and $R_2$ are taken together with the carbon to which they are attached, they may form a $C_3$—$C_6$ cycloalkyl ring optionally substituted with methyl;

W is oxygen or sulfur;

A is hydrogen, hydroxyl, $C_3$—$C_6$ alkenyloxy, $C_3$—$C_6$ alkynyloxy, $C_1$—$C_6$ alkylthio, $NR_{13}R_{14}$ or $C_1$—$C_6$ alkoxy optionally substituted with phenyl, halophenyl, $C_1$—$C_3$ alkylphenyl, $C_1$—$C_3$ alkoxyphenyl or di-$C_1$—$C_3$ alkylaminophenyl;

$R_{13}$ is hydrogen, $C_1$—$C_4$ alkyl optionally substituted with phenyl, halophenyl, $C_1$—$C_3$ alkylphenyl or $C_1$—$C_3$ alkoxyphenyl;

$R_{14}$ is hydrogen or $C_1$—$C_4$ alkyl;

X is hydrogen, halogen or methyl;

Y and Z are each hydrogen, halogen, $C_1$—$C_6$ alkyl, $C_1$—$C_4$ hydroxyalkyl, $C_1$—$C_6$ alkoxy, $C_1$—$C_4$ alkylthio, phenoxy, $C_1$—$C_4$ haloalkyl, $OCF_2CHF_2$, $OCF_3$, $OCHF_2$, nitro, cyano, $NR_4R_5$, $C_3$—$C_8$ straight or branched alkenyloxy optionally substituted with one to three halogens, $C_3$—$C_8$ straight or branched alkynyloxy optionally substituted with one to three halogens, or phenyl optionally substituted with one $C_1$—$C_4$ alkyl, $C_1$—$C_4$ alkoxy or halogen;

$R_4$ is hydrogen or $C_1$—$C_4$ alkyl;

$R_5$ is $C_1$—$C_4$ alkyl;

And, when taken together, Y and Z may form a ring in which YZ is represented by

(1) the structure: $—(CH_2)_n—$, where n is an integer of 2, 3 or 4, provided that X is hydrogen; or

(2) by the structure:

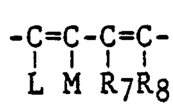

where, L, M, $R_7$ and $R_8$ each represent hydrogen, halogen, $C_1$—$C_4$ alkyl, $C_1$—$C_4$ alkoxy, $C_1$—$C_4$ alkylthio, $C_1$—$C_4$ alkylsulfonyl, $C_1$—$C_4$ haloalkyl, $NO_2$, CN, phenyl, phenoxy, amino, $OCF_3$, $OCHF_2$, $OCF_2CHF_2$, $C_1$—$C_4$ alkylamino, dialkyl($C_1$—$C_4$)amino, chlorophenyl, methylphenyl, $C_3$—$C_8$ straight or branched alkenyloxy optionally substituted with one to three halogens, $C_3$—$C_8$ straight or branched alkynyloxy optionally substituted with one to three halogens, or phenoxy substituted with one Cl, $CF_3$, $NO_2$ or $CH_3$ group, with the proviso that only one of L, M, $R_7$ or $R_8$ may represent a substituent other than hydrogen, halogen, $C_1$—$C_4$ alkyl or $C_1$—$C_4$ alkoxy; or

(3) by the structure

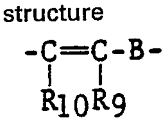 , 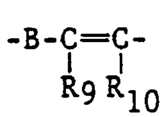 , 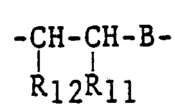 , or 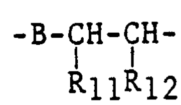 ;

where B is oxygen or sulfur; $R_9$ and $R_{10}$ each represent hydrogen, halogen, phenyl, or $C_1$—$C_4$ alkyl; $R_{11}$ and $R_{12}$ each represent hydrogen, $C_1$—$C_4$ alkyl or phenyl;
and when $R_1$ and $R_2$ are not the same, the *cis*- or *trans*-isomers or mixtures thereof or the optical isomers (*cis*- or *trans*- or mixtures thereof).

7. A method according to Claim 6, wherein A, $R_1$, $R_2$, W and X are as defined in said Claim 1 above; Y and Z each, independently, represent hydrogen, halogen, $C_1$—$C_6$ alkyl, $C_1$—$C_6$ alkoxy, CN, $NO_2$, $OCF_3$, $OCHF_2$, $OCF_2CHF_2$, phenoxy, $C_1$—$C_4$ haloalkyl, $C_1$—$C_4$ alkylthio, $C_1$—$C_4$ hydroxyalkyl, $NR_4R_5$, $C_3$—$C_8$ straight or branched alkenyloxy optionally substituted with one to three halogens, $C_3$—$C_8$ straight or branched alkynyloxy optionally substituted with one to three halogens, or phenyl optionally substituted with one $C_1$—$C_4$ alkyl, $C_1$—$C_4$ alkoxy or halogen; $R_4$ is hydrogen or $C_1$—$C_4$ alkyl; $R_5$ is $C_1$—$C_4$ alkyl; and when taken together Y and Z may form a ring in which YZ are represented by the structure: $-(CH_2)_n-$, where n is an integer of 2, 3 or 4, provided that X is hydrogen; and when $R_1$ and $R_2$ are not the same, the *cis*- or *trans*-isomers or mixtures thereof or the optical isomers (*cis*- or *trans*- or mixtures thereof).

8. A herbicidal composition comprising a herbicidally effective amount of a compound having the structure:

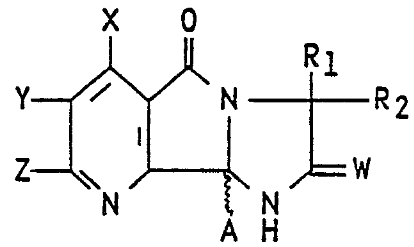

wherein $R_1$, $R_2$, A, W, X, Y and Z are as defined in Claim 6 and a non-toxic solid or liquid inert diluent therefore.

**Claims for the Contracting State: AT**

1. A method for preparing a compound having the structure:

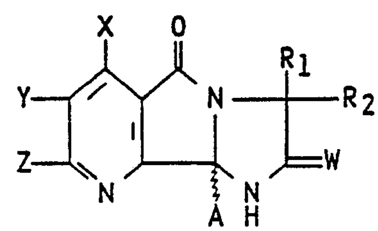

wherein

$R_1$ and $R_2$ each represent $C_1$—$C_3$ alkyl or cyclopropyl, with the proviso that the sum of the number of carbon atoms in $R_1$ and $R_2$ is 2 to 5; and when $R_1$ and $R_2$ are taken together with the carbon to which they are attached, they may form a $C_3$—$C_6$ cycloalkyl ring optionally substituted with methyl;

W is oxygen or sulfur;

A is hydroxyl, $C_3$—$C_6$ alkenyloxy, $C_3$—$C_6$ alkynyloxy, $C_1$—$C_6$ alkylthio, $NR_{13}R_{14}$ or $C_1$—$C_6$ alkoxy optionally substituted with phenyl, halophenyl, $C_1$—$C_3$ alkylphenyl, $C_1$—$C_3$ alkoxyphenyl or di-$C_1$—$C_3$ alkylaminophenyl;

$R_{13}$ is hydrogen, $C_1$—$C_4$ alkyl optionally substituted with phenyl, halophenyl, $C_1$—$C_3$ alkylphenyl or $C_1$—$C_3$ alkoxyphenyl;

$R_{14}$ is hydrogen or $C_1$—$C_4$ alkyl;

X is hydrogen, halogen or methyl;

Y and Z are each hydrogen, halogen, $C_1$—$C_6$ alkyl, $C_1$—$C_4$ hydroxyalkyl, $C_1$—$C_6$ alkoxy, $C_1$—$C_4$ alkylthio, phenoxy, $C_1$—$C_4$ haloalkyl, $OCF_2CHF_2$, $OCF_3$, $OCHF_2$, nitro, cyano, $NR_4R_5$, $C_3$—$C_8$ straight or branched alkenyloxy optionally substituted with one to three halogens, $C_3$—$C_8$ straight or branched alkynyloxy optionally substituted with one to three halogens, or phenyl optionally substituted with one $C_1$—$C_4$ alkyl, $C_1$—$C_4$ alkoxy or halogen;

$R_4$ is hydrogen or $C_1$—$C_4$ alkyl;

$R_5$ is $C_1$—$C_4$ alkyl;

And, when taken together, Y and Z may form a ring in which YZ is represented by

(1) the structure: $-(CH_2)_n-$, where n is an integer of 2, 3 or 4, provided that X is hydrogen; or

(2) by the structure:

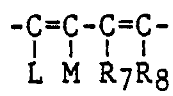

where, L, M, $R_7$ and $R_8$ each represent hydrogen, halogen, $C_1$—$C_4$ alkyl, $C_1$—$C_4$ alkoxy, $C_1$—$C_4$ alkylthio, $C_1$—$C_4$ alkylsulfonyl, $C_1$—$C_4$ haloalkyl, $NO_2$, CN, phenyl, phenoxy, amino, $OCF_3$, $OCHF_2$, $OCF_2CHF_2$, $C_1$—$C_4$ alkylamino, dialkyl($C_1$—$C_4$)amino, chlorophenyl, methylphenyl, $C_3$—$C_8$ straight or branched alkenyloxy optionally substituted with one to three halogens, $C_3$—$C_8$ straight or branched alkynyloxy optionally substituted with one to three halogens, or phenoxy substituted with one Cl, $CF_3$, $NO_2$ or $CH_3$ group, with the proviso that only one of L, M, $R_7$ or $R_8$ may represent a substituent other than hydrogen, halogen, $C_1$—$C_4$ alkyl or $C_1$—$C_4$ alkoxy; and when A is OH at least one of L, M, $R_7$, and $R_8$ is other than hydrogen; or

(3) by the structures:

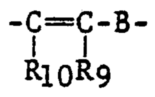, 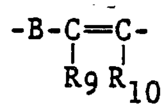, 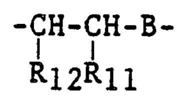, or 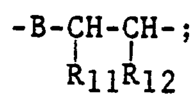;

where B is oxygen or sulfur; $R_9$ and $R_{10}$ each represent hydrogen, halogen, phenyl, or $C_1$—$C_4$ alkyl; $R_{11}$ and $R_{12}$ each represent hydrogen, $C_1$—$C_4$ alkyl or phenyl;
and when $R_1$ and $R_2$ are not the same, the *cis*- or *trans*-isomers or mixtures thereof or the optical isomers (*cis*- or *trans*- or mixtures thereof); comprising, reacting a compound having the formula:

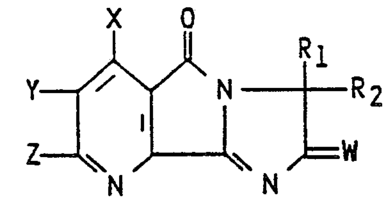

wherein $R_1$, $R_2$, W, X, Y and Z are as described above, with at least one equivalent of a nucleophile having the formula: AH, wherein A is as described above.

2. A method for the preparation of a compound having the structure:

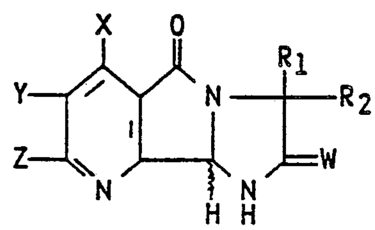

wherein $R_1$, $R_2$, W, X, Y and Z are as defined in Claim 1, comprising reacting a compound having the structure:

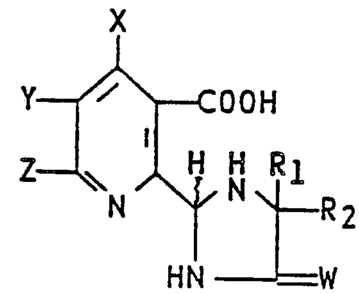

wherein $R_1$, $R_2$, W, X, Y and Z are as described above, with at least one molar equivalent of acetic anhydride in the presence of pyridine and acetonitrile, whereby the desired product is formed.

3. A method for the preparation of a compound having the formula:

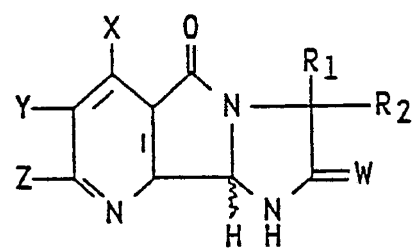

wherein $R_1$, $R_2$, W, X, Y and Z are as defined in Claim 1, comprising reacting a compound of the formula:

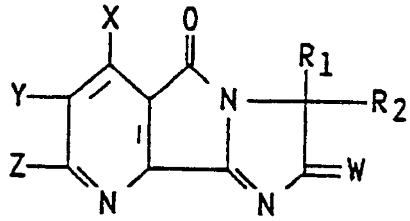

wherein X, Y, Z, $R_1$, $R_2$ and W, are as described above, with at least an equimolar amount of sodium borohydride or lithium borohydride, whereby the desired product is formed.

4. A method for the control of undesirable monocotyledonous and dicotyledonous plant species comprising: applying to the foliage of said plants or to soil containing seeds or other propagating organs thereof, a herbicidally effective amount of a compound having the structure:

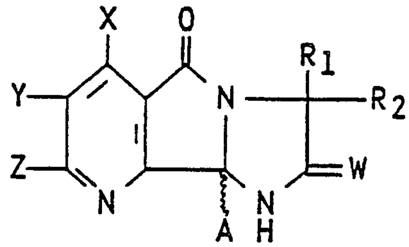

wherein

$R_1$ and $R_2$ each represent $C_1$—$C_3$ alkyl or cyclopropyl, with the proviso that the sum of the number of carbon atoms in $R_1$ and $R_2$ is 2 to 5; and when $R_1$ and $R_2$ are taken together with the carbon to which they are attached, they may form a $C_3$—$C_6$ cycloalkyl ring optionally substituted with methyl;

W is oxygen or sulfur;

A is hydrogen, hydroxyl, $C_3$—$C_6$ alkenyloxy, $C_3$—$C_6$ alkynyloxy, $C_1$—$C_6$ alkylthio, $NR_{13}R_{14}$ or $C_1$—$C_6$ alkoxy optionally substituted with phenyl, halophenyl, $C_1$—$C_3$ alkylphenyl, $C_1$—$C_3$ alkoxyphenyl or di-$C_1$—$C_3$ alkylaminophenyl;

$R_{13}$ is hydrogen, $C_1$—$C_4$ alkyl optionally substituted with phenyl, halophenyl, $C_1$—$C_3$ alkylphenyl or $C_1$—$C_3$ alkoxyphenyl;

$R_{14}$ is hydrogen or $C_1$—$C_4$ alkyl;

X is hydrogen, halogen or methyl;

Y and Z are each hydrogen, halogen, $C_1$—$C_6$ alkyl, $C_1$—$C_4$ hydroxyalkyl, $C_1$—$C_6$ alkoxy, $C_1$—$C_4$ alkylthio, phenoxy, $C_1$—$C_4$ haloalkyl, $OCF_2CHF_2$, $OCF_3$, $OCHF_2$, nitro, cyano, $NR_4R_5$, $C_3$—$C_8$ straight or branched alkenyloxy optionally substituted with one to three halogens, $C_3$—$C_8$ straight or branched alkynyloxy optionally substituted with one to three halogens, or phenyl optionally substituted with one $C_1$—$C_4$ alkyl, $C_1$—$C_4$ alkoxy or halogen;

$R_4$ is hydrogen or $C_1$—$C_4$ alkyl;

$R_5$ is $C_1$—$C_4$ alkyl;

And, when taken together, Y and Z may form a ring in which YZ is represented by

(1) the structure: $-(CH_2)_n-$, where n is an integer of 2, 3 or 4, provided that X is hydrogen; or

(2) by the structure:

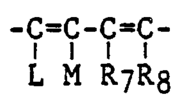

where, L, M, $R_7$ and $R_8$ each represent hydrogen, halogen, $C_1$—$C_4$ alkyl, $C_1$—$C_4$ alkoxy, $C_1$—$C_4$ alkylthio, $C_1$—$C_4$ alkylsulfonyl, $C_1$—$C_4$ haloalkyl, $NO_2$, CN, phenyl, phenoxy, amino, $OCF_3$, $OCHF_2$, $OCF_2CHF_2$, $C_1$—$C_4$ alkylamino, dialkyl($C_1$—$C_4$)amino, chlorophenyl, methylphenyl, $C_3$—$C_8$ straight or branched alkenyloxy optionally substituted with one to three halogens, $C_3$—$C_8$ straight or branched alkynyloxy optionally substituted with one to three halogens, or phenoxy substituted with one Cl, $CF_3$, $NO_2$ or $CH_3$ group, with the proviso that only one of L, M, $R_7$ or $R_8$ may represent a substituent other than hydrogen, halogen, $C_1$—$C_4$ alkyl or $C_1$—$C_4$ alkoxy; or

(3) by the structures:

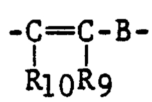 , 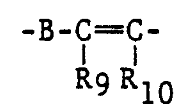 , 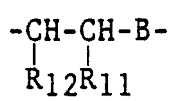 , or 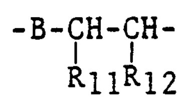 ;

where B is oxygen or sulfur; $R_9$ and $R_{10}$ each represent hydrogen, halogen, phenyl, or $C_1$—$C_4$ alkyl; $R_{11}$ and $R_{12}$ each represent hydrogen, $C_1$—$C_4$ alkyl or phenyl;
and when $R_1$ and $R_2$ are not the same, the *cis*- or *trans*-isomers or mixtures thereof or the optical isomers (*cis*- or *trans*- or mixtures thereof).

5. A method according to Claim 4, wherein A, $R_1$, $R_2$, W and X are as defined in said Claim 1 above; Y and Z each, independently, represent hydrogen, halogen, $C_1$—$C_6$ alkyl, $C_1$—$C_6$ alkoxy, CN, $NO_2$, $OCF_3$, $OCHF_2$, $OCF_2CHF_2$, phenoxy, $C_1$—$C_4$ haloalkyl, $C_1$—$C_4$ alkylthio, $C_1$—$C_4$ hydroxyalkyl, $NR_4R_5$, $C_3$—$C_8$ straight or branched alkenyloxy optionally substituted with one to three halogens, $C_3$—$C_8$ straight or branched alkynyloxy optionally substituted with one to three halogens, or phenyl optionally substituted with one $C_1$—$C_4$ alkyl, $C_1$—$C_4$ alkoxy or halogen; $R_4$ is hydrogen or $C_1$—$C_4$ alkyl; $R_5$ is $C_1$—$C_4$ alkyl; and when taken together Y and Z may form a ring in which YZ are represented by the structure: $-(CH_2)_n-$, where n is an integer of 2, 3 or 4, provided that X is hydrogen; and when $R_1$ and $R_2$ are not the same, the *cis*- or *trans*-isomers or mixtures thereof or the optical isomers (*cis*- or *trans*- or mixtures thereof).

6. A herbicidal composition comprising a herbicidally effective amount of a compound having the structure:

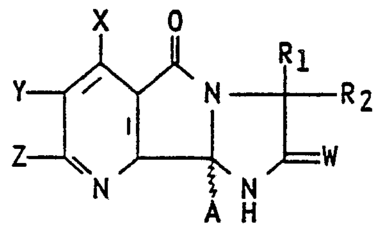

wherein $R_1$, $R_2$, A, W, X, Y and Z are as defined in Claim 4 and a non-toxic solid or liquid inert diluent therefore.

**Patentansprüche für die Vertragsstaaten: BE CH DE FR GB IT LI NL SE**

1. Verbindung mit der Struktur

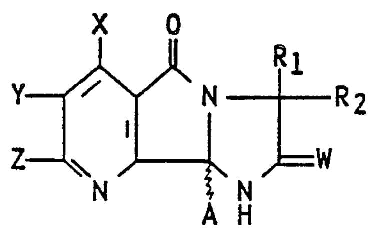

wobei

$R_1$ und $R_2$ jeweils für $C_{1-3}$-Alkyl oder Cyclopropyl stehen mit der Maßgabe, daß die Summe der Anzahl der Kohlenstoffatome in $R_1$ und $R_2$ 2 bis 5 beträgt, und wobei $R_1$ und $R_2$, wenn sie zusammengefaßt sind, mit dem Kohlenstoff, an den sie geknüpft sind, einen $C_{3-6}$-Cycloalkylring bilden können, der gegebenenfalls mit Methyl substituiert ist;

W für Sauerstoff oder Schwefel steht;

A für Wasserstoff, Hydroxyl, $C_{3-6}$-Alkenyloxy, $C_{3-6}$-Alkinyloxy, $C_{1-6}$-Alkylthio, $NR_{13}R_{14}$ oder $C_{1-6}$-Alkoxy, gegebenenfalls substituiert mit Phenyl, Halogenphenyl, $C_{1-3}$-Alkylphenyl, $C_{1-3}$-Alkoxyphenyl oder Di-$C_{1-3}$-Alkylaminophenyl steht;

$R_{13}$ für Wasserstoff, $C_{1-4}$-Alkyl, gegebenenfalls substituiert mit Phenyl, Halogenphenyl, $C_{1-3}$-Alkylphenyl oder $C_{1-3}$-Alkoxyphenyl, steht;

$R_{14}$ für Wasserstoff oder $C_{1-4}$-Alkyl steht;

X für Wasserstoff, Halogen oder Methyl steht;

Y und Z jeweils für Wasserstoff, Halogen, $C_{1-6}$-Alkyl, $C_{1-4}$-Hydroxyalkyl, $C_{1-6}$-Alkoxy, $C_{1-4}$-Alkylthio, Phenoxy, $C_{1-4}$-Halogenalkyl, $OCF_2CHF_2$, $OCF_3$, $OCHF_2$, Nitro, Cyano, $NR_4R_5$, $C_{3-8}$-geradkettiges oder verzweigtes Alkenyloxy, gegebenenfalls substituiert mit einem bis drei Halogenen, $C_{3-8}$-geradkettiges oder verzweigtes Alkinyloxy, gegebenenfalls substituiert mit einem bis drei Halogenen, oder Phenyl, gegebenenfalls substituiert mit einem $C_{1-4}$-Alkyl, $C_{1-4}$-Alkoxy oder Halogen stehen;

$R_4$ für Wasserstoff oder $C_{1-4}$-Alkyl steht;

$R_5$ für $C_{1-4}$-Alkyl steht;

und wobei

Y und Z unter Bildung eines Rings zusammengefaßt sein können, in dem YZ für folgende Strukturen steht:

(1) die Struktur $-(CH_2)_n-$, wobei n eine ganze Zahl von 2, 3 oder 4 ist mit der Maßgabe, daß X für Wasserstoff steht, oder

(2) die Struktur

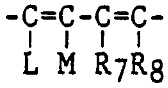

wobei L, M, $R_7$ und $R_8$ jeweils für Wasserstoff, Halogen, $C_{1-4}$-Alkyl, $C_{1-4}$-Alkoxy, $C_{1-4}$-Alkylthio, $C_{1-4}$-Alkylsulfonyl, $C_{1-4}$-Halogenalkyl, $NO_2$, CN, Phenyl, Phenoxy, Amino, $OCF_3$, $OCHF_2$, $OCF_2CHF_2$, $C_{1-4}$-Alkylamino, Dialkyl-($C_{1-4}$)-Amino, Chlorphenyl, Methylphenyl, $C_{3-8}$- geradkettiges oder verzweigtes Alkenyloxy, gegebenenfalls substituiert mit einem bis drei Halogenen, $C_{3-8}$- geradkettiges oder verzweigtes Alkinyloxy, gegebenenfalls substituiert mit einem bis drei Halogenen, oder Phenoxy, substituiert mit einer Cl-, $CF_3$-, $NO_2$- oder $CH_3$-Gruppe, stehen, mit der Maßgabe, daß nur einer von L, M, $R_7$ oder $R_8$ für einen Substituenten stehen kann, der nicht Wasserstoff, Halogen, $C_{1-4}$-Alkyl oder $C_{1-4}$-Alkoxy ist; und daß, falls A für OH steht, mindestens einer von L, M, $R_7$ und $R_8$ nicht Wasserstoff ist; oder

(3) die Strukturen

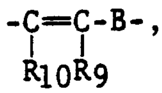 , 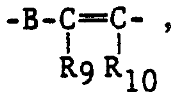 , 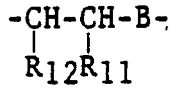 oder 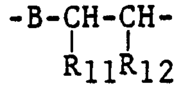 ,

wobei B für Sauerstoff oder Schwefel steht; $R_9$ und $R_{10}$ jeweils für Wasserstoff, Halogen, Phenyl oder $C_{1-4}$-Alkyl stehen; $R_{11}$ und $R_{12}$ jeweils für Wasserstoff, $C_{1-4}$-Alkyl oder Phenyl stehen; sowie die cis- oder trans-Isomeren oder Mischungen derselben oder die optischen Isomeren (cis- oder trans- oder Mischungen derselben), falls $R_1$ und $R_2$ nicht die gleiche Bedeutung haben.

2. Verbindung gemäß Anspruch 1, wobei A, $R_1$, $R_2$, W und X wie in Anspruch 1 definiert sind; Y und Z jeweils unabhängig für Wasserstoff, Halogen, $C_{1-6}$-Alkyl, $C_{1-6}$-Alkoxy, CN, $NO_2$, $OCF_3$, $OCHF_2$, $OCF_2CHF_2$, Phenoxy, $C_{1-4}$-Halogenalkyl, $C_{1-4}$-Alkylthio, $C_{1-4}$-Hydroxyalkyl, $NR_4R_5$, $C_{3-8}$- geradkettiges oder verzweigtes Alkenyloxy, gegebenenfalls substituiert mit einem bis drei Halogenen, $C_{3-8}$- geradkettiges oder verzweigtes Alkinyloxy, gegebenenfalls substituiert mit einem bis drei Halogenen, oder Phenyl, gegebenenfalls substituiert mit einem $C_{1-4}$-Alkyl, $C_{1-4}$-Alkoxy oder Halogen, stehen; $R_4$ für Wasserstoff oder $C_{1-4}$-Alkyl steht; $R_5$ für $C_{1-4}$-Alkyl steht; und wobei X und Z, falls sie zusammengefaßt sind, einen Ring bilden können, in dem YZ für die Struktur $-(CH_2)_n-$ steht, wobei n eine ganze Zahl von 2, 3 oder 4 ist, mit der Maßgabe, daß X für Wasserstoff steht; sowie die cis- oder trans-Isomeren oder Mischungen derselben oder die optischen Isomeren (cis- oder trans- oder Mischungen derselben), falls $R_1$ und $R_2$ nicht die gleiche Bedeutung haben.

3. Verfahren zur Herstellung einer Verbindung mit der Struktur

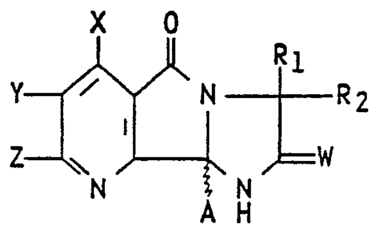

wobei $R_1$, $R_2$, A, W, X, Y und Z wie in Anspruch 1 definiert snd, umfassend die Umsetzung einer Verbindung mit der Formel

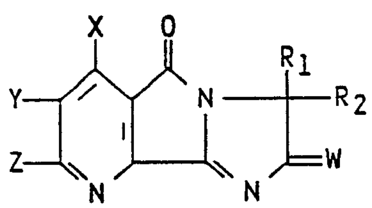

wobei $R_1$, $R_2$, W, X, Y und Z wie oben beschrieben sind, mit mindestens einem Äquivalent eines Nucleophils der Formel AH, wobei A die oben angegebene Bedeutung hat.

4. Verfahren zur Herstellung einer Verbindung mit der Struktur

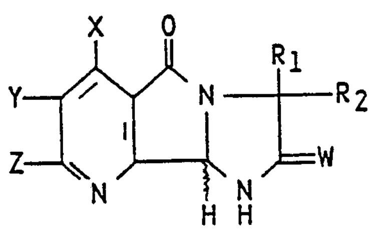

wobei $R_1$, $R_2$, W, X, Y und Z wie in Anspruch 1 definiert sind, umfassend die Umsetzung einer Verbindung mit der Struktur

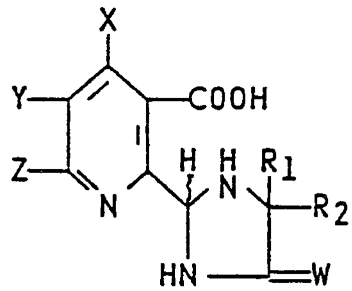

wobei $R_1$, $R_2$, W, X, Y und Z wie oben beschrieben sind, mit mindestens einem molaren Äquivalent Essigsäureanhydrid in Anwesenheit von Pyridin und Acetonitril, wobei das gewünschte Produkt gebildet wird.

5. Verfahren zur Herstellung einer Verbindung mit der Formel

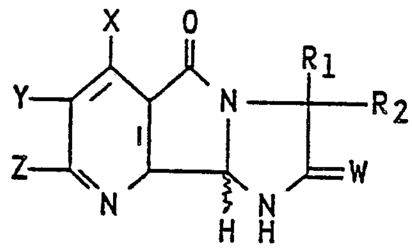

wobei $R_1$, $R_2$, W, X, Y und Z wie in Anspruch 1 definiert sind, umfassend die Umsetzung einer Verbindung der Formel

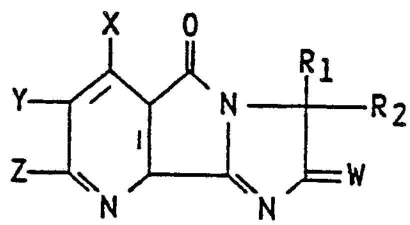

wobei X, Y, Z, $R_1$, $R_2$ und W wie oben beschrieben sind, mit mindestens einem molaren Äquivalent Natriumborhydrid oder Lithiumborhydrid, wobei das angestrebte Produkt gebildet wird.

6. Verfahren zur Bekämpfung von unerwünschten einkeimblättrigen und zweikeimblättrigen Pflanzenspezies, umfassend die Applikation einer herbizidwirksamen Menge einer Verbindung der folgenden Struktur auf die Blätter der Pflanzen oder auf den Boden, der Samen oder andere Fortpflanzungsorgane derselben enthält,

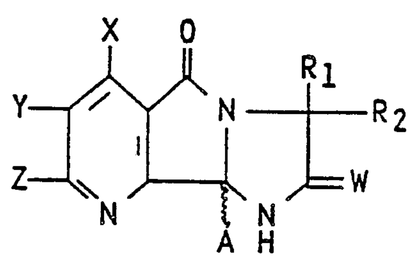

wobei

$R_1$ und $R_2$ jeweils für $C_{1-3}$-Alkyl oder Cyclopropyl stehen mit der Maßgabe, daß die Summe der Anzahl der Kohlenstoffatome in $R_1$ und $R_2$ 2 bis 5 beträgt, und wobei $R_1$ und $R_2$, wenn sie zusammengefaßt sind, mit dem Kohlenstoff, an den sie geknüpft sind, einen $C_{3-6}$-Cycloalkylring bilden können, der gegebenenfalls mit Methyl substituiert ist;

W für Sauerstoff oder Schwefel steht;

A für Wasserstoff, Hydroxyl, $C_{3-6}$-Alkenyloxy, $C_{3-6}$-Alkinyloxy, $C_{1-6}$-Alkylthio, $NR_{13}R_{14}$ oder $C_{1-6}$-Alkoxy, gegebenenfalls substituiert mit Phenyl, Halogenphenyl, $C_{1-3}$-Alkylphenyl, $C_{1-3}$-Alkoxyphenyl oder Di-$C_{1-3}$-Alkylaminophenyl steht;

$R_{13}$ für Wasserstoff, $C_{1-4}$-Alkyl, gegebenenfalls substituiert mit Phenyl, Halogenphenyl, $C_{1-3}$-Alkylphenyl oder $C_{1-3}$-Alkoxyphenyl, steht;

$R_{14}$ für Wasserstoff oder $C_{1-4}$-Alkyl steht;

X für Wasserstoff, Halogen oder Methyl steht;

Y und Z jeweils für Wasserstoff, Halogen, $C_{1-6}$-Alkyl, $C_{1-4}$-Hydroxyalkyl, $C_{1-6}$-Alkoxy, $C_{1-4}$-Alkylthio, Phenoxy, $C_{1-4}$-Halogenalkyl, $OCF_2CHF_2$, $OCF_3$, $OCHF_2$, Nitro, Cyano, $NR_4R_5$, $C_{3-8}$-geradkettiges oder verzweigtes Alkenyloxy, gegebenenfalls substituiert mit einem bis drei Halogenen, $C_{3-8}$-geradkettiges oder verzweigtes Alkinyloxy, gegebenenfalls substituiert mit einem bis drei Halogenen, oder Phenyl, gegebenenfalls substituiert mit einem $C_{1-4}$-Alkyl, $C_{1-4}$-Alkoxy oder Halogen stehen;

$R_4$ für Wasserstoff oder $C_{1-4}$-Alkyl steht;

$R_5$ für $C_{1-4}$-Alkyl steht;

und wobei

Y und Z unter Bildung eines Rings zusammengefaßt sein können, in dem YZ für folgende Strukturen steht:

(1) die Struktur $-(CH_2)_n-$, wobei n eine ganze Zahl von 2, 3 oder 4 ist mit der Maßgabe, daß X für Wasserstoff steht, oder

(2) die Struktur

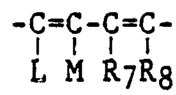

wobei L, M, $R_7$ und $R_8$ jeweils für Wasserstoff, Halogen, $C_{1-4}$-Alkyl, $C_{1-4}$-Alkoxy, $C_{1-4}$-Alkylthio, $C_{1-4}$-Alkylsulfonyl, $C_{1-4}$-Halogenalkyl, $NO_2$, CN, Phenyl, Phenoxy, Amino, $OCF_3$, $OCHF_2$, $OCF_2CHF_2$, $C_{1-4}$-Alkylamino, Dialkyl-$(C_{1-4})$-Amino, Chlorphenyl, Methylphenyl, $C_{3-8}$- geradkettiges oder verzweigtes Alkenyloxy, gegebenenfalls substituiert mit einem bis drei Halogenen, $C_{3-8}$- geradkettiges oder verzweigtes Alkinyloxy, gegebenenfalls substituiert mit einem bis drei Halogenen, oder Phenoxy, substituiert mit einer Cl-, $CF_3$-, $NO_2$- oder $CH_3$-Gruppe, stehen, mit der Maßgabe, daß nur einer von L, M, $R_7$ oder $R_8$ für einen Substituenten stehen kann, der nicht Wasserstoff, Halogen, $C_{1-4}$-Alkyl oder $C_{1-4}$-Alkoxy ist; und daß, falls A für OH steht, mindestens einer von L, M, $R_7$ und $R_8$ nicht Wasserstoff ist; oder

(3) die Strukturen

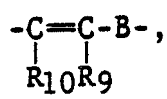, 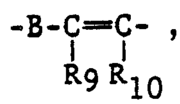, 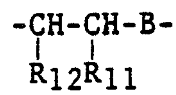 oder 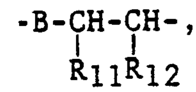,

wobei B für Sauerstoff oder Schwefel steht; $R_9$ und $R_{10}$ jeweils für Wasserstoff, Halogen, Phenyl oder $C_{1-4}$-Alkyl stehen; $R_{11}$ und $R_{12}$ jeweils für Wasserstoff, $C_{1-4}$-Alkyl oder Phenyl stehen; sowie die cis- oder trans-Isomeren oder Mischungen derselben oder die optischen Isomeren (cis- oder trans- oder Mischungen derselben), falls $R_1$ und $R_2$ nicht die gleiche Bedeutung haben.

7. Verfahren gemäß Anspruch 6, wobei A, $R_1$, $R_2$, W und X wie in Anspruch 1 definiert sind; Y und Z jeweils unabhängig für Wasserstoff, Halogen, $C_{1-6}$-Alkyl, $C_{1-6}$-Alkoxy, CN, $NO_2$, $OCF_3$, $OCHF_2$, $OCF_2CHF_2$, Phenoxy, $C_{1-4}$-Halogenalkyl, $C_{1-4}$-Alkylthio, $C_{1-4}$-Hydroxyalkyl, $NR_4R_5$, $C_{3-8}$- geradkettiges oder verzweigtes Alkenyloxy, gegebenenfalls substituiert mit einem bis drei Halogenen, $C_{3-8}$- geradkettiges oder verzweigtes Alkinyloxy, gegebenenfalls substituiert mit einem bis drei Halogenen, oder Phenyl, gegebenenfalls substituiert mit einem $C_{1-4}$-Alkyl, $C_{1-4}$-Alkoxy oder Halogen, stehen; $R_4$ für Wasserstoff oder $C_{1-4}$-Alkyl steht; $R_5$ für $C_{1-4}$-Alkyl steht; und wobei X und Z, falls sie zusammengefaßt sind, einen Ring bilden können, in dem YZ für die Struktur $-(CH_2)_n-$ steht, wobei n eine ganze Zahl von 2, 3 oder 4 ist, mit der Maßgabe, daß X für Wasserstoff steht; sowie die cis- oder trans-Isomeren oder Mischungen derselben oder die optischen Isomeren (cis- oder trans- oder Mischungen derselben), falls $R_1$ und $R_2$ nicht die gleiche Bedeutung haben.

8. Herbizide Zusammensetzung, umfassend eine herbizidwirksame Menge einer Verbindung mit der Struktur

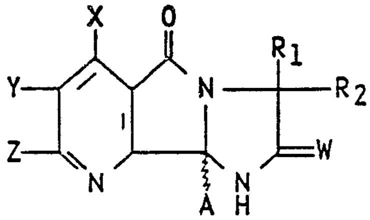

wobei $R_1$, $R_2$, A, W, X, Y und Z wie in Anspruch 6 definiert sind, sowie ein nicht-toxisches festes oder flüssiges inertes Verdünnungsmittel dafür.

**Patentansprüche für den Vertragsstaat: AT**

1. Verfahren zur Herstellung einer Verbindung mit der Struktur

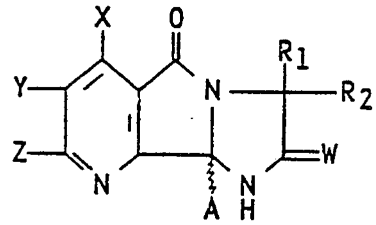

wobei

$R_1$ und $R_2$ jeweils für $C_{1-3}$-Alkyl oder Cyclopropyl stehen mit der Maßgabe, daß die Summe der Anzahl der Kohlenstoffatome in $R_1$ und $R_2$ 2 bis 5 beträgt, und wobei $R_1$ und $R_2$, wenn sie zusammengefaßt sind, mit dem Kohlenstoff, an den sie geknüpft sind, einen $C_{3-6}$-Cycloalkylring bilden können, der gegebenenfalls mit Methyl substituiert ist;

W für Sauerstoff oder Schwefel steht;

A für Wasserstoff, Hydroxyl, $C_{3-6}$-Alkenyloxy, $C_{3-6}$-Alkinyloxy, $C_{1-6}$-Alkylthio, $NR_{13}R_{14}$ oder $C_{1-6}$-Alkoxy, gegebenenfalls substituiert mit Phenyl, Halogenphenyl, $C_{1-3}$-Alkylphenyl, $C_{1-3}$-Alkoxyphenyl oder Di-$C_{1-3}$-Alkylaminophenyl steht;

$R_{13}$ für Wasserstoff, $C_{1-4}$-Alkyl, gegebenenfalls substituiert mit Phenyl, Halogenphenyl, $C_{1-3}$-Alkylphenyl oder $C_{1-3}$-Alkoxyphenyl, steht;

$R_{14}$ für Wasserstoff oder $C_{1-4}$-Alkyl steht;

X für Wasserstoff, Halogen oder Methyl steht;

Y und Z jeweils für Wasserstoff, Halogen, $C_{1-6}$-Alkyl, $C_{1-4}$-Hydroxyalkyl, $C_{1-6}$-Alkoxy, $C_{1-4}$-Alkylthio, Phenoxy, $C_{1-4}$-Halogenalkyl, $OCF_2CHF_2$, $OCF_3$, $OCHF_2$, Nitro, Cyano, $NR_4R_5$, $C_{3-8}$-geradkettiges oder verzweigtes Alkenyloxy, gegebenenfalls substituiert mit einem bis drei Halogenen, $C_{3-8}$-geradkettiges oder verzweigtes Alkinyloxy, gegebenenfalls substituiert mit einem bis drei Halogenen, oder Phenyl, gegebenenfalls substituiert mit einem $C_{1-4}$-Alkyl, $C_{1-4}$-Alkoxy oder Halogen stehen;

$R_4$ für Wasserstoff oder $C_{1-4}$-Alkyl steht;

$R_5$ für $C_{1-4}$-Alkyl steht;

und wobei

Y und Z unter Bildung eines Rings zusammengefaßt sein können, in dem YZ für folgende Strukturen steht:

(1) die Struktur $-(CH_2)_n-$, wobei n eine ganze Zahl von 2, 3 oder 4 ist mit der Maßgabe, daß X für Wasserstoff steht, oder

(2) die Struktur

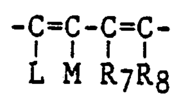

wobei L, M, $R_7$ und $R_8$ jeweils für Wasserstoff, Halogen, $C_{1-4}$-Alkyl, $C_{1-4}$-Alkoxy, $C_{1-4}$-Alkylthio, $C_{1-4}$-Alkylsulfonyl, $C_{1-4}$-Halogenalkyl, $NO_2$, CN, Phenyl, Phenoxy, Amino, $OCF_3$, $OCHF_2$, $OCF_2CHF_2$, $C_{1-4}$-Alkylamino, Dialkyl-($C_{1-4}$)-Amino, Chlorphenyl, Methylphenyl, $C_{3-8}$- geradkettiges oder verzweigtes Alkenyloxy, gegebenenfalls substituiert mit einem bis drei Halogenen, $C_{3-8}$- geradkettiges oder verzweigtes Alkinyloxy, gegebenenfalls substituiert mit einem bis drei Halogenen, oder Phenoxy, substituiert mit einer Cl-, $CF_3$-, $NO_2$- oder $CH_3$-Gruppe, stehen, mit der Maßgabe, daß nur einer von L, M, $R_7$ oder $R_8$ für einen Substituenten stehen kann, der nicht Wasserstoff, Halogen, $C_{1-4}$-Alkyl oder $C_{1-4}$-Alkoxy ist: und daß, falls A für OH steht, mindestens einer von L, M, $R_7$ und $R_8$ nicht Wasserstoff ist; oder

(3) die Strukturen

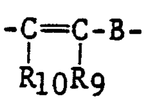 , 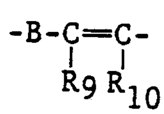 , 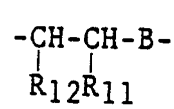 oder 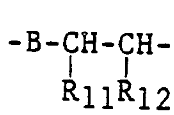 ,

wobei B für Sauerstoff oder Schwefel steht; $R_9$ und $R_{10}$ jeweils für Wasserstoff, Halogen, Phenyl oder $C_{1-4}$-Alkyl stehen; $R_{11}$ und $R_{12}$ jeweils für Wasserstoff, $C_{1-4}$-Alkyl oder Phenyl stehen; sowie die cis- oder trans-Isomeren oder Mischungen derselben oder die optischen Isomeren (cis- oder trans- oder Mischungen

derselben), falls $R_1$ und $R_2$ nicht die gleiche Bedeutung haben; umfassend die Umsetzung einer Verbindung mit der Formel

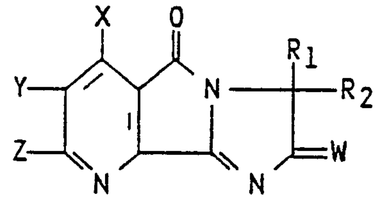

wobei $R_1$, $R_2$, W, X, Y und Z wie oben beschrieben sind, mit mindestens einem Äquivalent eines Nucleophils der Formel AH, wobei A die oben angegebene Bedeutung hat.

2. Verfahren zur Herstellung einer Verbindung mit der Struktur

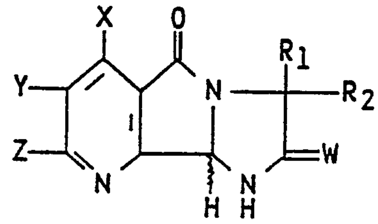

wobei $R_1$, $R_2$, W, X, Y und Z wie in Anspruch 1 definiert sind, umfassend die Umsetzung einer Verbindung mit der Struktur

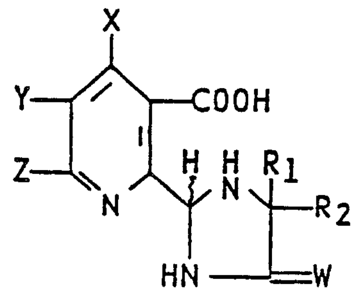

wobei $R_1$, $R_2$, W, X, Y und Z wie oben beschrieben sind, mit mindestens einem molaren Äquivalent Essigsäureanhydrid in Anwesenheit von Pyridin und Acetonitril, wobei das gewünschte Produkt gebildet wird.

3. Verfahren zur Herstellung einer Verbindung mit der Formel

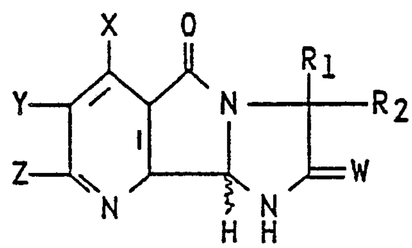

wobei $R_1$, $R_2$, W, X, Y und Z wie in Anspruch 1 definiert sind, umfassend die Umsetzung einer Verbindung der Formel

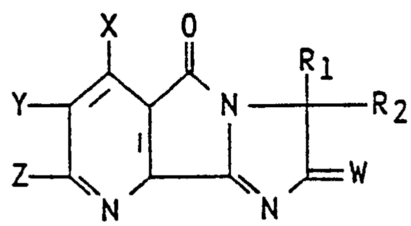

wobei X, Y, Z, $R_1$, $R_2$ und W wie oben beschrieben sind, mit mindestens einer äquimolaren Menge Natriumborhydrid oder Lithiumborhydrid, wobei das angestrebte Produkt gebildet wird.

4. Verfahren zur Bekämpfung von unerwünschten einkeimblättrigen und zweikeimblättrigen Pflanzenspezies, umfassend die Applikation einer herbizidwirksamen Menge einer Verbindung der folgenden Struktur auf die Blätter der Pflanzen oder auf den Boden, der Samen oder andere Fortpflanzungsorgane derselben enthält,

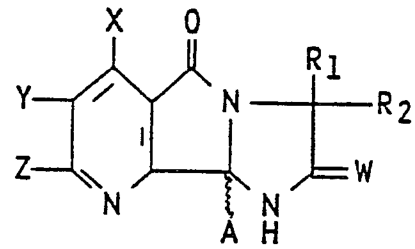

wobei

$R_1$ und $R_2$ jeweils für $C_{1-3}$-Alkyl oder Cyclopropyl stehen mit der Maßgabe, daß die Summe der Anzahl der Kohlenstoffatome in $R_1$ und $R_2$ 2 bis 5 beträgt, und wobei $R_1$ und $R_2$, wenn sie zusammengefaßt sind, mit dem Kohlenstoff, an den sie geknüpft sind, einen $C_{3-6}$-Cycloalkylring bilden können, der gegebenenfalls mit Methyl substituiert ist;

W für Sauerstoff oder Schwefel steht;

A für Wasserstoff, Hydroxyl, $C_{3-6}$-Alkenyloxy, $C_{3-6}$-Alkinyloxy, $C_{1-6}$-Alkylthio, $NR_{13}R_{14}$ oder $C_{1-6}$-Alkoxy, gegebenenfalls substituiert mit Phenyl, Halogenphenyl, $C_{1\geq3}$-Alkylphenyl, $C_{1-3}$-Alkoxyphenyl oder Di-$C_{1-3}$-Alkylaminophenyl steht;

$R_{13}$ für Wasserstoff, $C_{1-4}$-Alkyl, gegebenenfalls substituiert mit Phenyl, Halogenphenyl, $C_{1-3}$-Alkylphenyl oder $C_{1-3}$-Alkoxyphenyl, steht;

$R_{14}$ für Wasserstoff oder $C_{1-4}$-Alkyl steht;

X für Wasserstoff, Halogen oder Methyl steht;

Y und Z jeweils für Wasserstoff, Halogen, $C_{1-6}$-Alkyl, $C_{1-4}$-Hydroxyalkyl, $C_{1-6}$-Alkoxy, $C_{1-4}$-Alkylthio, Phenoxy, $C_{1-4}$-Halogenalkyl, $OCF_2CHF_2$, $OCF_3$, $OCHF_2$, Nitro, Cyano, $NR_4R_5$, $C_{3-8}$-geradkettiges oder verzweigtes Alkenyloxy, gegebenenfalls substituiert mit einem bis drei Halogenen, $C_{3-8}$-geradkettiges oder verzweigtes Alkinyloxy, gegebenenfalls substituiert mit einem bis drei Halogenen, oder Phenyl, gegebenenfalls substituiert mit einem $C_{1-4}$-Alkyl, $C_{1-4}$-Alkoxy oder Halogen stehen;

$R_4$ für Wasserstoff oder $C_{1-4}$-Alkyl steht;

$R_5$ für $C_{1-4}$-Alkyl steht;

und wobei

Y und Z unter Bildung eines Rings zusammengefaßt sein können, in dem YZ für folgende Strukturen steht:

(1) die Struktur $-(CH_2)_n-$, wobei n eine ganze Zahl von 2, 3 oder 4 ist mit der Maßgabe, daß X für Wasserstoff steht, oder

(2) die Struktur

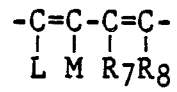

wobei L, M, $R_7$ und $R_8$ jeweils für Wasserstoff, Halogen, $C_{1-4}$-Alkyl, $C_{1-4}$-Alkoxy, $C_{1-4}$-Alkylthio, $C_{1-4}$-Alkylsulfonyl, $C_{1-4}$-Halogenalkyl, $NO_2$, CN, Phenyl, Phenoxy, Amino, $OCF_3$, $OCHF_2$, $OCF_2CHF_2$, $C_{1-4}$-Alkylamino, Dialkyl-($C_{1-4}$)-Amino, Chlorphenyl, Methylphenyl, $C_{3-8}$- geradkettiges oder verzweigtes Alkenyloxy, gegebenenfalls substituiert mit einem bis drei Halogenen, $C_{3-8}$- geradkettiges oder verzweigtes Alkinyloxy, gegebenenfalls substituiert mit einem bis drei Halogenen, oder Phenoxy, substituiert mit einer Cl-, $CF_3$-, $NO_2$- oder $CH_3$-Gruppe, stehen, mit der Maßgabe, daß nur einer von L, M, $R_7$ oder $R_8$ für einen Substituenten stehen kann, der nicht Wasserstoff, Halogen, $C_{1-4}$-Alkyl oder $C_{1-4}$-Alkoxy ist; und daß, falls A für OH steht, mindestens einer von L, M, $R_7$ und $R_8$ nicht Wasserstoff ist; oder

(3) die Strukturen

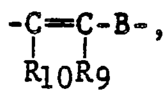, 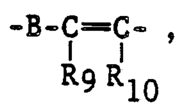, 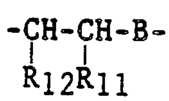 oder 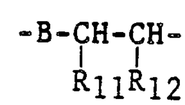,

wobei B für Sauerstoff oder Schwefel steht; $R_9$ und $R_{10}$ jeweils für Wasserstoff, Halogen, Phenyl oder $C_{1-4}$-Alkyl stehen; $R_{11}$ und $R_{12}$ jeweils für Wasserstoff, $C_{1-4}$-Alkyl oder Phenyl stehen; sowie die cis- oder trans-Isomeren oder Mischungen derselben oder die optischen Isomeren (cis- oder trans- oder Mikschungen derselben), falls $R_1$ und $R_2$ nicht die gleiche Bedeutung haben.

5. Verfahren gemäß Anspruch 4, wobei A, $R_1$, $R_2$, W und X wie in Anspruch 1 definiert sind; Y und Z jeweils unabhängig für Wasserstoff, Halogen, $C_{1-6}$-Alkyl, $C_{1-6}$-Alkoxy, CN, $NO_2$, $OCF_3$, $OCHF_2$, $OCF_2CHF_2$, Phenoxy, $C_{1-4}$-Halogenalkyl, $C_{1-4}$-Alkylthio, $C_{1-4}$-Hydroxyalkyl, $NR_4R_5$, $C_{3-8}$- geradkettiges oder

verzweigtes Alkenyloxy, gegebenenfalls substituiert mit einem bis drei Halogenen, $C_{3-8}$- geradkettiges oder verzweigtes Alkinyloxy, gegebenenfalls substituiert mit einem bis drei Halogenen, oder Phenyl, gegebenenfalls substituiert mit einem $C_{1-4}$-Alkyl, $C_{1-4}$-Alkoxy oder Halogen, stehen; $R_4$ für Wasserstoff oder $C_{1-4}$-Alkyl steht; $R_5$ für $C_{1-4}$-Alkyl steht; und wobei X und Z, falls sie zusammengefaßt sind, einen Ring bilden können, in dem YZ für die Struktur $-(CH_2)_n-$ steht, wobei n eine ganze Zahl von 2, 3 oder 4 ist, mit der Maßgabe, daß X für Wasserstoff steht; sowie die cis- oder trans-Isomeren oder Mischungen derselben oder die optischen Isomeren (cis- oder trans- oder Mischungen derselben), falls $R_1$ und $R_2$ nicht die gleiche Bedeutung haben.

6. Herbizide Zusammensetzung, umfassend eine herbizidwirksame Menge einer Verbindung mit der Struktur

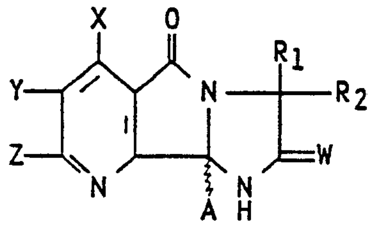

wobei $R_1$, $R_2$, A, W, X, Y und Z wie in Anspruch 4 definiert sind, sowie ein nicht-toxisches festes oder flüssiges inertes Verdünnungsmittel dafür.

**Revendications pour les Etats contractants: BE CH DE FR GB IT LI NL SE**

1. Composé répondant à la structure:

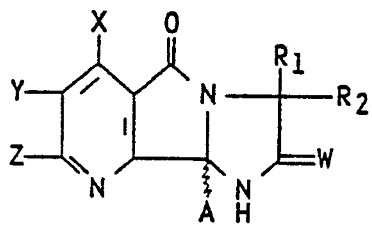

dans laquelle

$R_1$ et $R_2$ représentent chacun un alkyle en $C_1$—$C_3$ ou un cyclopropyle, sous réserve que la somme du nombre des atomes de carbone dans $R_1$ et $R_2$ soit de 2 à 5; et, lorsque $R_1$ et $R_2$ sont pris ensemble avec l'atome de carbone auquel ils sont fixés, ils peuvent former un cycle cycloalkyle en $C_3$—$C_6$ facultativement substitué par un méthyle;

W est un oxygène ou un soufre;

A est un hydrogène, un hydroxyle, un alcényloxy en $C_3$—$C_6$, un alcynyloxy en $C_3$—$C_6$, un alkylthio en $C_1$—$C_6$, $NR_{13}R_{14}$ ou un alcoxy en $C_1$—$C_6$ facultativement substitué par un phényle, un halogénophényle, un (alkyl en $C_1$—$C_3$)phényle, un (alcoxy en $C_1$—$C_3$)phényle ou un di(alkyl en $C_1$—$C_3$)aminophényle;

$R_{13}$ est un hydrogène, un alkyle en $C_1$—$C_4$ facultativement substitué par un phényle, un halogénophényle, un (alkyl en $C_1$—$C_3$)phényle ou un (alcoxy en $C_1$—$C_3$)phényle;

$R_{14}$ est un hydrogène ou un alkyle en $C_1$—$C_4$;

X est un hydrogène, un halogène ou un méthyle;

Y et Z sont chacun un hydrogène, un halogène, un alkyle en $C_1$—$C_6$, un hydroxyalkyle en $C_1$—$C_4$, un alcoxy en $C_1$—$C_6$, un alkylthio en $C_1$—$C_4$, un phénoxy, un halogénoalkyle en $C_1$—$C_4$, $OCF_2CHF_2$, $OCF_3$, $OCHF_2$, un nitro, un cyano, $NR_4R_5$, un alcényloxy en $C_3$—$C_8$ droit ou ramifié facultativement substitué par un à trois halogènes, un alcynyloxy en $C_3$—$C_8$ droit ou ramifié facultativement substitué par un à trois halogènes, ou un phényle facultativement substitué par un alkyle en $C_1$—$C_4$, un alcoxy en $C_1$—$C_4$ ou un halogène;

$R_4$ est un hydrogène ou un alkyle en $C_1$—$C_4$;

$R_5$ est un alkyle en $C_1$—$C_4$;

et, ensemble Y et Z peuvent former un cycle dans lequel YZ est représenté par

(1) la structure: $-(CH_2)_n-$, dans laquelle n est un entier valant 2, 3 ou 4 sous réserve que X soit un hydrogène; ou

(2) par la structure:

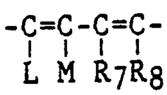

dans laquelle L, M, $R_7$ et $R_8$ représentent chacun un hydrogène, un halogène, un alkyle en $C_1$—$C_4$, un alcoxy en $C_1$—$C_4$, un alkylthio en $C_1$—$C_4$, un alkylsulfonyle en $C_1$—$C_4$, un halogénoalkyle en $C_1$—$C_4$, $NO_2$, CN, un

phényle, un phénoxy, un amino, $OCF_3$, $OCHF_2$, $OCF_2CHF_2$, un alkylamino en $C_1$—$C_4$, un di(alkyl en $C_1$—$C_4$)-amino, un chlorophényle, un méthylphényle, un alcényloxy en $C_3$—$C_8$ droit ou ramifié facultativement substitué par un à trois halogènes, un alcynyloxy en $C_3$—$C_8$ droit ou ramifié facultativement substitué par un à trois halogènes, ou un phénoxy substitué par un groupe Cl, $CF_3$, $NO_2$ ou $CH_3$, sous réserve qu'un seul de L, M, $R_7$ ou $R_8$ puisse représenter un substituant autre qu'un hydrogène, un halogène, un alkyle en $C_1$—$C_4$ ou un alcoxy en $C_1$—$C_4$; et lorsque A est OH, au moins un de L, M, $R_7$ et $R_8$ est autre qu'un hydrogène; ou

(3) par les structures:

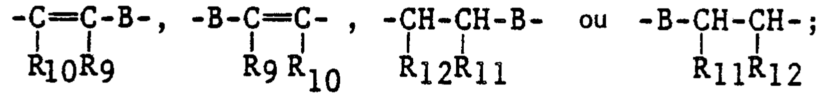

où B est un oxygène ou un soufre; $R_9$ et $R_{10}$ représentent chacun un hydrogène, un halogène, un phényle ou un alkyle en $C_1$—$C_4$; $R_{11}$ et $R_{12}$ représentent chacun un hydrogène, un alkyle en $C_1$—$C_4$ ou un phényle; et lorsque $R_1$ et $R_2$ sont différents, les isomères cis ou trans, ou leurs mélanges, ou les isomères optiques (cis ou trans, ou leurs mélanges).

2. Composé selon la revendication 1, où A, $R_1$, $R_2$, W et X sont comme défini dans la revendication 1; Y et Z représentent chacun indépendamment un hydrogène, un halogène, un alkyle en $C_1$—$C_6$, un alcoxy en $C_1$—$C_6$, CN, $NO_2$, $OCF_3$, $OCHF_2$, $OCF_2CHF_2$, un phénoxy, un halogénoalkyle en $C_1$—$C_4$, un alkylthio en $C_1$—$C_4$, un hydroxyalkyle en $C_1$—$C_4$, $NR_4R_5$, un alcényloxy en $C_3$—$C_8$ droit ou ramifié facultativement substitué par un à trois halogènes, un alcynyloxy en $C_1$—$C_8$ droit ou ramifié facultativement substitué par un à trois halogènes ou un phényle facultativement substitué par un alkyle en $C_1$—$C_4$, un alcoxy en $C_1$—$C_4$ ou un halogène; $R_4$ est un hydrogène ou un alkyle en $C_1$—$C_4$; $R_5$ est un alkyle en $C_1$—$C_4$; et ensemble X et Y peuvent former un cycle dans lequel YZ est représenté par la structure: $—(CH_2)_n—$, dans laquelle n est un entier valant 2, 3 ou 4, sous réserve que X soit un hydrogène; et, lorsque $R_1$ et $R_2$ sont différents, les isomères cis ou trans, ou leurs mélanges, ou les isomères optiques (cis ou trans, ou leurs mélanges).

3. Procédé pour préparer un composé de structure:

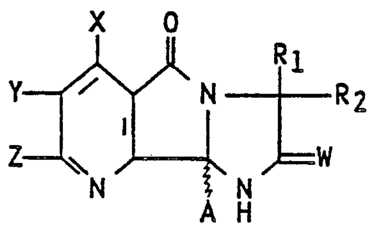

dans laquelle $R_1$, $R_2$, A, W, X, Y et Z sont comme défini dans la revendication 1, comprenant la réacton d'un composé de formule:

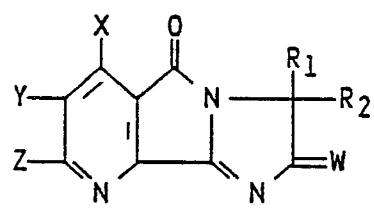

dans laquelle $R_1$, $R_2$, W, X, Y et Z sont comme décrit ci-dessus, avec au moins un équivalent d'un nucléophile de formule: AH, dans laquelle A est comme décrit ci-dessus.

4. Procédé pour la préparation d'un composé de structure:

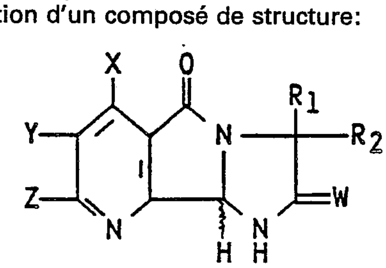

dans laquelle $R_1$, $R_2$, W, X, Y et Z sont comme défini dans la revendication, comprenant la réaction d'un composé de structure:

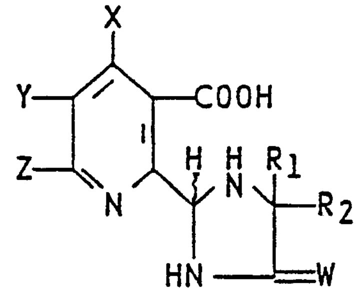

dans laquelle $R_1$, $R_2$, W, X, Y et Z sont comme décrit ci-dessus, avec au moins un équivalent molaire d'anhydride acétique en présence de pyridine et d'acétonitrile, pour former le produit désiré.

5. Procédé pour la préparation d'un composé de formule:

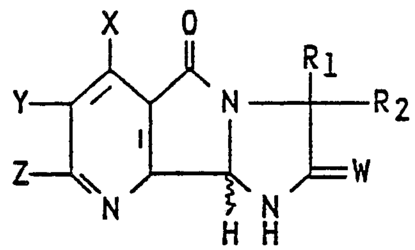

dans laquelle $R_1$, $R_2$, W, X, Y et Z sont comme défini dans la revendication 1, comprenant la réaction d'un composé de formule:

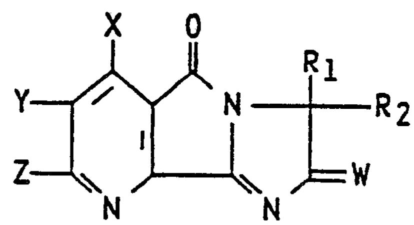

dans laquelle X, Y, Z, $R_1$, $R_2$ et W sont comme décrit ci-dessus, avec au moins une quantité équimolaire de borohydrure de sodium ou de borohydrure de lithium, pour former le produit désiré.

6. Procédé de lutte contre les espèces de plantes monocotylédones et dicotylédones indésirables comprenant: l'application, au feuillage desdites plantes ou au sol contenant des graines ou d'autres organes de leur propagation, d'une quantité herbicide efficace d'un composé de structure:

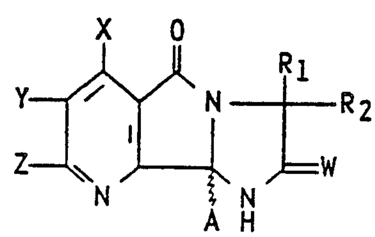

dans laquelle

$R_1$ et $R_2$ représentent chacun un alkyle en $C_1$—$C_3$ ou un cyclopropyle, sous réserve que la somme du nombre des atomes de carbone dans $R_1$ et $R_2$ soit de 2 à 5; et, lorsque $R_1$ et $R_2$ sont pris ensemble avec l'atome de carbone auquel ils sont fixés, ils peuvent former un cycle cycloalkyle en $C_3$—$C_6$ facultativement substitué par un méthyle;

W est un oxygène ou un soufre;

A est un hydrogène, un hydroxyle, un alcényloxy en $C_3$—$C_6$, un alcynyloxy en $C_3$—$C_6$, un alkylthio en $C_1$—$C_6$, $NR_{13}R_{14}$ ou un alcoxy en $C_1$—$C_6$ facultativement substitué par un phényle, un halogénophényle, un (alkyl en $C_1$—$C_3$)phényle, un (alcoxy en $C_1$—$C_3$)phényle ou un di(alkyl en $C_1$—$C_3$)aminophényle;

$R_{13}$ est un hydrogène, un alkyle en $C_1$—$C_4$ facultativement substitué par un phényle, un halogénophényle, un (alkyl en $C_1$—$C_3$)phényle ou un (alcoxy en $C_1$—$C_3$)phényle;

$R_{14}$ est un hydrogène ou un alkyle en $C_1$—$C_4$;

X est un hydrogène, un halogène ou un méthyle;

Y et Z sont chacun un hydrogène, un halogène, un alkyle en $C_1$—$C_6$, un hydroxyalkyle en $C_1$—$C_4$, un alcoxy en $C_1$—$C_6$, un alkylthio en $C_1$—$C_4$, un phénoxy, un halogénoalkyle en $C_1$—$C_4$, $OCF_2CHF_2$, $OCF_3$, $OCHF_2$, un nitro, un cyano, $NR_4R_5$, un alcényloxy en $C_3$—$C_8$ droit ou ramifié facultativement substitué par un à trois halogènes, un alcynyloxy en $C_3$—$C_8$ droit ou ramifié facultativement substitué par un à trois halogènes, ou un phényle facultativement substitué par un alkyle en $C_1$—$C_4$, un alcoxy en $C_1$—$C_4$ ou un halogène;

$R_4$ est un hydrogène ou un alkyle en $C_1$—$C_4$;

$R_5$ est un alkyle en $C_1$—$C_4$;

et, ensemble Y et Z peuvent former un cycle dans lequel YZ est représenté par

(1) la structure: $-(CH_2)_n-$, dans laquelle n est un entier valant 2, 3 ou 4 sous réserve que X soit un hydrogène; ou

(2) par la structure:

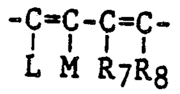

dans laquelle L, M, $R_7$ et $R_8$ représentent chacun un hydrogène, un halogène, un alkyle en $C_1$—$C_4$, un alcoxy en $C_1$—$C_4$, un alkylthio en $C_1$—$C_4$, un alkylsulfonyle en $C_1$—$C_4$, un halogénoalkyle en $C_1$—$C_4$, $NO_2$, CN, un phényle, un phénoxy, un amino, $OCF_3$, $OCHF_2$, $OCF_2CHF_2$, un alkylamino en $C_1$—$C_4$, un di(alkyl en $C_1$—$C_4$)-amino, un chlorophényle, un méthylphényle, un alcényloxy en $C_3$—$C_8$ droit ou ramifié facultativement substitué par un à trois halogènes, un alcynyloxy en $C_3$—$C_8$ droit ou ramifié facultativement substitué par un à trois halogènes, ou un phénoxy substitué par un groupe Cl, $CF_3$, $NO_2$ ou $CH_3$, sous réserve qu'un seul de L, M, $R_7$ ou $R_8$ puisse représenter un substituant autre qu'un hydrogène, un halogène, un alkyle en $C_1$—$C_4$ ou un alcoxy en $C_1$—$C_4$; ou

(3) par les structures:

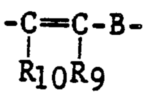 , 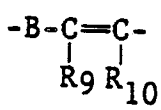 , 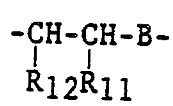 ou 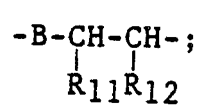 ;

où B est un oxygène ou un soufre; $R_9$ et $R_{10}$ représentent chacun un hydrogène, un halogène, un phényle ou un alkyle en $C_1$—$C_4$; $R_{11}$ et $R_{12}$ représentent chacun un hydrogène, un alkyle en $C_1$—$C_4$ ou un phényle; et lorsque $R_1$ et $R_2$ sont différents, les isomères cis ou trans, ou leurs mélanges, ou les isomères optiques (cis ou trans, ou leurs mélanges).

7. Procédé selon la revendication 6, où A, $R_1$, $R_2$, W et X sont comme défini dans la revendication 1; Y et Z représentent chacun indépendamment un hydrogène, un halogène, un alkyle en $C_1$—$C_6$ un alcoxy en $C_1$—$C_6$, CN, $NO_2$, $OCF_3$, $OCHF_2$, $OCF_2CHF_2$, un phénoxy, un halogénoalkyle en $C_1$—$C_4$, un alkylthio en $C_1$—$C_4$, un hydroxyalkyle en $C_1$—$C_4$, $NR_4R_5$, un alcényloxy en $C_3$—$C_8$ droit ou ramifié facultativement substitué par un à trois halogènes, un alcynyloxy en $C_1$—$C_8$ droit ou ramifié facultativement substitué par un à trois halogènes ou un phényle facultativement substitué par un alkyle en $C_1$—$C_4$, un alcoxy en $C_1$—$C_4$ ou un halogène; $R_4$ est un hydrogène ou un alkyle en $C_1$—$C_4$; $R_5$ est un alkyle en $C_1$—$C_4$; et ensemble X et Y peuvent former un cycle dans lequel YZ est représenté par la structure: $-(CH_2)_n-$, dans laquelle n est un entier valant 2, 3 ou 4, sous réserve que X soit un hydrogène; et, lorsque $R_1$ et $R_2$ sont différents, les isomères cis ou trans, ou leurs mélanges, ou les isomères optiques (cis ou trans, ou leurs mélanges).

8. Composition herbicide comprenant une quantité herbicide efficace d'un composé de structure:

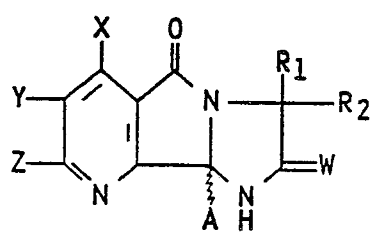

dans laquelle $R_1$, $R_2$, A, W, X, Y et Z sont comme défini dans la revendication 6 et un diluant inerte non toxique solide ou liquide de celui-ci.

**Revendications pour l'Etat contractant: AT**

1. Procédé pour préparer un composé de structure:

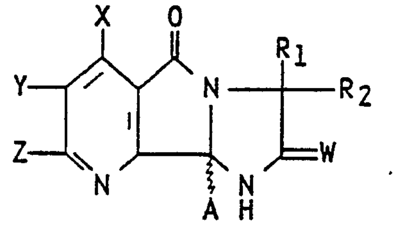

dans laquelle

$R_1$ et $R_2$ représentent chacun un alkyle en $C_1$—$C_3$ ou un cyclopropyle, sous réserve que la somme du nombre des atomes de carbone dans $R_1$ et $R_2$ soit de 2 à 5; et lorsque $R_1$ et $R_2$ sont pris ensemble avec l'atome de carbone auquel ils sont fixés, ils peuvent former un cycle cycloalkyle en $C_3$—$C_6$ facultativement substitué par un méthyle;

W est un oxygène ou un soufre;

A est un hydroxyle, un alcényloxy en $C_3$—$C_6$, un alcynyloxy en $C_3$—$C_6$, un alkylthio en $C_1$—$C_6$, $NR_{13}R_{14}$ ou un alcoxy en $C_1$—$C_6$ facultativement substitué par un phényle, un halogénophényle, un (alkyl en $C_1$—$C_3$)-phényle, un (alcoxy en $C_1$—$C_3$)phényle ou un di(alkyl en $C_1$—$C_3$)aminophényle;

$R_{13}$ est un hydrogène, un alkyle en $C_1$—$C_4$ facultativement substitué par un phényle, un halogéno-phényle, un (alkyl en $C_1$—$C_3$)phényle ou un (alcoxy en $C_1$—$C_3$)phényle;

$R_{14}$ est un hydrogène ou un alkyle en $C_1$—$C_4$;

X est un hydrogène, un halogène ou un méthyle;

Y et Z sont chacun un hydrogène, un halogène, un alkyle en $C_1$—$C_6$, un hydroxyalkyle en $C_1$—$C_4$, un alcoxy en $C_1$—$C_6$, un alkylthio en $C_1$—$C_4$, un phénoxy, un halogénoalkyle en $C_1$—$C_4$, $OCF_2CHF_2$, $OCF_3$, $OCHF_2$, un nitro, un cyano, $NR_4R_5$, un alcényloxy en $C_3$—$C_8$ droit ou ramifié facultativement substitué par un à trois halogènes, un alcynyloxy en $C_3$—$C_8$ droit ou ramifié facultativement substitué par un à trois halogènes, ou un phényle facultativement substitué par un alkyle en $C_1$—$C_4$, un alcoxy en $C_1$—$C_4$ ou un halogène;

$R_4$ est un hydrogène ou un alkyle en $C_1$—$C_4$;

$R_5$ est un alkyle en $C_1$—$C_4$;

et, ensemble Y et Z peuvent former un cycle dans lequel YZ est représenté par

(1) la structure: $-(CH_2)_n-$, dans laquelle n est un entier valant 2, 3 ou 4 sous réserve que X soit un hydrogène; ou

(2) par la structure:

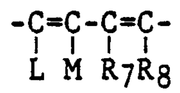

dans laquelle L, M, $R_7$ et $R_8$ représentent chacun un hydrogène, un halogène, un alkyle en $C_1$—$C_4$, un alcoxy en $C_1$—$C_4$, un alkylthio en $C_1$—$C_4$, un alkylsulfonyle en $C_1$—$C_4$, un halogénoalkyle en $C_1$—$C_4$, $NO_2$, CN, un phényle, un phénoxy, un amino, $OCF_3$, $OCHF_2$, $OCF_2CHF_2$, un alkylamino en $C_1$—$C_4$, un di(alkyl en $C_1$—$C_4$)-amino, un chlorophényle, un méthylphényle, un alcényloxy en $C_3$—$C_8$ droit ou ramifié facultativement substitué par un à trois halogènes, un alcynyloxy en $C_3$—$C_8$ droit ou ramifié facultativement substitué par un à trois halogènes, ou un phénoxy substitué par un groupe Cl, $CF_3$, $NO_2$ ou $CH_3$, sous réserve qu'un seul de L, M, $R_7$ ou $R_8$ puisse représenter un substituant autre qu'un hydrogène, un halogène, un alkyle en $C_1$—$C_4$ ou un alcoxy en $C_1$—$C_4$; et lorsque A est OH, au moins un de L, M, $R_7$ et $R_8$ est autre qu'un hydrogène; ou

(3) par les structures:

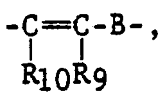 , 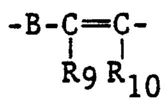 , 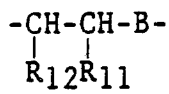 ou 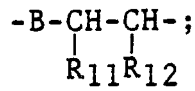

où B est un oxygène ou un soufre; $R_9$ et $R_{10}$ représentent chacun un hydrogène, un halogène, un phényle ou un alkyle en $C_1$—$C_4$; $R_{11}$ et $R_{12}$ représentent chacun un hydrogène, un alkyle en $C_1$—$C_4$ ou un phényle; et lorsque $R_1$ et $R_2$ sont différents, les isomères cis ou trans, ou leurs mélanges, ou les isomères optiques (cis ou trans, ou leurs mélanges); comprenant la réaction d'un comosé de formule:

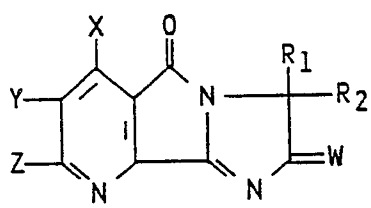

dans laquelle $R_1$, $R_2$, W, X, Y et Z sont comme décrit ci-dessus, avec au moins un équivalent d'un nucléophile de formule: AH, dans laquelle A est comme décrit ci-dessus.

2. Procédé pour la préparation d'un composé de structure:

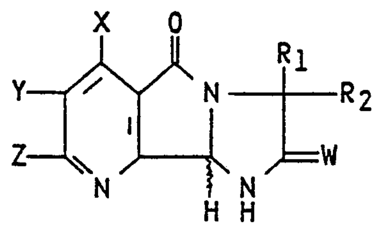

dans laquelle $R_1$, $R_2$, W, X, Y et Z sont comme défini dans la revendication, comprenant la réaction d'un composé de structure:

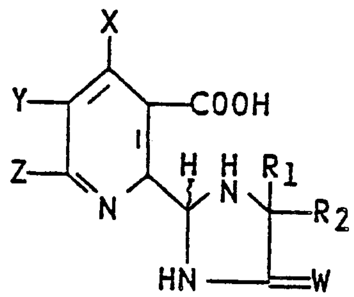

dans laquelle $R_1$, $R_2$, W, X, Y et Z sont comme décrit ci-dessus, avec au moins un équivalent molaire d'anhydride acétique en présence de pyridine et d'acétonitrile, pour former le produit désiré.

3. Procédé pour la préparation d'un composé de formule:

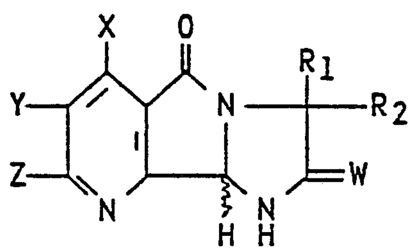

dans laquelle $R_1$, $R_2$, W, X, Y et Z sont comme défini dans la revendication 1, comprenant la réaction d'un composé de formule:

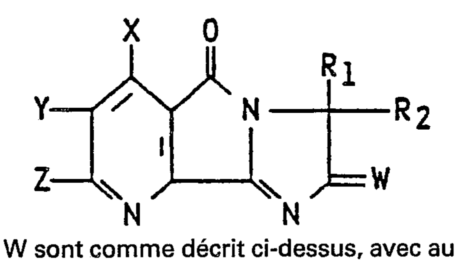

dans laquelle X, Y, Z, $R_1$, $R_2$ et W sont comme décrit ci-dessus, avec au moins une quantité équimolaire de borohydrure de sodium ou de borohydrure de lithium, pour former le produit désiré.

4. Procédé de lutte contre les espèces de plantes monocotylédones et dicotylédones indésirables comprenant: l'application, au feuillage desdites plantes ou au sol contenant des graines ou d'autres organes de leur propagation, d'une quantité herbicide efficace d'un composé de structure:

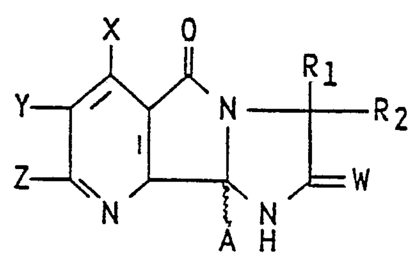

dans laquelle

$R_1$ et $R_2$ représentent chacun un alkyle en $C_1$—$C_3$ ou un cyclopropyle, sous réserve que la somme du nombre des atomes de carbone dans $R_1$ et $R_2$ soit de 2 à 5; et, lorsque $R_1$ et $R_2$ sont pris ensemble avec l'atome de carbone auquel ils sont fixés, ils peuvent former un cycle cycloalkyle en $C_3$—$C_6$ facultativement substitué par un méthyle;

W est un oxygène ou un soufre;

A est un hydrogène, un hydroxyle, un alcényloxy en $C_3$—$C_6$, un alcynyloxy en $C_3$—$C_6$, un alkylthio en $C_1$—$C_6$, $NR_{13}R_{14}$ ou un alcoxy en $C_1$—$C_6$ facultativement substitué par un phényle, un halogénophényle, un (alkyl en $C_1$—$C_3$)phényle, un (alcoxy en $C_1$—$C_3$)phényle ou un di(alkyl en $C_1$—$C_3$)aminophényle;

$R_{13}$ est un hydrogène, un alkyle en $C_1$—$C_4$ facultativement substitué par un phényle, un halogénophényle, un (alkyl en $C_1$—$C_3$)phényle ou un (alcoxy en $C_1$—$C_3$)phényle;

$R_{14}$ est un hydrogène ou un alkyle en $C_1$—$C_4$;

X est un hydrogène, un halogène ou un méthyle;

Y et Z sont chacun un hydrogène, un halogène, un alkyle en $C_1$—$C_6$, un hydroxyalkyle en $C_1$—$C_4$, un alcoxy en $C_1$—$C_6$, un alkylthio en $C_1$—$C_4$, un phénoxy, un halogénoalkyle en $C_1$—$C_4$, $OCF_2CHF_2$, $OCF_3$, $OCHF_2$, un nitro, un cyano, $NR_4R_5$, un alcényloxy en $C_3$—$C_8$ droit ou ramifié facultativement substitué par un à trois halogènes, un alcynyloxy en $C_3$—$C_8$ droit ou ramifié facultativement substitué par un à trois halogènes, ou un phényle facultativement substitué par un alkyle en $C_1$—$C_4$, un alcoxy en $C_1$—$C_4$ ou un halogène;

$R_4$ est un hydrogène ou un alkyle en $C_1$—$C_4$;

$R_5$ est un alkyle en $C_1$—$C_4$;

et, ensemble Y et Z peuvent former un cycle dans lequel YZ est représenté par

(1) la structure: $-(CH_2)_n-$, dans laquelle n est un entier valant 2, 3 ou 4 sous réserve que X soit un hydrogène; ou

(2) par la structure:

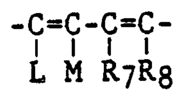

dans laquelle L, M, $R_7$ et $R_8$ représentent chacun un hydrogène, un halogène, un alkyle en $C_1$—$C_4$, un alcoxy en $C_1$—$C_4$, un alkylthio en $C_1$—$C_4$, un alkylsulfonyle en $C_1$—$C_4$, un halogénoalkyle en $C_1$—$C_4$, $NO_2$, CN, un phényle, un phénoxy, un amino, $OCF_3$, $OCHF_2$, $OCF_2CHF_2$, un alkylamino en $C_1$—$C_4$, un di(alkyl en $C_1$—$C_4$)-amino, un chlorophényle, un méthylphényle, un alcényloxy en $C_3$—$C_8$ droit ou ramifié facultativement substitué par un à trois halogènes, un alcynyloxy en $C_3$—$C_8$ droit ou ramifié facultativement substitué par un à trois halogènes, ou un phénoxy substitué par un groupe Cl, $CF_3$, $NO_2$ ou $CH_3$, sous réserve qu'un seul de L, M, $R_7$ ou $R_8$ puisse représenter un substituant autre qu'un hydrogène, un halogène, un alkyle en $C_1$—$C_4$ ou un alcoxy en $C_1$—$C_4$; ou

(3) par les structures:

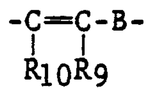 , 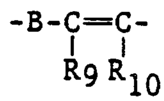 , 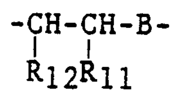 ou 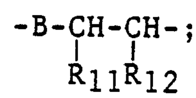 ;

où B est un oxygène ou un soufre; $R_9$ et $R_{10}$ représentent chacun un hydrogène, un halogène, un phényle ou un alkyle en $C_1$—$C_4$; $R_{11}$ et $R_{12}$ représentent chacun un hydrogène, un alkyle en $C_1$—$C_4$ ou un phényle; et lorsque $R_1$ et $R_2$ sont différents, les isomères cis ou trans, ou leurs mélanges, ou les isomères optiques (cis ou trans, ou leurs mélanges).

5. Procédé selon la revendication 4, où A, $R_1$, $R_2$, W et X sont comme défini dans la revendication 1; Y et Z représentent chacun indépendamment un hydrogène, un halogène, un alkyle en $C_1$—$C_6$, un alcoxy en $C_1$—$C_6$, CN, $NO_2$, $OCF_3$, $OCHF_2$, $OCF_2CHF_2$, un phénoxy, un halogénoalkyle en $C_1$—$C_4$, un alkylthio en $C_1$—$C_4$, un hydroxyalkyle en $C_1$—$C_4$, $NR_4R_5$, un alcényloxy en $C_3$—$C_8$ droit ou ramifié facultativement substitué par un à trois halogènes, un alcynyloxy en $C_1$—$C_8$ droit ou ramifié facultativement substitué par un à trois halogènes ou un phényle facultativement substitué par un alkyle en $C_1$—$C_4$, un alcoxy en $C_1$—$C_4$ ou un halogène; $R_4$ est un hydrogène ou un alkyle en $C_1$—$C_4$; $R_5$ est un alkyle en $C_1$—$C_4$; et ensemble X et Y peuvent former un cycle dans lequel YZ est représenté par la structure: $-(CH_2)_n-$, dans laquelle n est un entier valant 2, 3 ou 4, sous réserve que X soit un hydrogène; et, lorsque $R_1$ et $R_2$ sont différents, les isomères cis ou trans, ou leurs mélanges, ou les isomères optiques (cis ou trans, ou leurs mélanges).

6. Composition herbicide comprenant une quantité herbicide efficace d'un composé de structure:

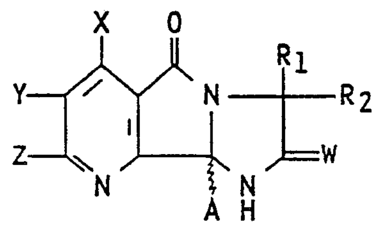

dans laquelle $R_1$, $R_2$, A, W, X, Y et Z sont comme défini dans la revendication 4 et un diluant inerte non toxique solide ou liquide de celui-ci.